# EUROPEAN PATENT APPLICATION

(11) **EP 4 768 478 A1**
(43) Date of publication of application: **01.07.2026**
(21) Application number: 25226569.9
(22) Date of filing: 22.12.2025
(51) Int. Cl.: C07D 401/12, C07D 401/14, C07D 471/10, C07D 487/10, A61P 25/28, A61K 31/4545

(54) **THALIDOMIDE AND QUINOLINE PROTAC COMPOUNDS ACTING AGAINST NEURODEGENERATIVE ILLNESSES**

(30) Priority: 30.12.2024 US 202463740183 P
(71) Applicant: Industrial Technology Research Institute, 310401 Hsinchu (TW)
(72) Inventor: HUNG, Chiu-Lien, Kaohsiung City (TW); CHEN, Chih-Hung, Hsinchu County (TW); CHANG, Yow-Lone, Hsinchu City (TW); WANG, Hsiang-Ching, Hsinchu City (TW); CHEN, Wan-Ru, Hsinchu City (TW)
(74) Representative: Bandpay & Greuter

(57) **Abstract**

A compound or a salt thereof is provided. The compound has a structure represented by Formula (I), Formula (II) or Formula (III): 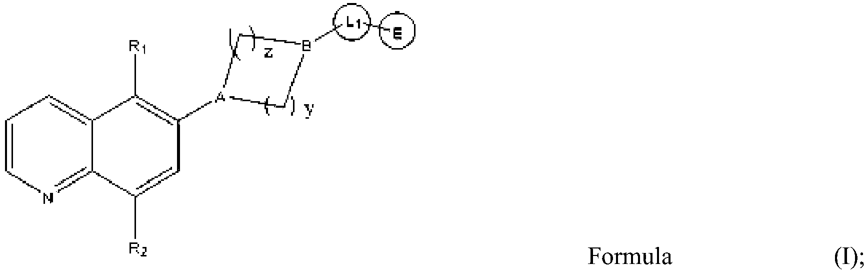 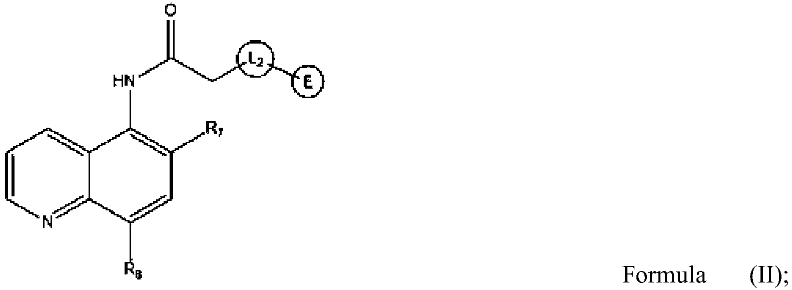 and

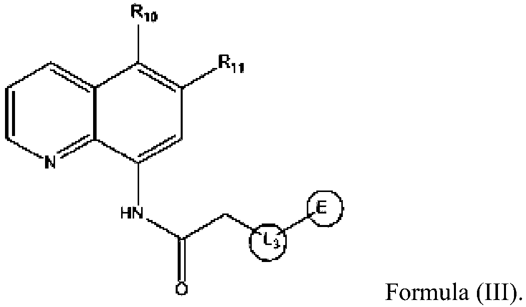

## Description

### CROSS REFERENCE TO RELATED APPLICATIONS

### TECHNICAL FIELD

The present invention relates to quinoline compounds and use thereof, and, in particular, it relates to the related uses of proteolysis-targeting chimeras (PROTACs) containing quinoline compounds.

### BACKGROUND

Neurodegenerative diseases are a class of diseases that primarily affect neurons (components of the nervous system). This class of diseases is characterized by the gradual atrophy of neurons (nerve cells) in the nervous system, leading to a decline or even loss of cognitive, memory, motor, and emotional abilities.

Neurodegenerative diseases are closely associated with protein abnormalities, primarily due to misfolding and the accumulation and deposition of proteins, which impair the function of nerve cells and may even lead to their death. The accumulation of these abnormal proteins is a key pathological feature in many neurodegenerative diseases, such as Alzheimer's disease (AD), Parkinson's disease (PD), and Huntington's disease (HD), all of which are associated with abnormal protein deposits.

Recent studies have shown that transactive response DNA-binding protein-43 (TDP-43) is a major pathogenic factor in a variety of neurodegenerative diseases, with its pathological marker deposition rate reaching 50% and 90% in patients with frontotemporal dementia (FTD) and amyotrophic lateral sclerosis (ALS), respectively.

Currently, the vast majority of conventional treatments for neurodegenerative diseases can only provide symptomatic relief, lacking effective therapies that target pathological mechanisms or the etiology of the disease. More specifically, current treatment strategies for neurodegenerative diseases do not include treatments targeting the degradation of harmful proteins. Accordingly, for neurodegenerative diseases there is an urgent need for technological solutions that can eliminate harmful proteins.

### SUMMARY

The present disclosure provides a compound or a salt thereof, wherein the compound has a structure represented by Formula (I), Formula (II) or Formula (III): 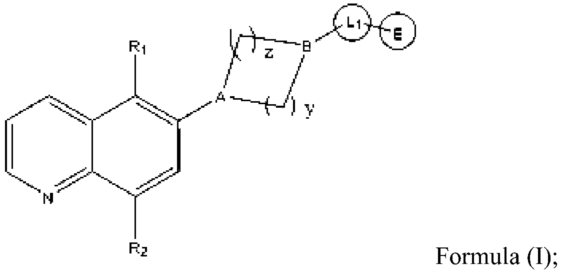 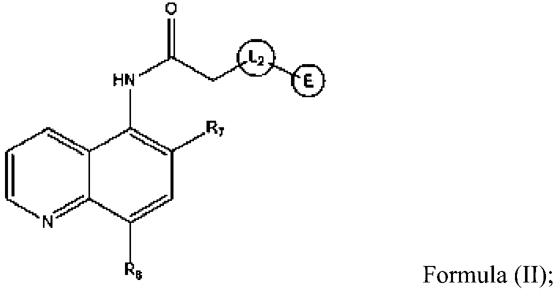 and 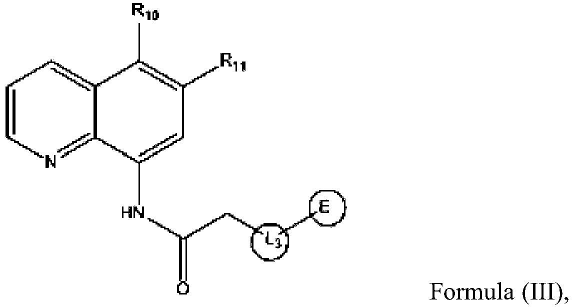 wherein R₁ is H, cyano, NH₂, NO₂, NHR₃, NHR₃R₄, a heterocycle, NHCOR₅ or 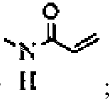 R₂ is NHCOR₆ or NH₂; A and B are independently N, C, or absent; y and z are independently an integer from 1 to 3; R₃, R₄, R₅ and R₆ are independently C₁-C₆ alkyl, and the the alkyl is a linear or branched alkyl, and is optionally replaced by one or more halogen atoms, oxygen atoms, sulfur atoms or amines; R₇ is SCH₃ or OCH₃; R₈ is NHCOR₉ or NH₂; R₉ is C₁-C₆ alkyl; R₁₀ is H, cyano, NH₂, NO₂, NHR₃, NHR₃R₄, a heterocycle or NHCOR₅; R₁₁ is SCH₃, OCH₃ or a heterocycle; L₁ is a linker having a structure selected from a group consisiting of the structures shown in the following:

| Structure of L₁ |
|---|
| 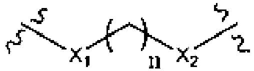 |
| 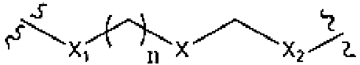 |
| 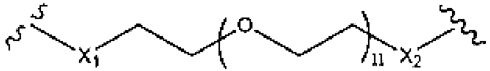 |
| 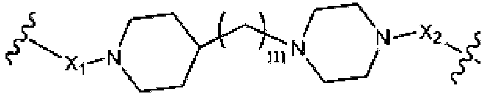 |
| 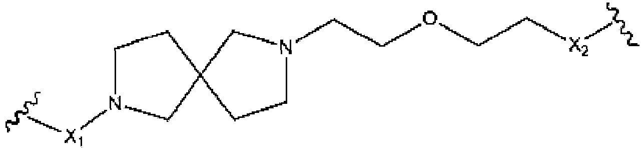 |
| 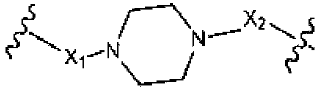 |
| 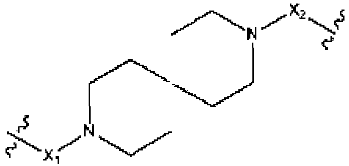 |
| 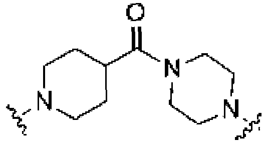 |
| 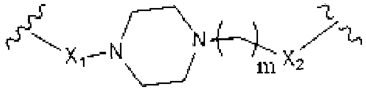 |
| 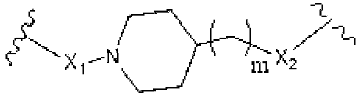 |
| 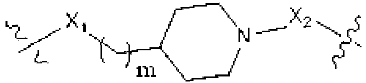 |
| 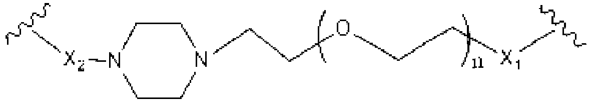 |
| 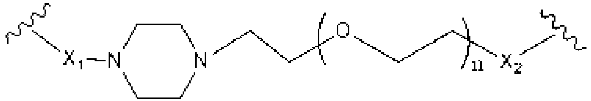 |
| 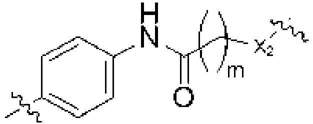 |

; L₂ and L₃ are independently a linker having a structure selected from a group consisiting of the structures shown in the following:

| Structures of L₂ and L₃ |
|---|
| 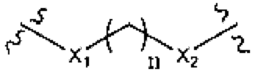 |
| 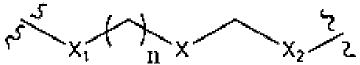 |
| 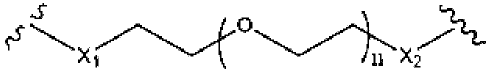 |
| 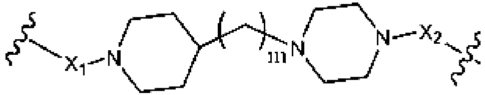 |
| 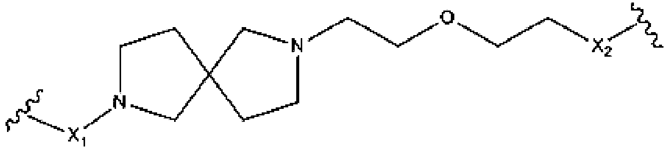 |
| 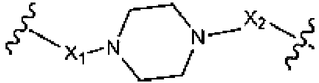 |
| 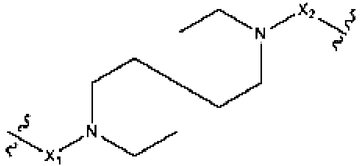 |
| 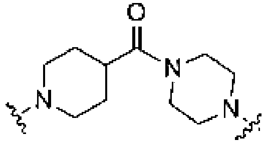 |

, wherein X₁ is a bond, -NH-, -O, -CO-, CONH or -PhNHCO-; X₂ is a bond, -NH-, -O-, -NHCOCH₂NH-, -NHCOCH₂O- or -alkyne-; X is a heterocycle; n is an integer from 1 to 6; m is an integer from 0 to 8, and wherein E is an E3 ubiquitin ligase binding domain, which comprises one of the structures shown in the following:

| Structure of E3 | | |
|---|---|---|
| 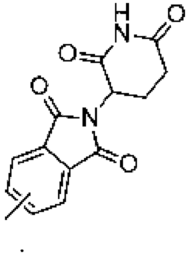 | 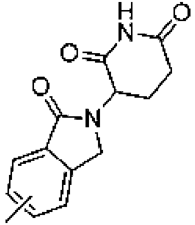 | 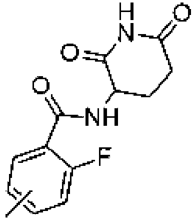 |

The present disclosure also provides a pharmaceutical composition, comprising: the compound or a salt thereof mentioned above; and a pharmaceutically acceptable carrier or salt.

The present disclosure further provides an agent for degrading transactive response DNA-binding protein-43 and/or inhibiting the activity of transactive response DNA-binding protein-43, comprising the compound or a salt thereof mentioned above.

In addition, the present disclosure provides a pharmaceutical composition for treating and/or preventing a disease associated with transactive response DNA-binding protein-43 accumulation, comprising the compound or a salt thereof mentioned above.

### BRIEF DESCRIPTION OF THE DRAWINGS

The present invention can be more fully understood by reading the subsequent detailed description and examples with references made to the accompanying drawings, wherein:

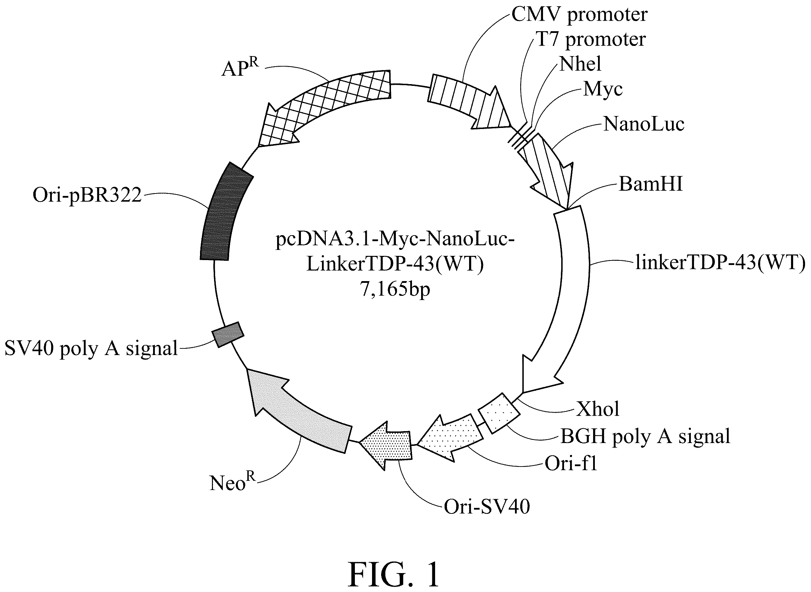

FIG. 1 shows the expression vector, pcDNA3.1-Myc-NanoLuc-LinkerTDP43 (abbreviated as NanoLuc-TDP-43(WT)), employed in the experiments analyzing the activity of compounds that induce degradation of transactive response DNA-binding protein-43 (TDP-43) via the NanoLuc-TDP-43 fusion protein assay system in one embodiment of the present disclosure. WT: wild type; and

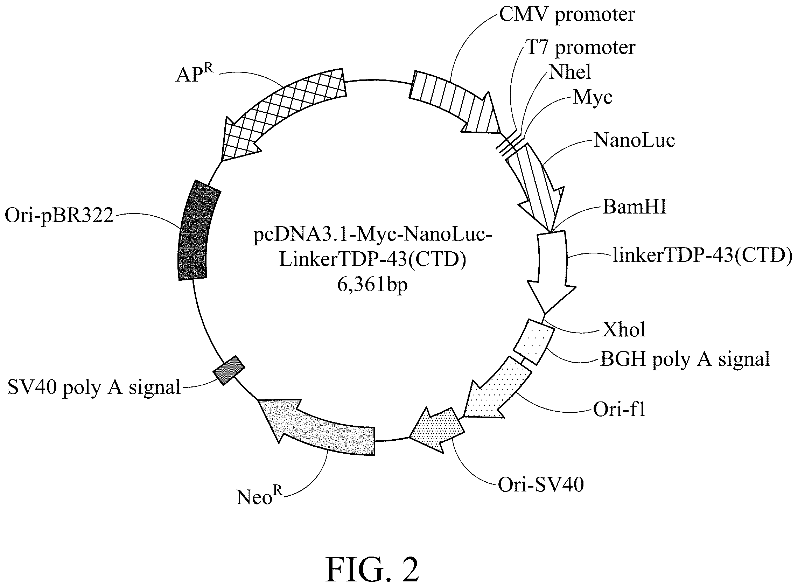

FIG. 2 shows the expression vector, pcDNA3.1-Myc-NanoLuc-LinkerTDP43CTD (abbreviated as NanoLuc-TDP-43(CTD)), employed in the experiments analyzing the activity of compounds that induce degradation of transactive response DNA-binding protein-43 (TDP-43) via the NanoLuc-TDP-43 fusion protein assay system in one embodiment of the present disclosure. CTD: C-terminal domain.

### DETAILED DESCRIPTION

The following description is made for the purpose of illustrating the general principles of the invention and should not be taken in a limiting sense. The scope of the invention is best determined by reference to the appended claims.

Transactive response DNA-binding protein-43 kDa (TDP-43) is a protein encoded by the *TARDBP* gene in humans.

The transactive response DNA-binding protein-43 has 414 amino acid residues and consists of an N-terminal domain (NTD), two highly conserved folded RNA recognition motifs, and an unstructured C-terminal domain (CTD). The N-terminal domain amino acid residues 1-76, exhibits a well-defined folded structure, and has been shown to be capable of forming dimers or oligomers. Two highly conserved folded RNA recognition motifs span the positions 106-176 of amino acid residue (RRM1) and the positions 191-259 of amino acid residue (RRM2), respectively, and are essential for binding to target RNA and DNA. The unstructured C-terminal domain spans the positions 274-414 of amino acid residue, contains a glycine-rich region, participates in protein-protein interactions, and carries most mutations associated with familial amyotrophic lateral sclerosis (ALS).

Transactive response DNA-binding protein-43 is a highly conserved intranuclear RNA/DNA-binding protein involved in the regulation of RNA processing. However, its mislocalization and aggregation in the cytoplasm are hallmarks of various neurodegenerative diseases, particularly amyotrophic lateral sclerosis (ALS) and frontotemporal dementia (FTD). Therefore, the clearance of harmful proteins is expected to be one of the therapies that can fundamentally solve neurodegenerative diseases.

Proteolysis targeting chimeras (PROTACs) are small molecule drugs with two different functional ligands, one with the ability to bind to a protein of interest (POI) and the other to recruit an E3 ubiquitin ligase. Through binding the proteolysis targeting chimera to the target protein, the E3 ubiquitin ligase will be able to get close to the target protein and label it with ubiquitin. The target protein which is labeled by ubiquitin will be recognized and degraded by the proteasome and degraded into small fragments of peptides, so that the proteolysis targeting chimera is separated from the target protein and can be recycled in cells.

Based on the foregoing, the present disclosure provides a compound or a salt thereof. The compound or a salt thereof of the present disclosure may be used for the preparation of an agent, a medicament or a pharmaceutical composition, but it is not limited thereto. The agent mentioned above may be an agent for degrading transactive response DNA-binding protein-43 and/or inhibiting the activity of transactive response DNA-binding protein-43. Moreover, the medicament or pharmaceutical composition mentioned above may be a medicament or pharmaceutical composition for treating and/or preventing a disease associated with transactive response DNA-binding protein-43 accumulation, or may be a medicament or pharmaceutical composition for treating and/or preventing a disease which can be alleviated and/or cured by degrading transactive response DNA-binding protein-43 and/or inhibiting the activity of transactive response DNA-binding protein-43, but it is not limited thereto. Alternatively, the medicament or pharmaceutical composition of the present disclosure may be directly used for treatment and/or prevention of a disease while the disease mentioned above may be a disease associated with transactive response DNA-binding protein-43 accumulation or a disease which can be alleviated and/or cured by inhibiting the activity of transactive response DNA-binding protein-43 and/or degrading transactive response DNA-binding protein-43, but it is also not limited thereto.

In one embodiment, the compound or a salt thereof of the present disclosure may be in a form of proteolysis targeting chimera.

The compound of the present disclosure mentioned above may have a structure represented by following Formula (I), Formula (II) or Formula (III), but it is not limited thereto: 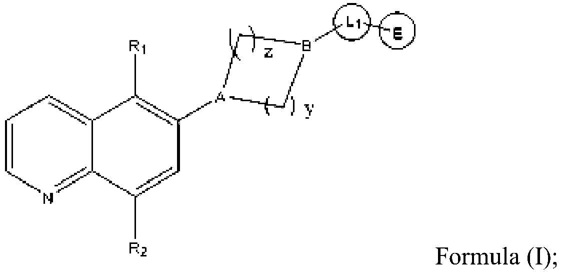 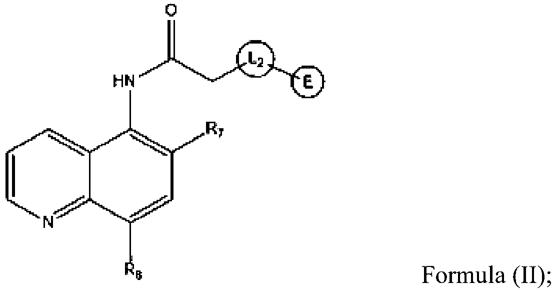 and

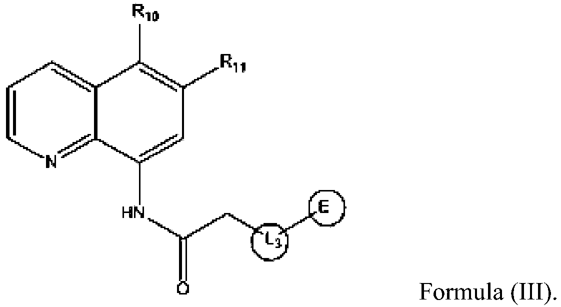

R₁ may be H, cyano, NH₂, NO₂, NHR₃, NHR₃R₄, a heterocycle, NHCOR₅, 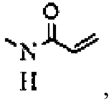 etc., but it is not limited thereto. R₂ may be NHCOR₆, NH₂, etc., but it is also not limited thereto. R₃, R₄, R₅ and R₆ independently msy be C₁-C₆ alkyl. The foregoing alkyl may be a linear or branched alkyl, and may be optionally replaced by one or more halogen atoms, oxygen atoms, sulfur atoms or amines. The foregoing halogen atoms may be F, Cl or Br, but it is not limited thereto.

A and B independently may be N, C, or absent.

y and z independently may be an integer from 1 to 3.

R₇ may be SCH₃ or OCH₃. R₈ may be NHCOR₉ or NH₂, and R₉ may be C₁-C₆ alkyl.

R₁₀ may be H, cyano, NH₂, NO₂, NHR₃, NHR₃R₄, a heterocycle or NHCOR₅, but it is not limited thereto. The definitions of R₃, R₄ and R₅ are the same as described above.

R₁₁ may be SCH₃, OCH₃ or a heterocycle, but it is not limited thereto.

L₁ may be a linker having one of the structures shown in the following Table 1, but it is nor limited thereto:

**Table 1**

| Structure of L₁ |
|---|
| 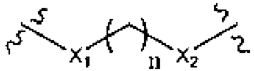 |
| 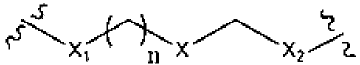 |
| 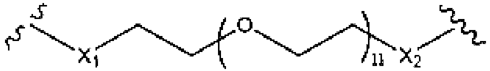 |
| 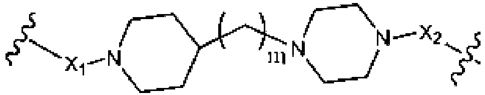 |
| 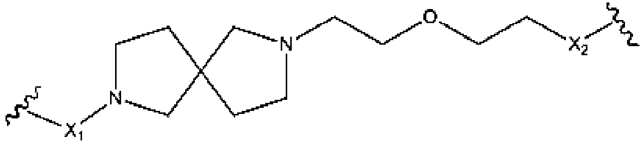 |
| 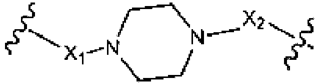 |
| 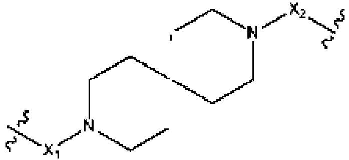 |
| 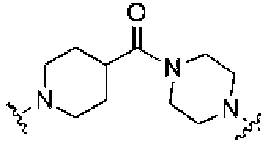 |
| 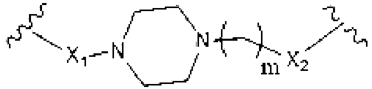 |
| 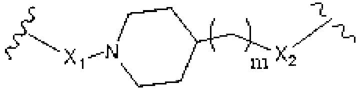 |
| 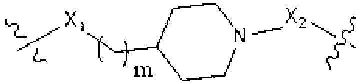 |
| 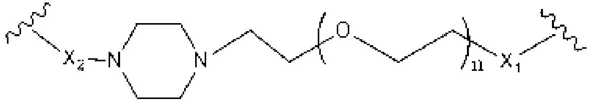 |
| 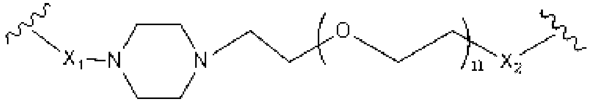 |
| 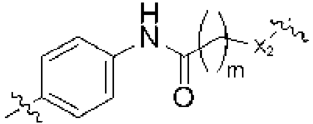 |

L₂ and L₃ independently may be a linker having one of the structures shown in the following Table 2:

**Table 2**

| Structures of L₂ and L₃ |
|---|
| 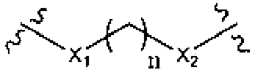 |
| 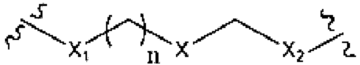 |
| 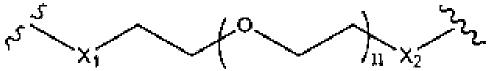 |
| 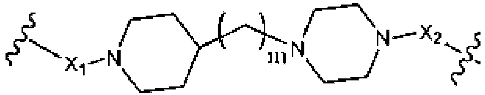 |
| 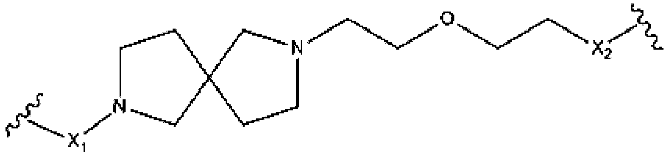 |
| 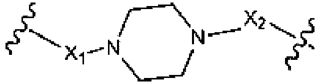 |
| 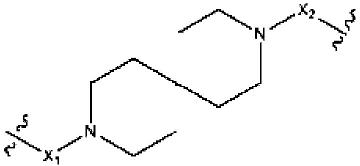 |
| 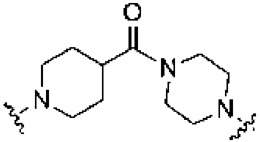 |

In Table 1 and Table 2, the definitions of X₁, X₁, X, n and m are described as the following.

X₁ may be a bond, -NH-, -O, -CO-, CONH or -PhNHCO-, but it is not limited thereto. X₂ may be a bond, -NH-, -O-, -NHCOCH₂NH-, -NHCOCH₂O- or -alkyne-, but it is also not limited thereto.

X is a heterocycle, but it is also not limited thereto.

n may be an integer from 1 to 6, and m may be an integer from 0 to 8.

Furthermore, E is an E3 ubiquitin ligase binding domain, which may comprise one of the structures shown in the following Table 3, but it is not limited thereto:

**Table 3**

| | | |
|---|---|---|
| Structure of E3 | | |
| 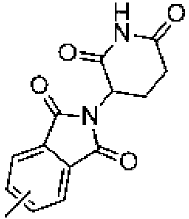 | 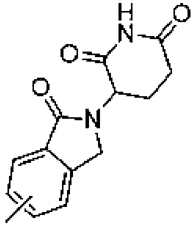 | 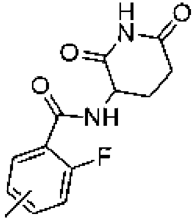 |

In one embodiment, the foregoing compound of the present disclosure may have a structure represented by Formula (I) shown above.

In one specific embodiment of the above-mentioned embodiment in which the foregoing compound of the present disclosure may have a structure represented by Formula (I) shown above, in the Formula (I) shown above, R₁ may be H, cyano, NO₂, N(CH₃)₂, NHAc or 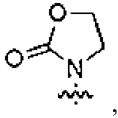 but it is not limited thereto. Moreover, in another specific embodiment of the above-mentioned embodiment in which the foregoing compound of the present disclosure may have a structure represented by Formula (I) shown above, in the Formula (I) shown above, R₂ may be NHAc, but it is also not limited thereto.

Furthermore, in one specific embodiment, the foregoing compound of the present disclosure may have a structure represented by Formula (I) shown above, and the foregoing compound of the present disclosure, may comprise, but is not limited to one of the compounds shown in the following Table 4:

**Table 4**

| **Compound number** | **Structure** |
|---|---|
| **D101** | 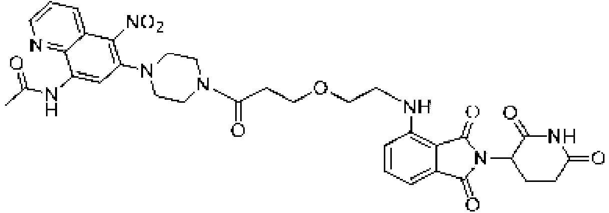 |
| **D102** | 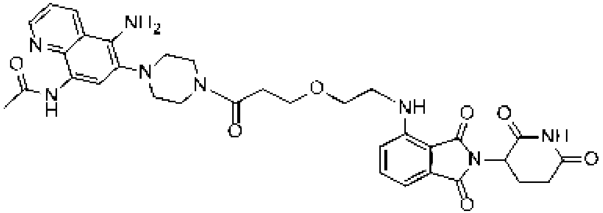 |
| **D103** | 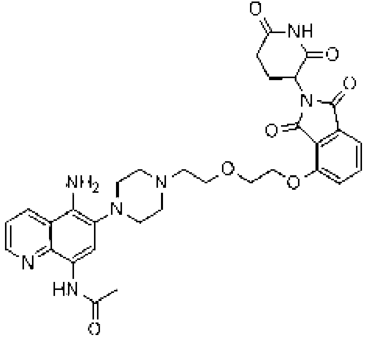 |
| **D104** | 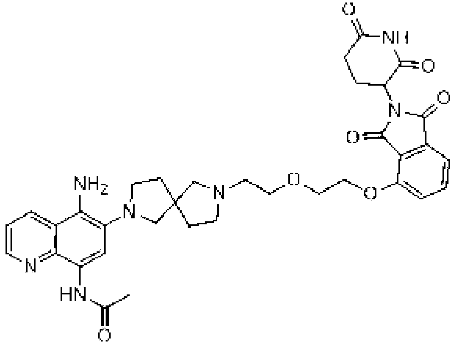 |
| **D105** | 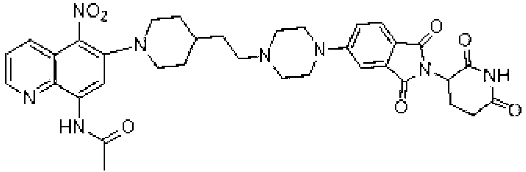 |
| **D106** | 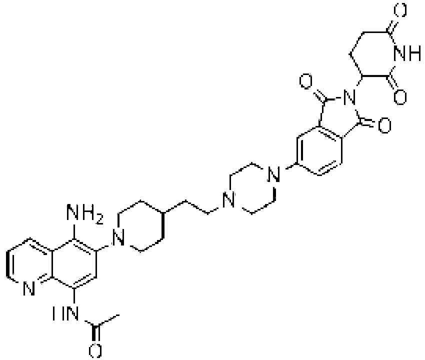 |
| **D107** | 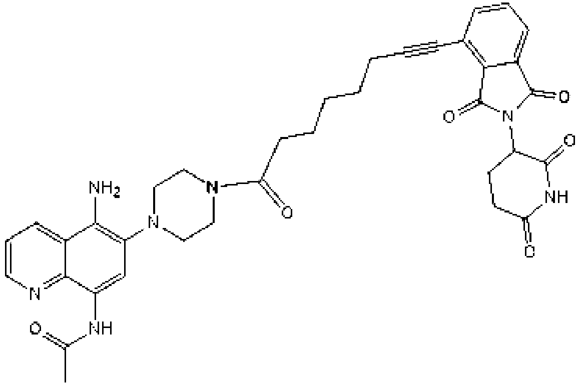 |
| **D108** | 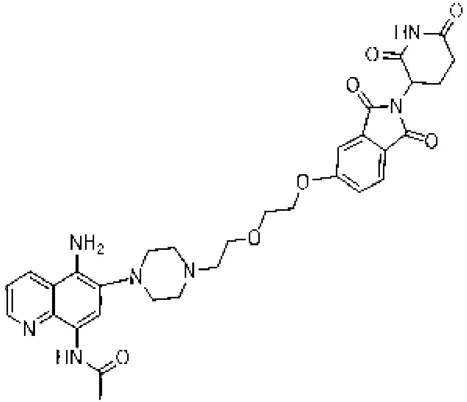 |
| **D109** | 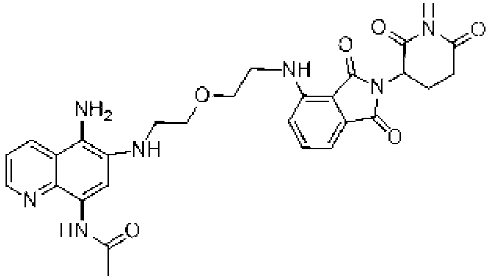 |
| **D110** | 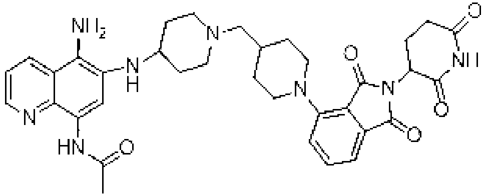 |
| **D111** | 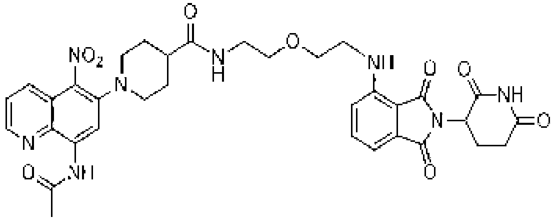 |
| **D112** | 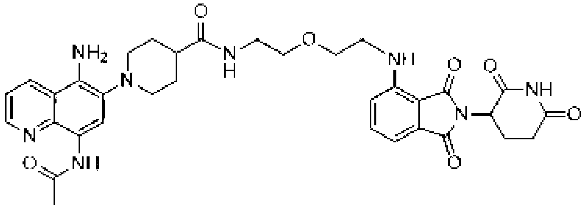 |
| **D113** | 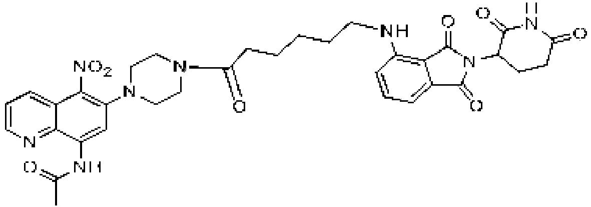 |
| **D114** | 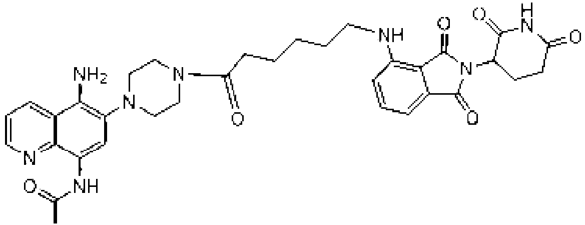 |
| **D115** | 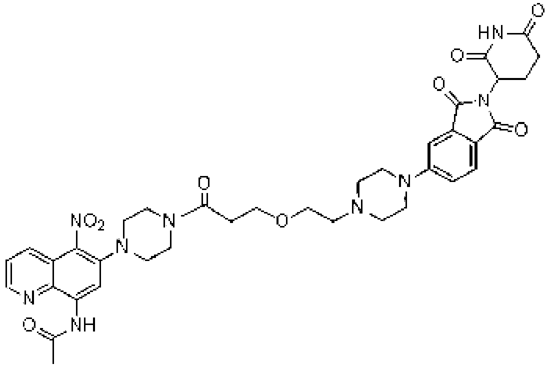 |
| **D116** | 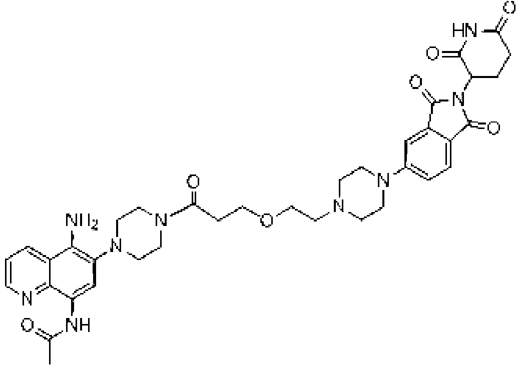 |
| **D117** | 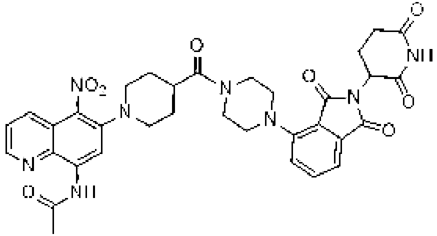 |
| **D118** | 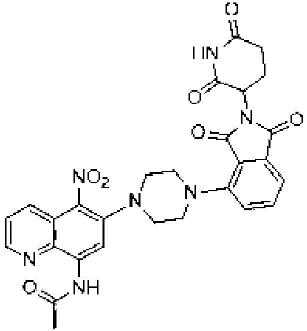 |
| **D119** | 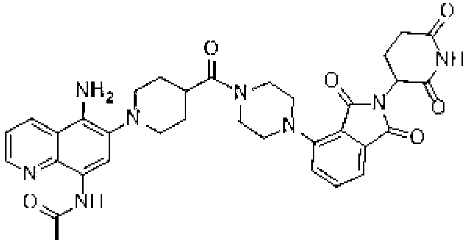 |
| **D120** | 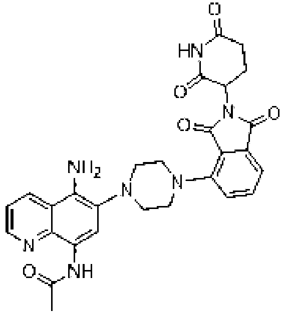 |
| **D121** | 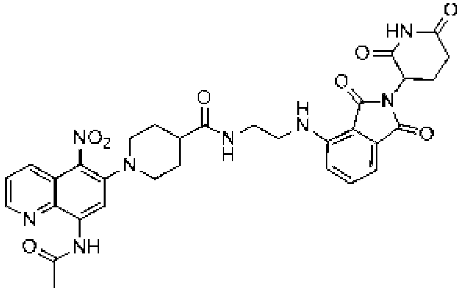 |
| **D122** | 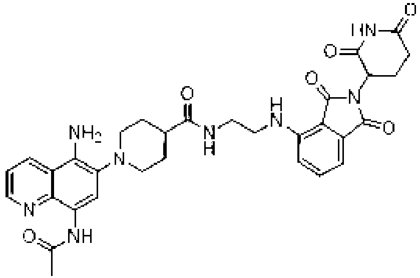 |
| **D123** | 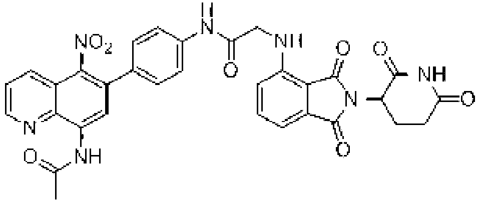 |
| **D124** | 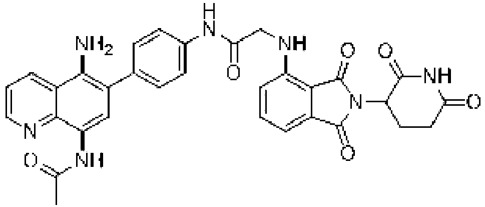 |
| **D125** | 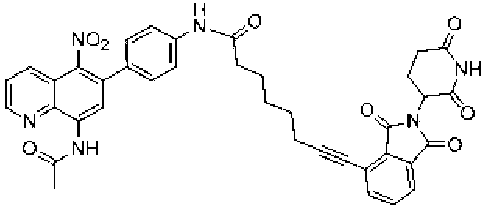 |
| **D126** | 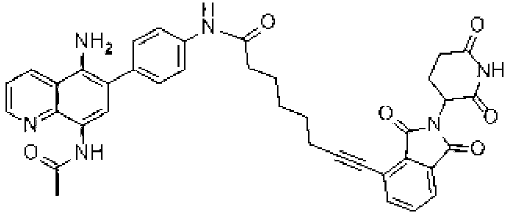 |
| **D127** | 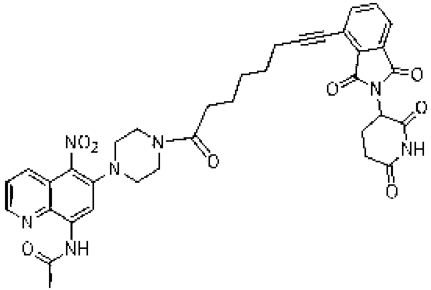 |
| **D128** | 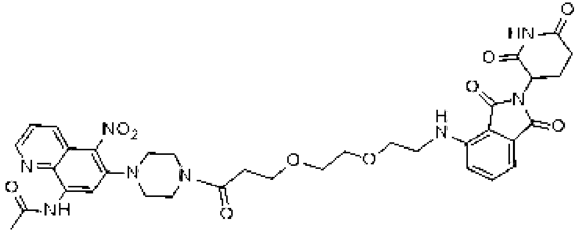 |
| **D129** | 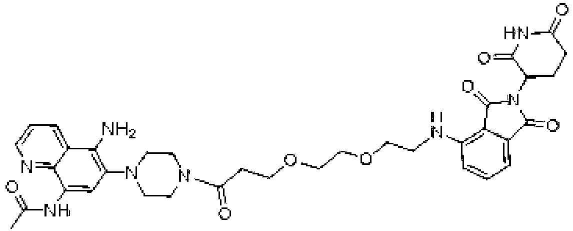 |
| **D130** | 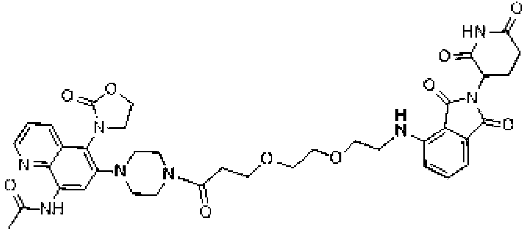 |
| **D131** | 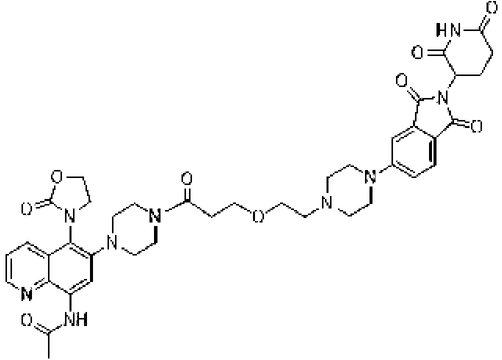 |
| **D132** | 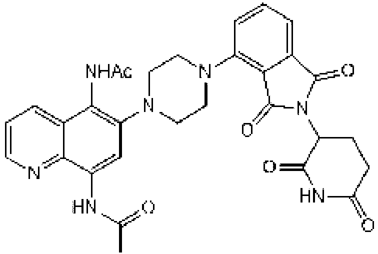 |
| **D133** | 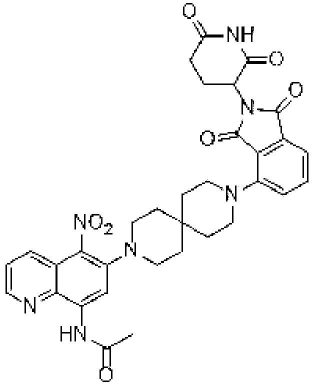 |
| **D134** | 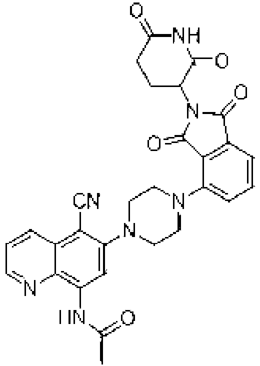 |
| **D135** | 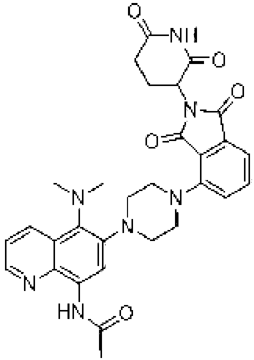 |
| **D136** | 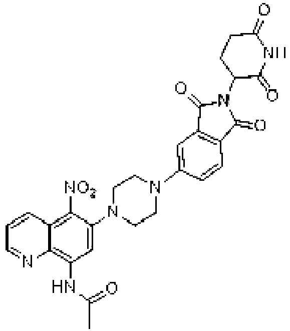 |
| **D137** | 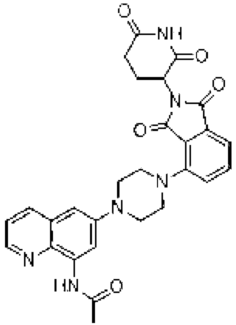 |
| **D138** | 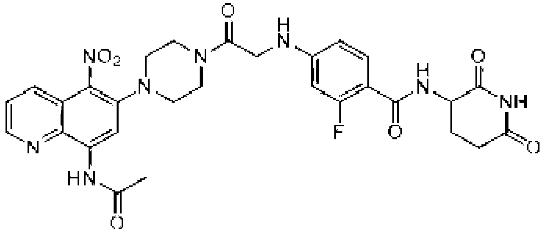 |
| **D139** | 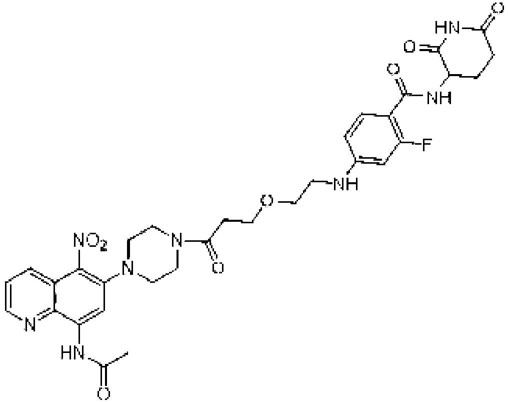 |
| **D140** | 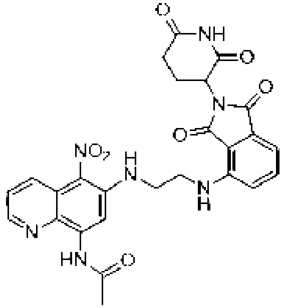 |
| **D141** | 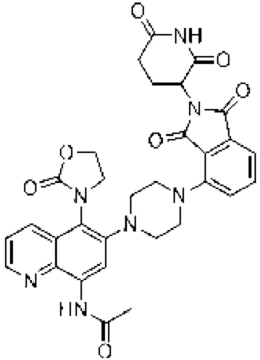 |
| **D142** | 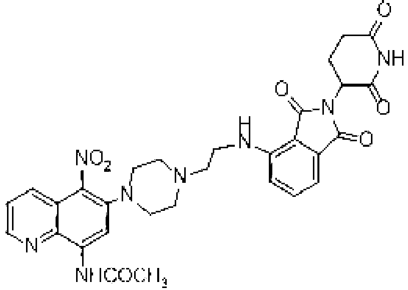 |
| **D143** | 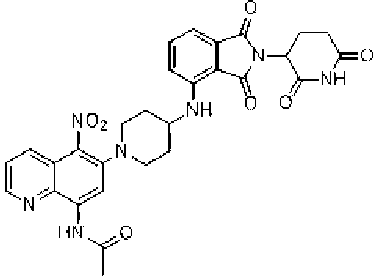 |
| **D144** | 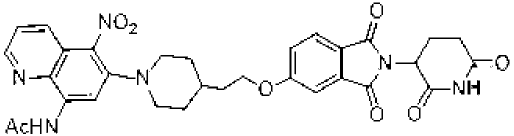 |
| **D145** | 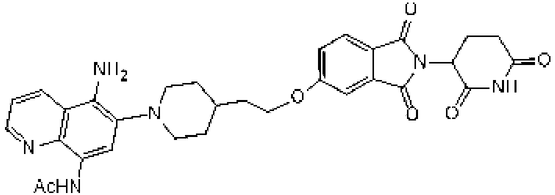 |
| **D146** | 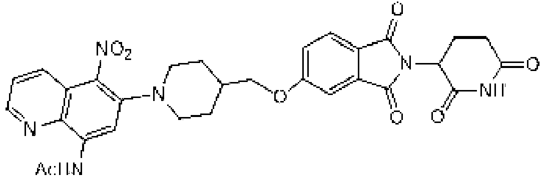 |
| **D147** | 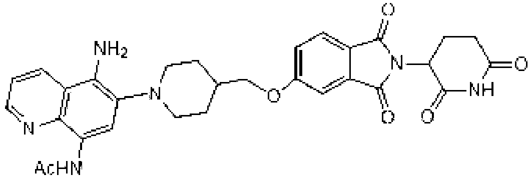 |
| **D148** | 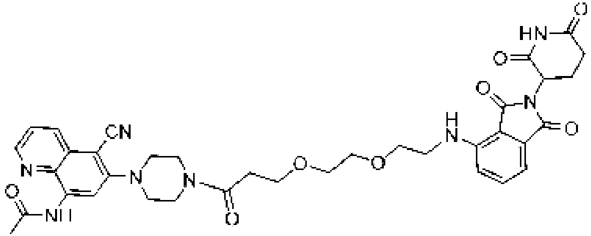 |
| **D149** | 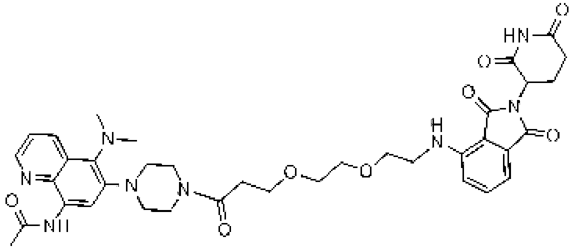 |
| **D150** | 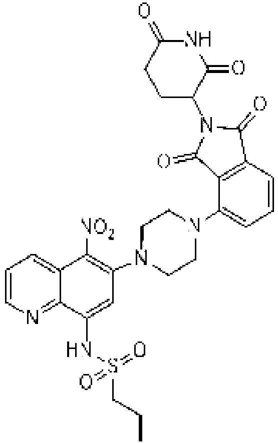 |
| **D151** . | 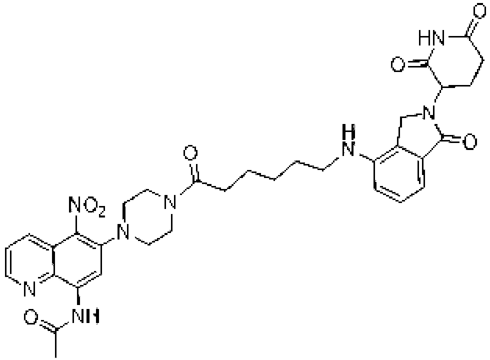 |

In another embodiment, the foregoing compound of the present disclosure may have a structure represented by Formula (II) shown above.

In one specific embodiment of the above-mentioned embodiment in which the foregoing compound of the present disclosure may have a structure represented by Formula (II) shown above, in the Formula (II) shown above, R₇ may be SCH₃, but it is not limited thereto. Moreover, in another specific embodiment of the above-mentioned embodiment in which the foregoing compound of the present disclosure may have a structure represented by Formula (II) shown above, in the Formula (II) shown above, R₈ may be NHAc, but it is not limited thereto.

Furthermore, in one specific embodiment, the foregoing compound of the present disclosure may have a structure represented by Formula (II) shown above, and the foregoing compound of the present disclosure, may comprise, but is not limited to one of the compounds shown in the following Table 5:

**Table 5**

| **Compound number** | **Structure** |
|---|---|
| **D201** | 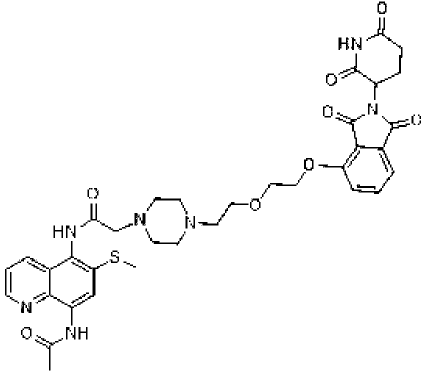 |
| **D202** | 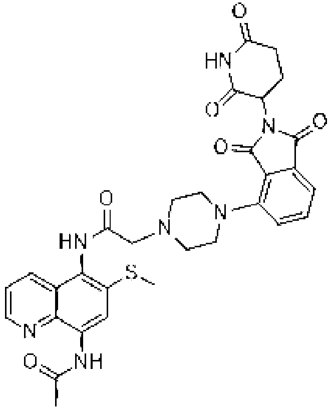 |
| **D203** | 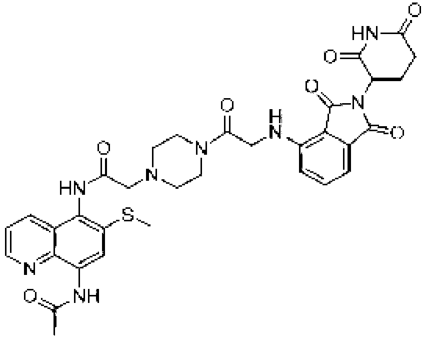 |
| **D204** . | 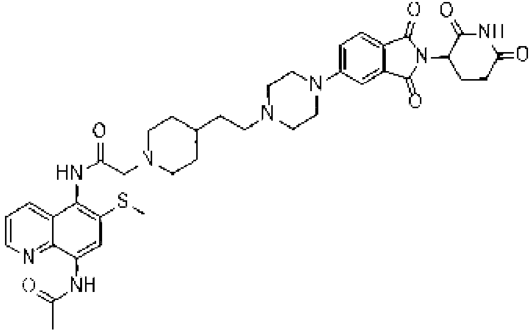 |

In further another embodiment, the foregoing compound of the present disclosure may have a structure represented by Formula (III) shown above.

In one specific embodiment of the above-mentioned embodiment in which the foregoing compound of the present disclosure may have a structure represented by Formula (III) shown above, in the Formula (III) shown above, R₁₀ msy be NO₂, CN or 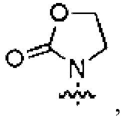 but it is not limited thereto. Moreover, in another specific embodiment of the above-mentioned embodiment in which the foregoing compound of the present disclosure may have a structure represented by Formula (III) shown above, in the Formula (III) shown above, R₁₁ may be OCH₃, SCH₃, morpholine, n-methylpiperazine, pyrrolidin-3-amine, 4-aminopiperidineor 4-(N-Boc-amino)piperidine, but it is not limited thereto.

Furthermore, in one specific embodiment, the foregoing compound of the present disclosure may have a structure represented by Formula (III) shown above, and the foregoing compound of the present disclosure, may comprise, but is not limited to one of the compounds shown in the following Table 6:

**Table 6**

| **Compound number** | **Structure** |
|---|---|
| **D301** | 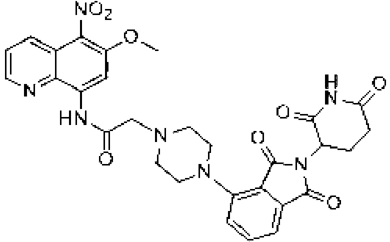 |
| **D302** | 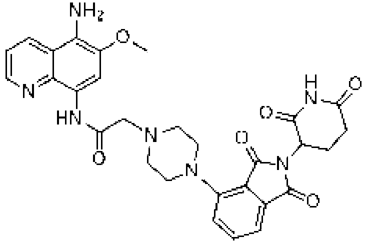 |
| **D303** | 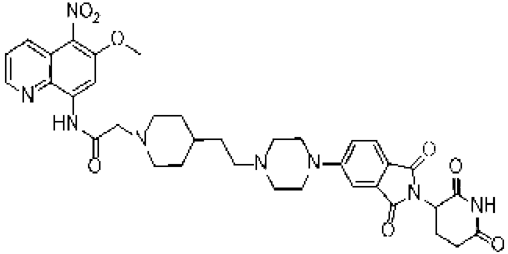 |
| **D304** | 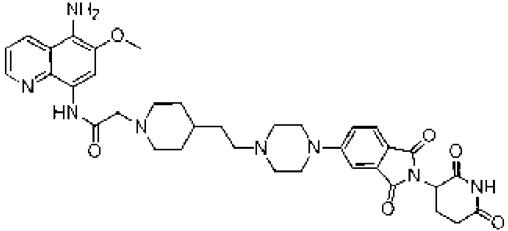 |
| **D305** | 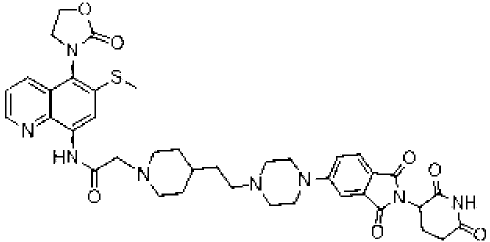 |
| **D306** | 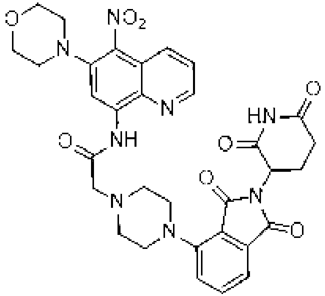 |
| **D307** | 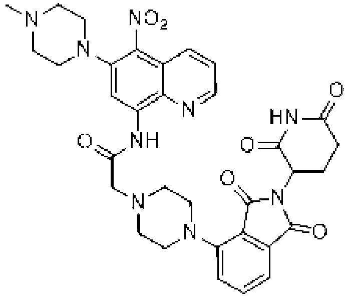 |
| **D308** | 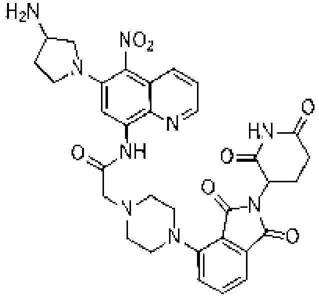 |
| **D309** | 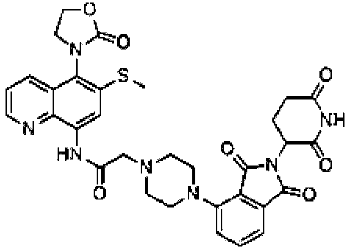 |
| **D310** | 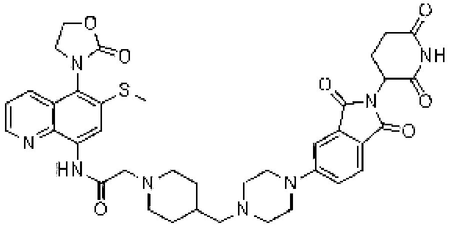 |
| **D311** | 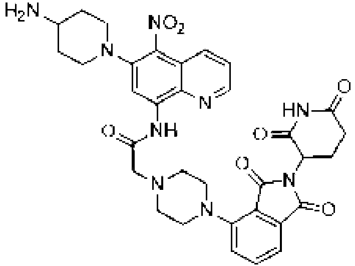 |
| **D312** | 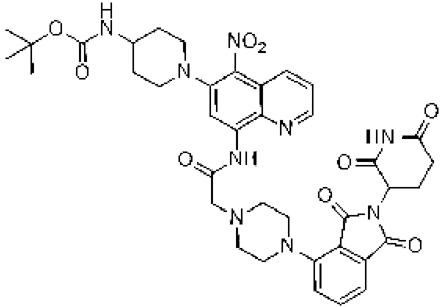 |
| **D313** | 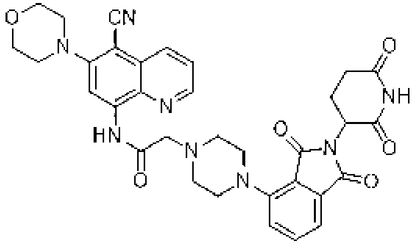 |
| **D314** | 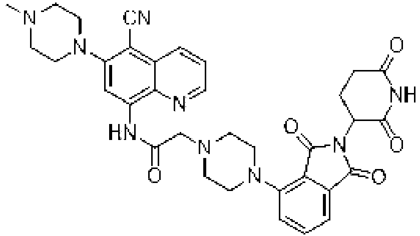 |
| **D315** | 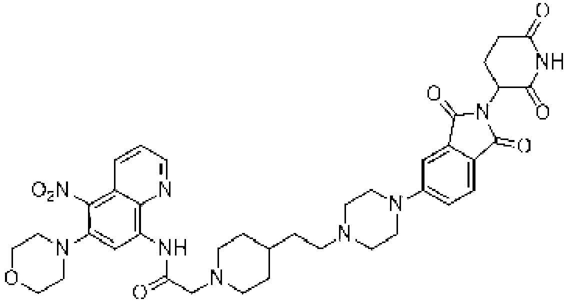 |
| **D316** . | 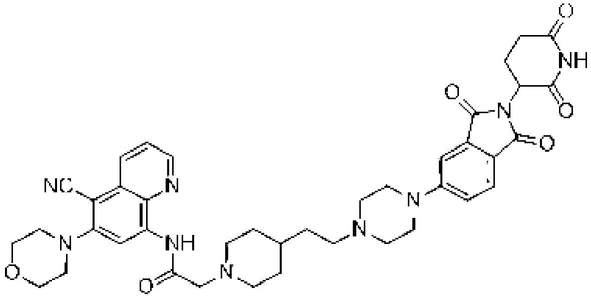 |

According to the structures of the compounds of the present disclosure shown above, it can be known that the compound or a salt thereof of the present disclosure may be a proteolysis targeting chimera for transactive response DNA-binding protein-43, which can effectively bind to the transactive response DNA-binding protein-43, thereby enabling the transactive response DNA-binding protein-43 to be recognized, cleaved, and degraded by proteasome. The aforementioned transactive response DNA-binding protein-43 may refer to the full-length transactive response DNA-binding protein-43 or may only refer to the separate C-terminal domain of transactive response DNA-binding protein-43, without particular limitation. Specifically, the aforementioned compound or a salt thereof of the present disclosure may bind to the full-length transactive response DNA-binding protein-43 or may only bind to the separate C-terminal domain of transactive response DNA-binding protein-43, without particular limitation.

In addition, based on the foregoing, the present disclosure may also provide a pharmaceutical composition, which may comprise, but is not limited to, any compound or a salt thereof of the present disclosure mentioned above and a pharmaceutically acceptable carrier or salt.

The pharmaceutical composition of the present disclosure may be a medicament or pharmaceutical composition for treating and/or preventing a disease associated with transactive response DNA-binding protein-43 accumulation, or may be a medicament or pharmaceutical composition for treating and/or preventing a disease which can be alleviated and/or cured by inhibiting the activity of transactive response DNA-binding protein-43 and/or degrading transactive response DNA-binding protein-43, but it is not limited thereto. Alternatively, the pharmaceutical composition of the present disclosure may be used for treatment and/or prevention of a disease while the disease mentioned above may be a disease associated with transactive response DNA-binding protein-43 accumulation or a disease which can be alleviated and/or cured by inhibiting the activity of transactive response DNA-binding protein-43 and/or degrading transactive response DNA-binding protein-43, but it is also not limited thereto. In one embodiment, the disease mentioned above may comprise, but is not limited to, a neurodegenerative disease. Examples of the neurodegenerative disease may comprise Alzheimer's disease (AD), Parkinson's disease (PD), amyotrophic lateral sclerosis (ALS), dementia with Lewy bodies (DLB), multiple system atrophy (MSA), frontotemporal dementia (FTD), Huntington's disease (HD), etc., but it is not limited thereto.

The pharmaceutically acceptable carrier mentioned above may comprise, but is not limited to, a solvent, a dispersion medium, a coating, an antibacterial and antifungal agent, or an isotonic and absorption delaying agent, etc. which is suitable for pharmaceutical administration. The pharmaceutical composition can be formulated into dosage forms for different administration routes utilizing conventional methods.

Moreover, the pharmaceutically acceptable salt mentioned above may comprise, but is not limited to, salts including inorganic cation, such as alkali metal salts such as sodium salt, potassium salt or amine salt, such as alkaline-earth metal salt such as magnesium salt or calcium salt, such as the salt containing bivalent or quadrivalent cation such as zinc salt, aluminum salt or zirconium salt. In addition, the pharmaceutically acceptable salt may also be organic salt, such as dicyclohexylamine salt, methyl-D-glucamine, and amino acid salt such as arginine, lysine, histidine, or glutamine.

Furthermore, the pharmaceutical composition of the present disclosure can be administered to a subject in need thereof, but is not limited thereto. The administration route of the pharmaceutical composition of the present disclosure may include parenteral manner, oral manner, via inhalation spray, or by implanted reservoir, but is not limited thereto. The parenteral methods may comprise, but is not limited to, subcutaneous, intracutaneous, intravenous, intramuscular, intraarticular, intraarterial, intrasynovial, intrasternal, intrathecal, intralesional injection, as well as infusion techniques, etc.

The subject in need to be administrated the pharmaceutical composition mentioned above may comprise, but is not limited to, a vertebrate. Moreover, the vertebrate mentioned above may comprise a fish, an amphibian, a reptile, a bird or a mammal, but it is not limited thereto. Examples of the mammal may comprise, but are not limited to a human, an orangutan, a monkey, a horse, a donkey, a dog, a cat, a rabbit, a guinea pig, a rat and a mouse. In one embodiment, the subject mentioned above may be a human.

In addition, based on the foregoing, the present disclosure may further provide a use of any the compound or a salt thereof of the present disclosure mentioned above or any pharmaceutical composition of the present disclosure mentioned above in the manufacture of an agent for degrading transactive response DNA-binding protein-43 and/or inhibiting the activity of transactive response DNA-binding protein-43. The aforementioned transactive response DNA-binding protein-43 may refer to the full-length transactive response DNA-binding protein-43 or may only refer to the separate C-terminal domain of transactive response DNA-binding protein-43, without particular limitation. Namely, the aforementioned agent for degrading transactive response DNA-binding protein-43 and/or inhibiting the activity of transactive response DNA-binding protein-43 may degrade the full-length transactive response DNA-binding protein-43 or may degrade the separate C-terminal domain of transactive response DNA-binding protein-43, without particular limitation.

Furthermore, similarly, based on the foregoing, the present disclosure may further provide a use of any the compound or a salt thereof of the present disclosure mentioned above or any the pharmaceutical composition of the present disclosure mentioned above in the manufacture of a medicament for treating and/or preventing a disease associated with transactive response DNA-binding protein-43 accumulation.

In one embodiment, the aforementioned disease associated with transactive response DNA-binding protein-43 accumulation may comprise, but is not limited to, a neurodegenerative disease. Examples of the neurodegenerative disease may comprise Alzheimer's disease (AD), Parkinson's disease (PD), amyotrophic lateral sclerosis (ALS), dementia with Lewy bodies (DLB), multiple system atrophy (MSA), frontotemporal dementia (FTD), Huntington's disease (HD), etc., but it is not limited thereof.

Similarly, according to the foregoing, the present disclosure may provide an agent for degrading transactive response DNA-binding protein-43 and/or inhibiting the activity of transactive response DNA-binding protein-43. The agent for degrading transactive response DNA-binding protein-43 and/or inhibiting the activity of transactive response DNA-binding protein-43 may comprise, but is not limited to, any the compound or a salt thereof of the present disclosure mentioned above or any the pharmaceutical composition of the present disclosure mentioned above.

Moreover, the present disclosure may also provide a method for *in vivo* degrading transactive response DNA-binding protein-43 and/or inhibiting the activity of transactive response DNA-binding protein-43, and this method may comprise administering any the compound or a salt thereof of the present disclosure mentioned above or any the pharmaceutical composition of the present disclosure mentioned above to a subject in need thereof, but it is not limited thereto.

Alternatively, the present disclosure may also provide a method for *in vitro* degrading transactive response DNA-binding protein-43 and/or inhibiting of the activity of transactive response DNA-binding protein-43, and this method may comprise *in vitro* contacting and/or reacting any the compound or a salt thereof of the present disclosure mentioned above or any the pharmaceutical composition of the present disclosure mentioned above with the transactive response DNA-binding protein-43 and an E3 ubiquitin ligase, but it is not limited thereto.

In addition, similarly based on the aforementioned, the present disclosure may further provide a pharmaceutical composition for treating and/or preventing a disease associated with transactive response DNA-binding protein-43 accumulation. This pharmaceutical composition for treating and/or preventing a disease associated with transactive response DNA-binding protein-43 accumulation may comprise any the compound or a salt thereof of the present disclosure mentioned above or any the pharmaceutical composition of the present disclosure mentioned above, but it is not limited thereto.

The present disclosure may also provide a method for treating and/or preventing a disease associated with transactive response DNA-binding protein-43 accumulation, and this method may comprise, but is not limited to, administering any the compound or a salt thereof of the present disclosure mentioned above or any the pharmaceutical composition of the present disclosure mentioned above to a subject in need thereof.

The related description for the disease associated with transactive response DNA-binding protein-43 accumulation mentioned herein is as stated above, and thus is not repeated herein.

With regard to the subject in need of any the compound or a salt thereof of the present disclosure mentioned above or any the pharmaceutical composition of the present disclosure mentioned above, the related description thereof can refer to the related description for the subject in the foregoing content regarding the pharmaceutical composition of the present disclosure, and thus is not repeated herein.

### EXAMPLES

### A. Preparation of compounds

### A-1. Compound abbreviation

EtOH: ethanol;
DCM: dichloromethane;
DIPEA: N,N-diisopropylethylamine;
DMF: dimethylformamide;
DMSO: dimethyl sulfoxide;
T3P: 1-propanephosphonic acid cyclic anhydride;
Et3N: triethylamine;
TFA: trifluoroacetic acid;
THF: tetrahydrofuran.

### A-2. Preparation of compounds of class I

### Compounds of class I have a structure in which an E3 ubiquitin ligase binding domains is linked at position 6 of the quinoline skeleton via a linker.

### (1) Method A

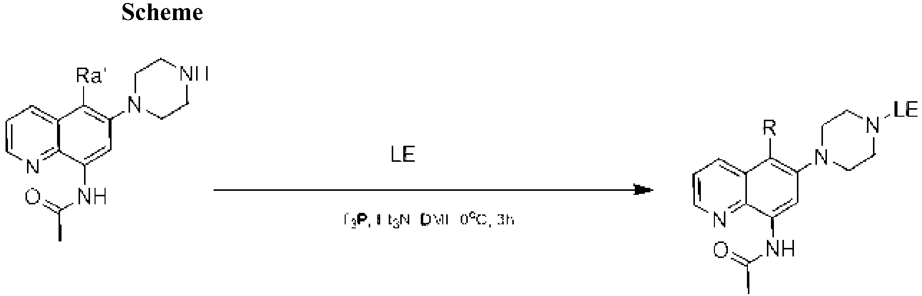

Ra' is NO₂, H, CN, N(CH₃)₂ or

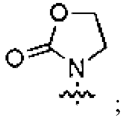

LE:

| | |
|---|---|
| LE-012 | 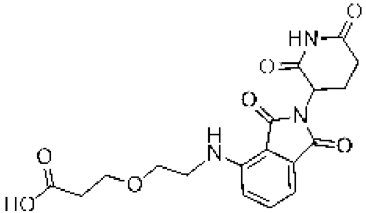 |
| LE-014 | 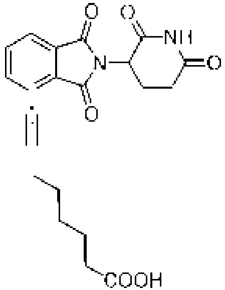 |
| LE-016 | 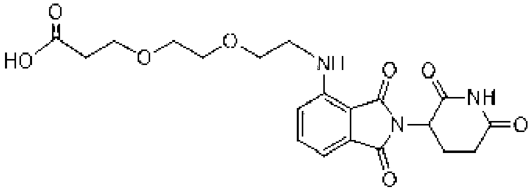 |
| LE-017 | 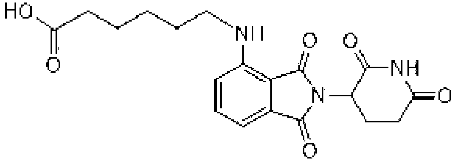 |
| LE-018 | 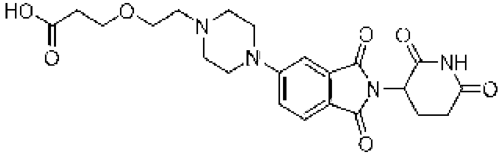 |
| LE-035 | 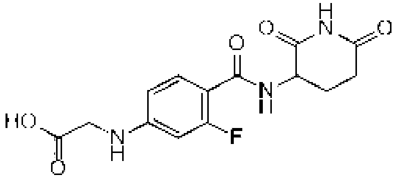 |
| LE-036 | 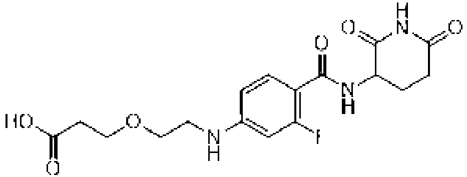 |
| LE-042 | 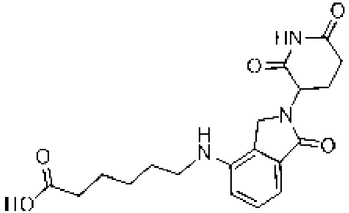 |

### Preparation example 1

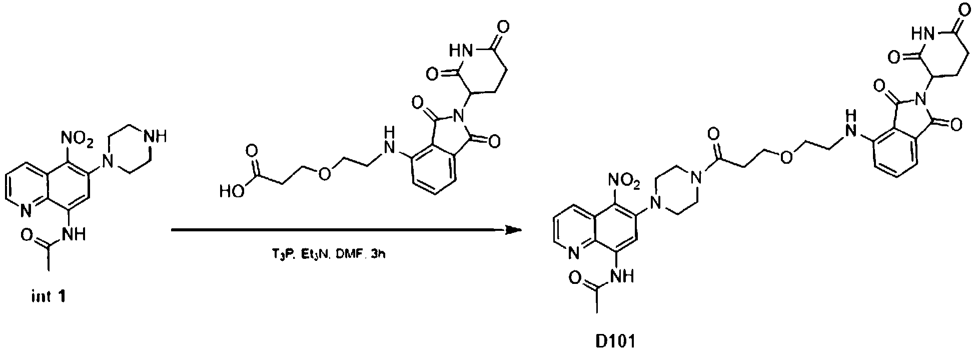

### Preparation of Compound D101

64 mg of int-1, i.e., N-(5-nitro-6-(piperazin-1-yl)quinolin-8-yl)acetamide, was dissolved in 1 mL of DMF. Next, 1 eq of pomalidomide-PEG1-C₂-COOH, 3 eq of Et₃N, and 1.5 eq of T₃P were added thereto and stirred at room temperature for 3 hours to perform the reaction. After the reaction was completed, the reacted mixture was added to water and extracted with DCM. After that, the DCM layer was taken out, dried and concentrated, and then it was purified by a column to afford 93 mg of Compound **D101** (67.7% yield).

### (2) Method B

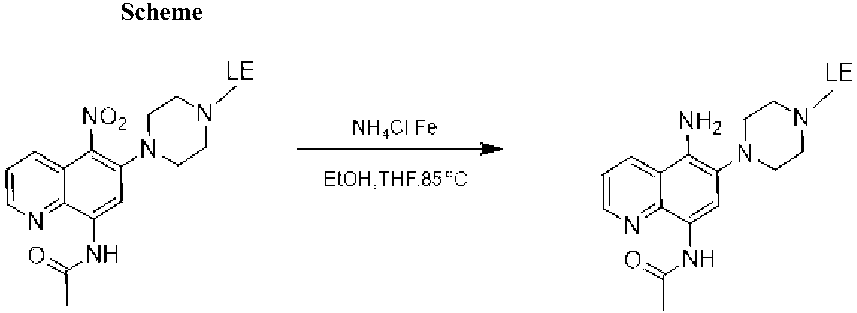

### Preparation example 2

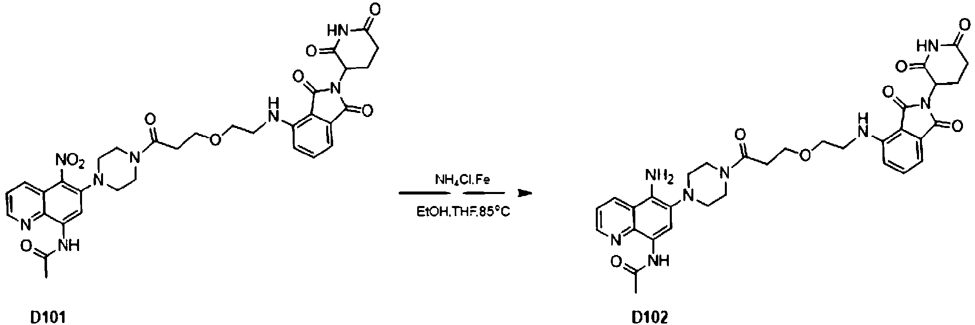

### Preparation of Compound D102

53 mg of Compound **D101** was dissolved in EtOH/THF. Next, 3 eq of Fe and 1.5 eq of NH₄Cl was added to thereto, and stirred at 85°C for 16 hours to carry out the reaction. After the reaction was completed, the reacted mixture was filtered. The obtained filtrate was concentrated to dryness, and then extracted with DCM/H₂O. After that, the DCM layer was taken out, dried and concentrated, and then it was purified by a column to afford 36 mg of Compound **D102** (70.1% yield).

### (3) Method C

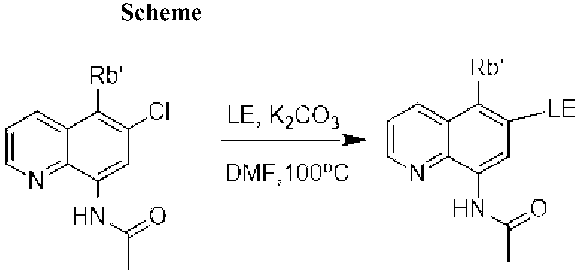

Rb' is NO₂ or NHAc;

LE:

| | |
|---|---|
| LE-013 | 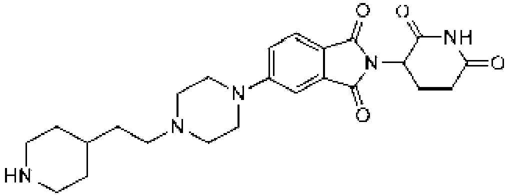 |
| LE-023 | 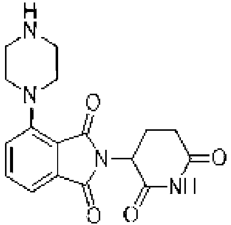 |
| LE-024 | 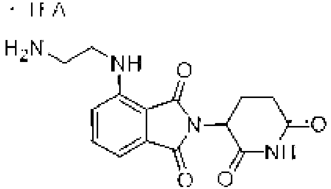 |
| LE-029 | 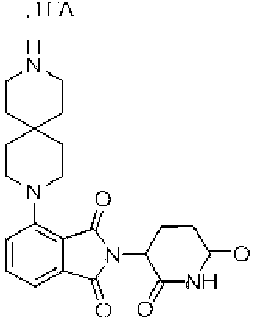 |
| LE-033 | 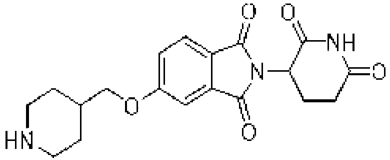 |
| LE-034 | 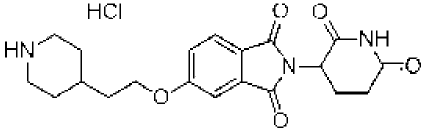 |
| LE-043 | 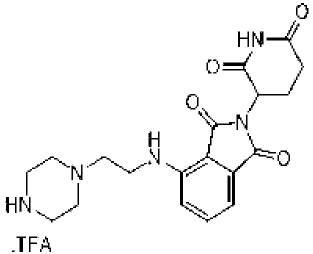 |
| LE-044 | 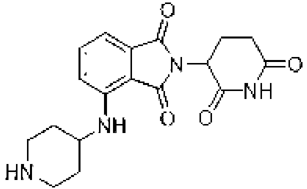 |

### Preparation example 3

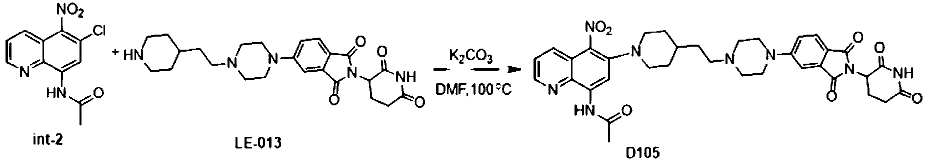

### Preparation of Compound D105

27 mg of **int-2,** i.e., 8-acetylamino-6-chloro-5-nitroquinoline, was dissolved in 2 mL of DMF. Next, 1.5 eq of LE-013, i.e., 2-(2,6-dioxopiperidin-3-yl)-5-(4-(2-(piperidin-4-yl)ethyl)piperazin-1-yl)isoindoline-1,3-dione, and 4.5 eq of K₂CO₃ were added thereto and stirred at 85°C for 16 hours to carry out the reaction. After the reaction was completed, the reacted mixture was added to water, stirred and filtered. After filtration, the solid was taken and extracted with DCM/H₂O. After that, the DCM layer was taken out, dried and concentrated, and then it was purified by a column to afford 56 mg of Compound **D105** (82.0% yield).

### (4) Method D

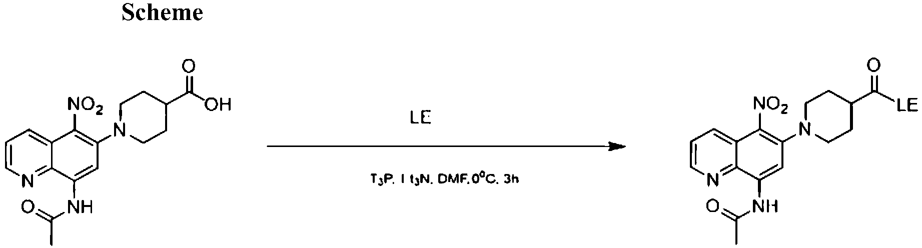

LE:

| | |
|---|---|
| LE-019 | 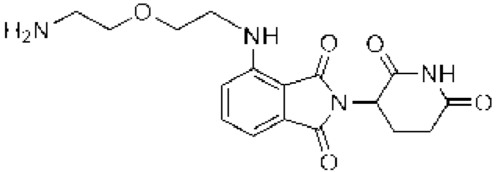 |
| LE-023 | 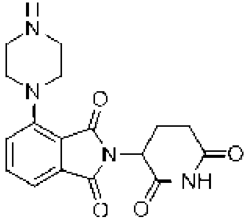 |
| LE-024 | 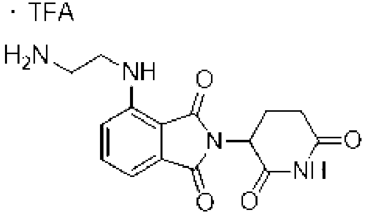 |

### Preparation example 4

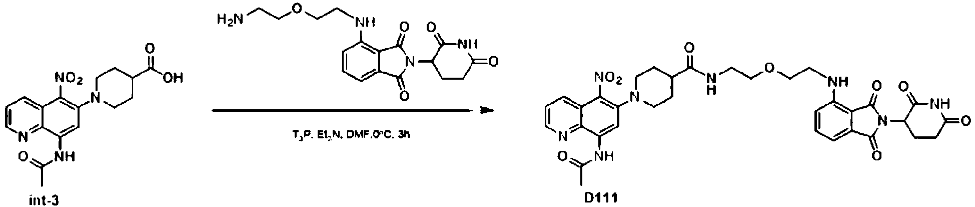

### Preparation of Compound D111

72 mg of **int-3,** i.e., 1-(8-acetamido-5-nitroquinolin-6-yl)piperidine-4-carboxylic acid, was dissolved in 1 mL of DMF. Next, 1 eq of Thalidomide-NH-PEG1-NH₂, 3 eq of Et₃N and 1.5 eq of T₃P were added thereto and stirred at room temperature for 3 hours to perform the reaction. After the reaction was completed, the reacted mixture was added to water and extracted with DCM. After that, the DCM layer was taken out, dried and concentrated, and then it was purified by a column to afford 66 mg of Compound **D111** (47.1% yield).

### (5) Method E

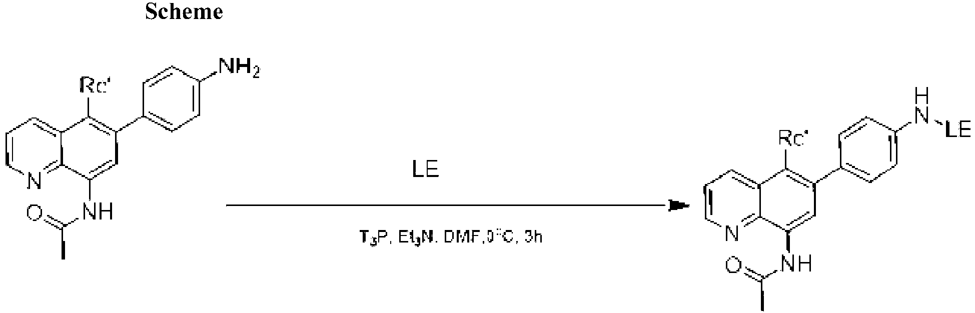

Rc' is NO₂

LE:

| | |
|---|---|
| LE-014 | 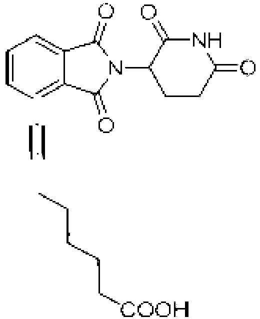 |
| LE-025 | 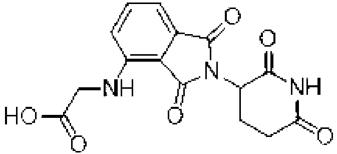 |

### Preparation example 5

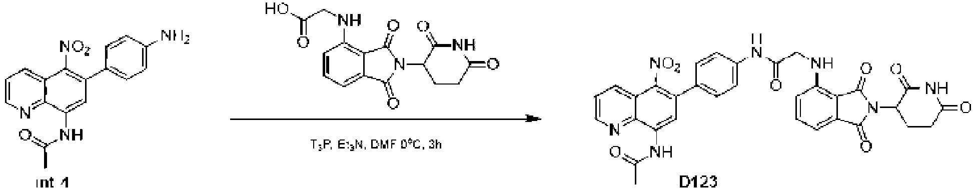

### Preparation of Compound D123

88 mg of **int-4,** i.e., *N*-(6-(4-aminophenyl)-5-nitroquinolin-8-yl)acetamide, was dissolved in 1 mL of DMF. Next, 1 eq of Thalidomide-NH-CH₂-COOH, 3 eq of Et₃N and 1.5 eq of T₃P were added thereto and stirred at room temperature for 3 hours to perform the reaction. After the reaction was completed, the reacted mixture was added to water and extracted with DCM. After that, the DCM layer was taken out, dried and concentrated, and then it was purified by a column to afford 64 mg of Compound **D123** (50.3% yield).

### (6) Method F

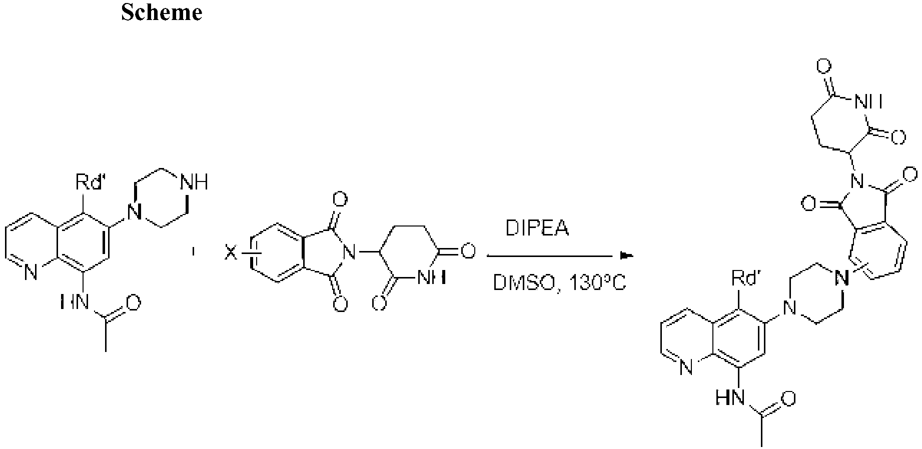

Rd' is CN, N(CH₃)₂, H or 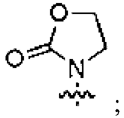
X is F or Br.

### Preparation example 6

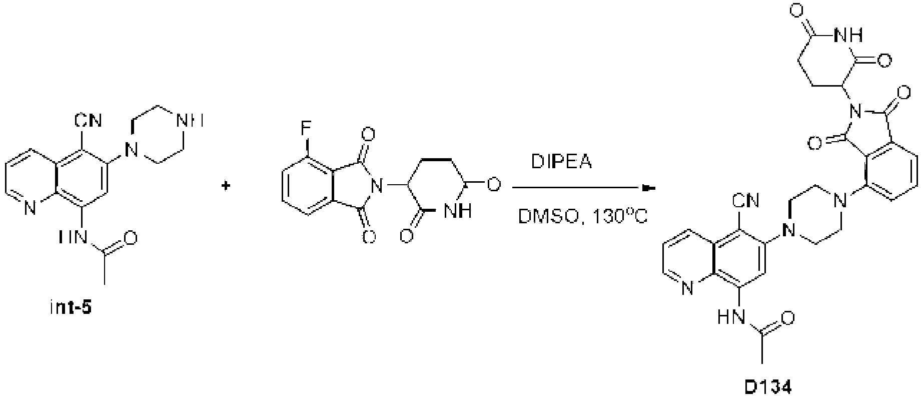

### Preparation of Compound D134

27 mg of **int-5,** i.e., *N*-(5-cyano-6-(piperazin-1-yl)quinolin-8-yl)acetamide, was dissolved in 2 mL of DMSO. Next, 1.0 eq of Thalidomide 4-fluoride, and 3.0 eq of DIPEA were added thereto and stirred at 130°C for 3 hours to carry out the reaction. After the reaction was completed, the reacted mixture was added to water, stirred and filtered. After filtration, the solid was taken and extracted with DCM and H₂O. After that, the DCM layer was taken out, dried and concentrated, and then it was purified by a column to afford 32 mg of Compound **D134** (31.7% yield).

In this example, **Compounds D101 to D151** were prepared. The structures of **Compounds D101 to D151** are shown in Table 8 below, and the methods respectively used to prepare **Compounds D101 to D151,** as well as the respective proton nuclear magnetic resonance (HNMR) analysis results and liquid chromatography-mass spectrometry (LCMS) analysis results for **Compounds D101 to D151,** are shown in Table 9.

**Table 8**

| **Compound number** | **Structure** |
|---|---|
| **D101** | 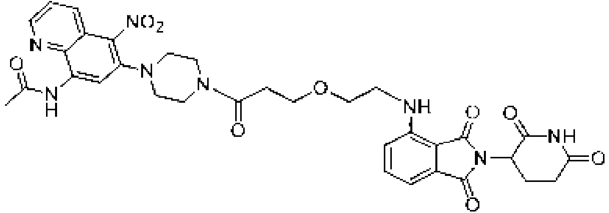 |
| **D102** | 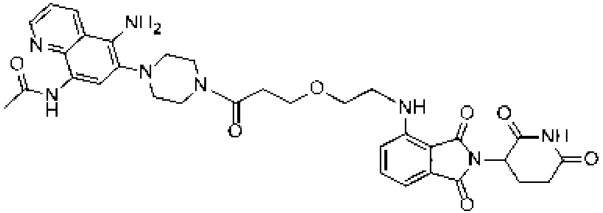 |
| **D103** | 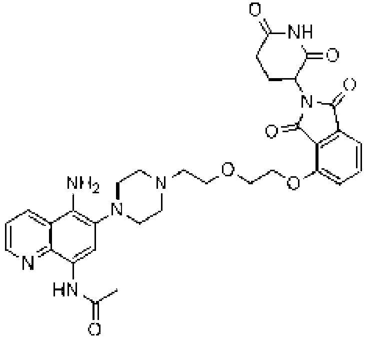 |
| **D104** | 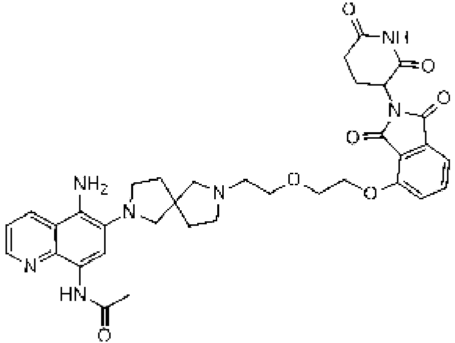 |
| **D105** | 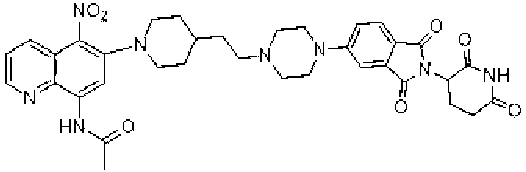 |
| **D106** | 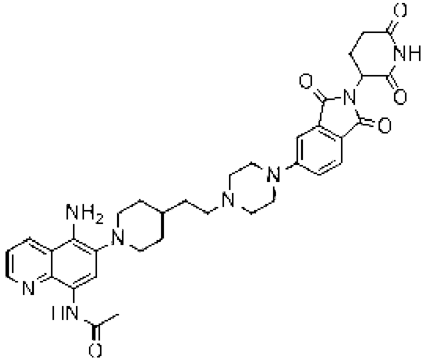 |
| **D107** | 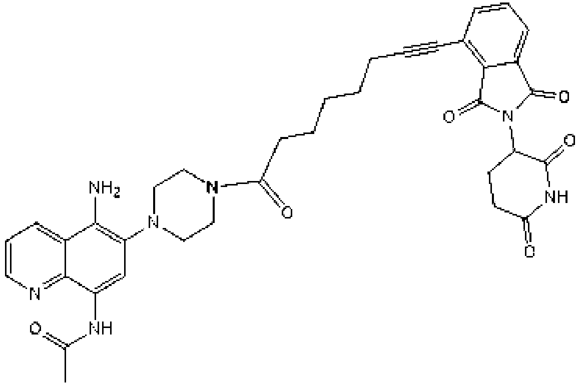 |
| **D108** | 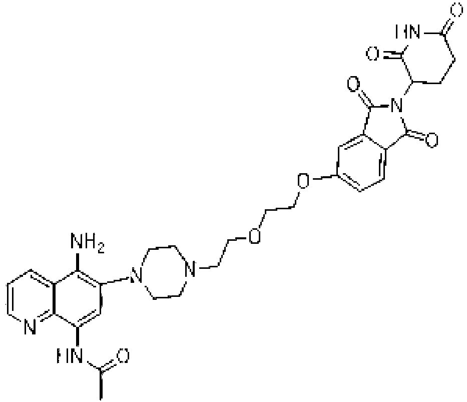 |
| **D109** | 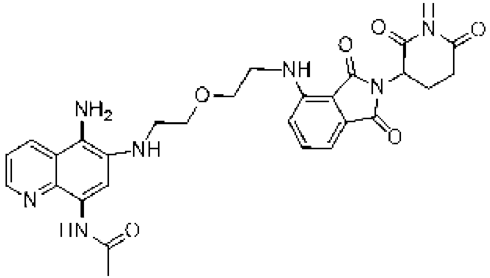 |
| **D110** | 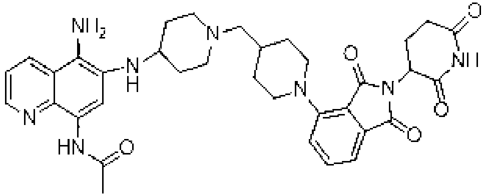 |
| **D111** | 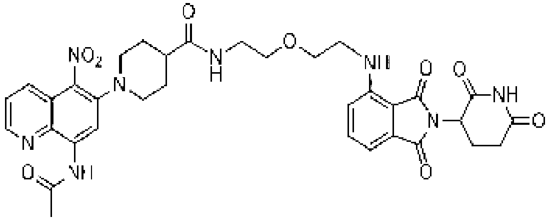 |
| **D112** | 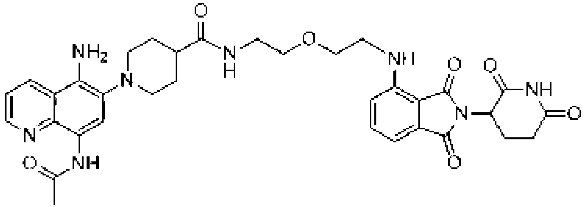 |
| **D113** | 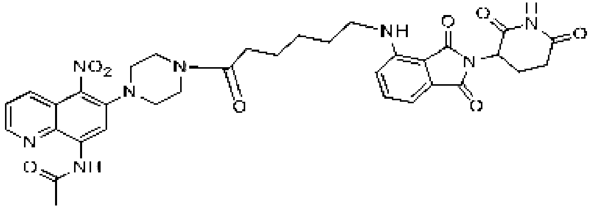 |
| **D114** | 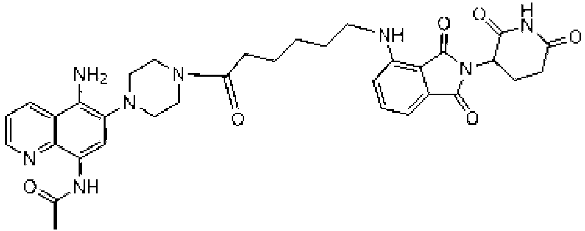 |
| **D115** | 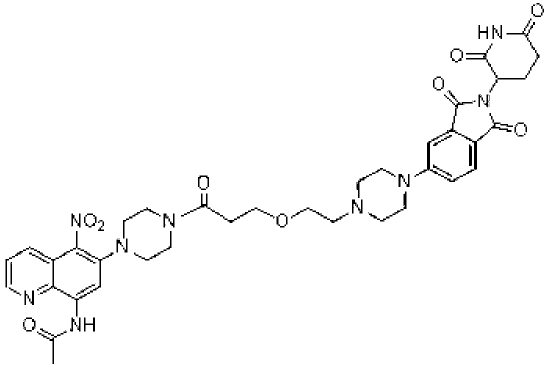 |
| **D116** | 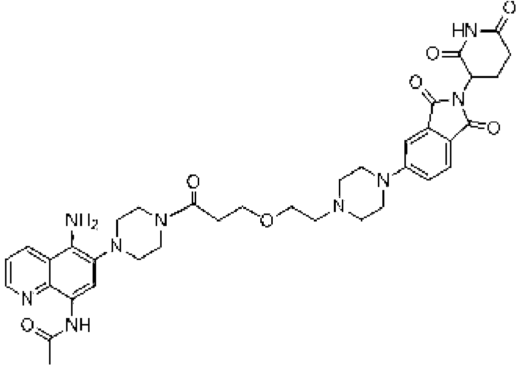 |
| **D117** | 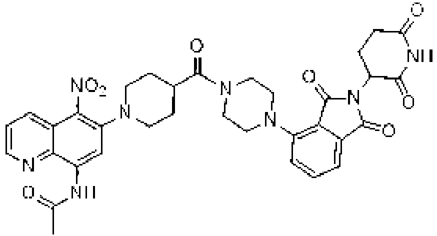 |
| **D118** | 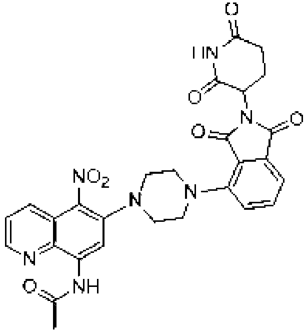 |
| **D119** | 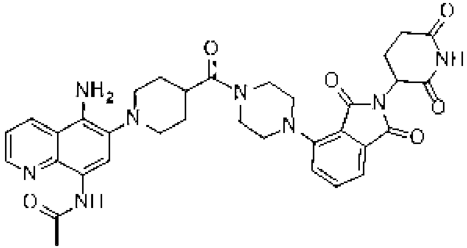 |
| **D120** | 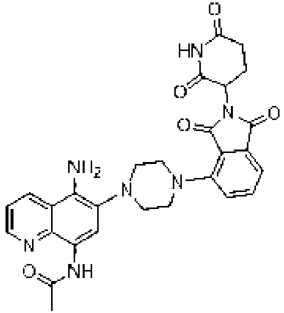 |
| **D121** | 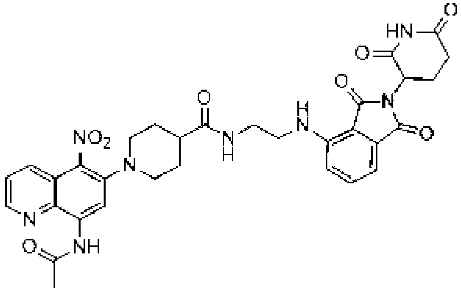 |
| **D122** | 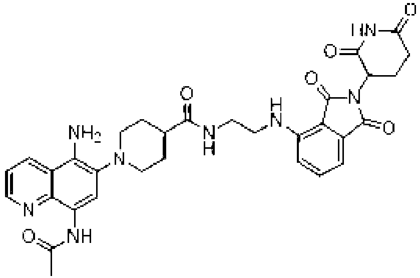 |
| **D123** | 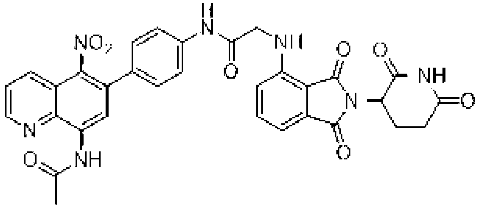 |
| **D124** | 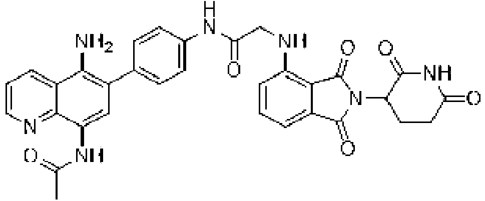 |
| **D125** | 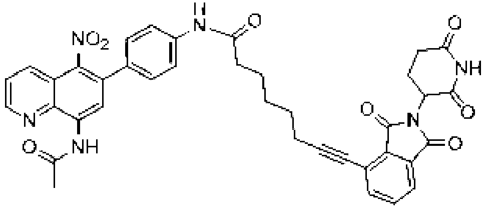 |
| **D126** | 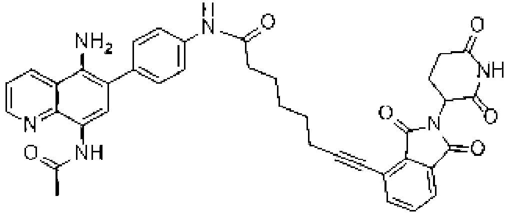 |
| **D127** | 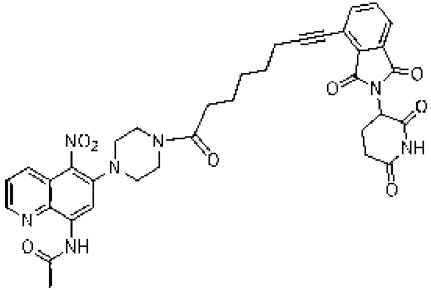 |
| **D128** | 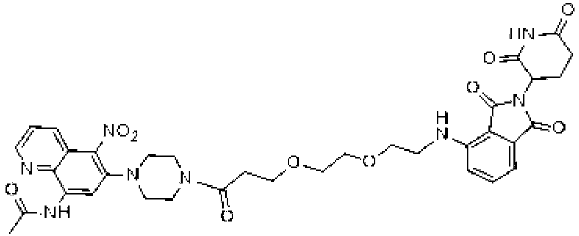 |
| **D129** | 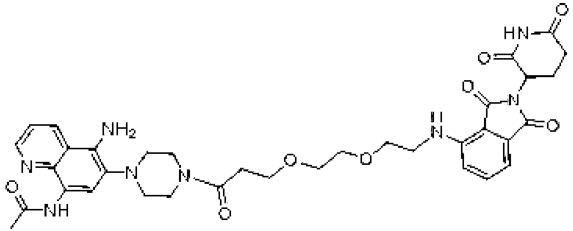 |
| **D130** | 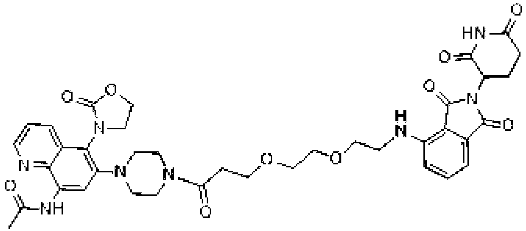 |
| **D131** | 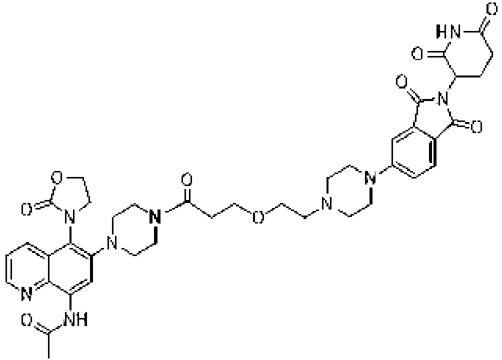 |
| **D132** | 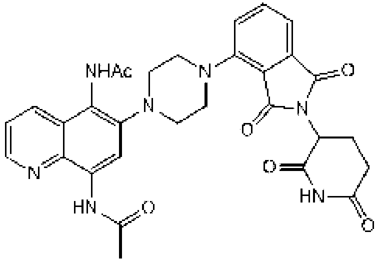 |
| **D133** | 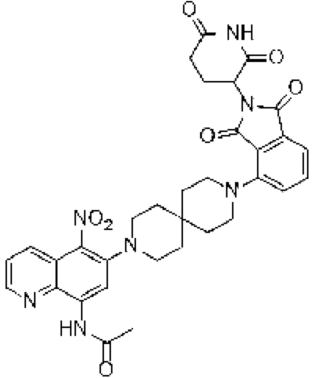 |
| **D134** | 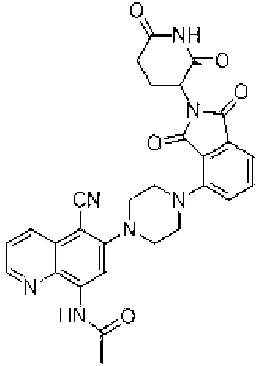 |
| **D135** | 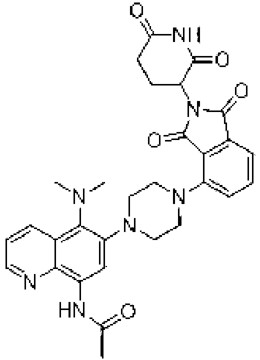 |
| **D136** | 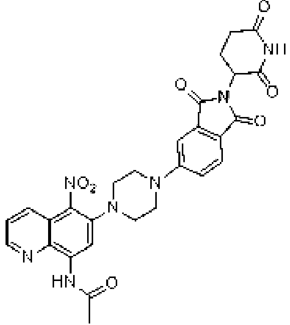 |
| **D137** | 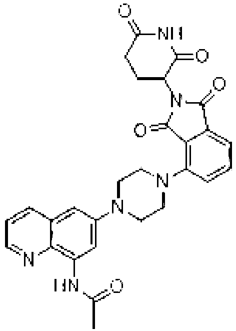 |
| **D138** | 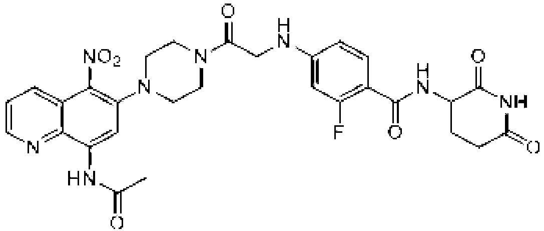 |
| **D139** | 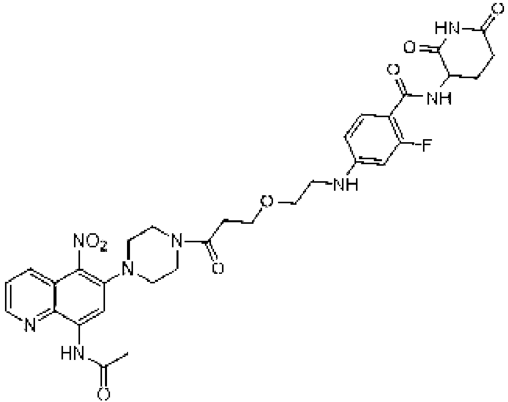 |
| **D140** | 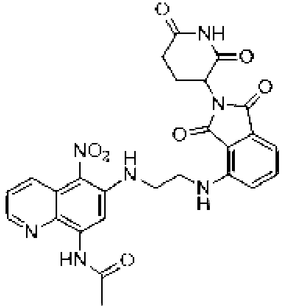 |
| **D141** | 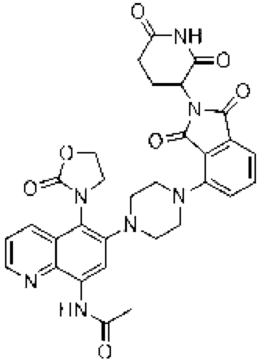 |
| **D142** | 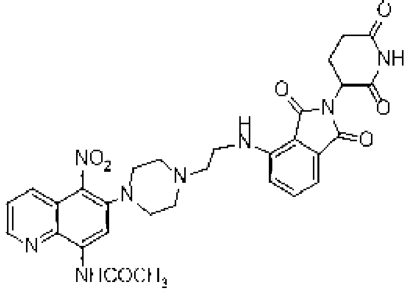 |
| **D143** | 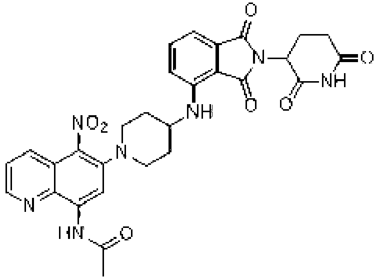 |
| **D144** | 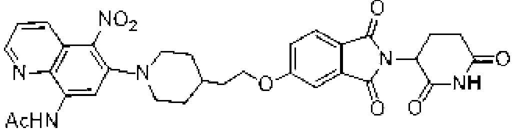 |
| **D145** | 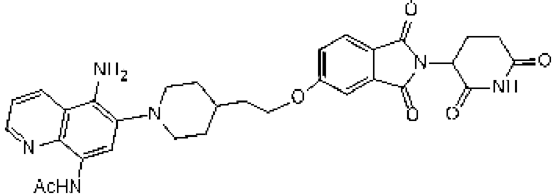 |
| **D146** | 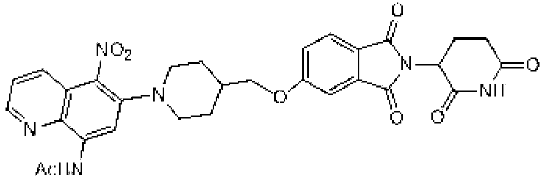 |
| **D147** | 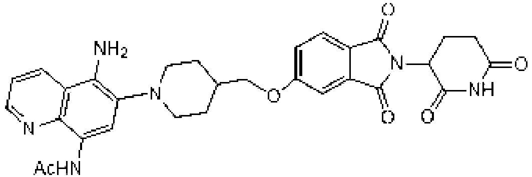 |
| **D148** | 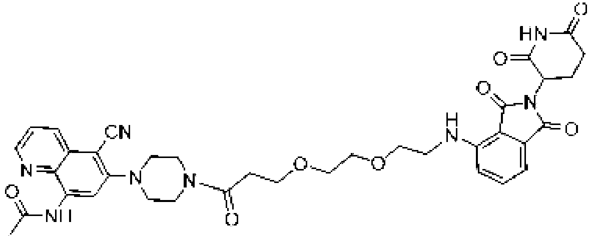 |
| **D149** | 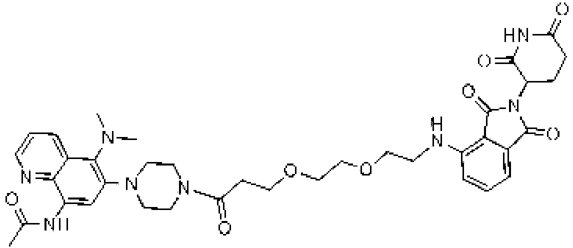 |
| **D150** | 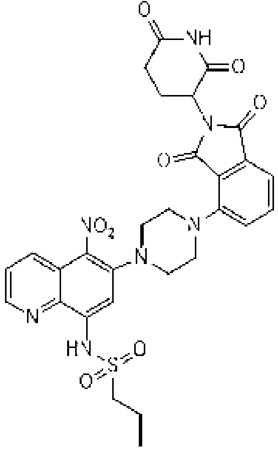 |
| **D151** | 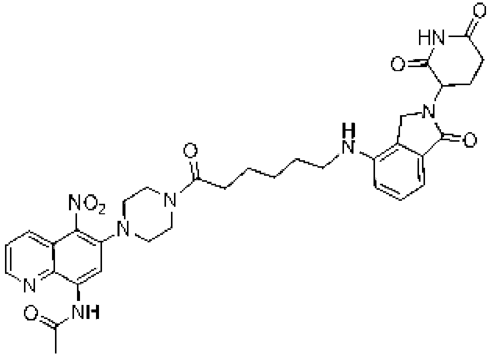 |

**Table 9**

| **Compound number** | **HNMR** | **LCMS** | **Method** |
|---|---|---|---|
| **D101** | ¹H NMR (500 MHz, CDCl₃): δ 9.92 (s, 1H), 8.73 (s, 1H), 8.72-8.71(m, 1H), 8.50(s, 1H), 8.24(d, *J* =7.5 Hz, 1H), 7.58 - 7.56 (m, 1H), 7.48 (t, *J* =8.5 , 1H), 7.08(d, *J* =7.0 Hz, 1H), 6.91(d, *J* =9.0 Hz, 1H), 6.53(s, 1H), 4.93 (dd, *J =* 12.0, 5.0 Hz, 1H), 3.87 (t, *J =* 6.5 Hz, 2H), 3.81 (t, *J =* 5.0 Hz, 2H), 3.73 (t, *J =* 5.5 Hz, 2H), 3.70 - 3.69 (m, 2H), 3.49 - 3.46 (m, 2H), 3.30 - 3.25 (m, 4H),2.86 - 2.74 (m, 3H), 2.71- 2.67 (m, 2H), 2.52 - 2.40 (m, 4H),2.15-2.12(m, 1H). | MS-ESI m/z: 687.7 [M+H]+. | **A** |
| **D102** | ¹H NMR (500 MHz, CDCl₃): δ 9.57 (s, 1H), 8.75 (s, 1H), 8.69(s, 2H), 8.20(d, *J* =9.0 Hz, 1H), 7.48 (t, *J* =8.5 , 1H), 7.43 (dd, *J =* 8.5, 4.5 Hz, 1H), 7.07(d, *J* =7.0 Hz, 1H), 6.92 (d, *J* =8.5 Hz, 1H), 6.53(s, 1H), 4.90 (dd, *J =* 11.5, 5.0 Hz, 1H), 4.47(s, 2H), 3.89 (t, *J =* 5.5 Hz, 4H), 3.76 - 3.71 (m, 4H), 3.01 - 2.96 (m, 2H), 2.89 (s, 2H), 2.85 (s, 2H), 2.81- 2.65 (m, 5H), 2.32 (s, 3H), 2.18-2.08(m, 1H). | MS-ESI *m*/*z:* 656.8 [M+H]+. | **B** |
| **D103** | ¹H NMR (500 MHz, CDCl₃) : δ 9.54 (s, 1H), 8.75 (s, 1H), 8.71(s, 1H), 8.19(d, *J* =8.5 Hz, 1H), 8.06 (s, 1H), 7.79(d, *J* =8.0 Hz, 1H), 7.40(s, 2H), 7.24(s, 1H), 4.95 (dd, *J =* 12.0, 4.0 Hz, 1H), 4.47(s, 2H), 4.29(s, 2H), 3.91(s, 2H), 3.77(s, 2H), 3.05(s, 4H),2.91- 2.74 (m, 9H), 2.31 (s, 3H), 2.18-2.13(m, 1H). | MS-ESI *m*/*z*: 630.7 [M+H]+. | **B** |
| **D104** | ¹H NMR (500 MHz, CDCl₃): δ 9.94 (s, 1H), 8.61 (s, 1H), 8.53(d, *J* =4.5 Hz, 1H), 8.45 (d, *J* =8.5 Hz, 1H), 7.63- 7.60 (m, 1H), 7.51- 7.49 (m, 1H), 7.43- 7.41 (m, 1H), 7.24- 7.22 (m, 2H), 4.95 (dd, *J =* 12.0, 6.5 Hz, 1H), 4.33(s, 2H), 3.90(s, 2H), 3.76(s, 2H), 3.49(s, 2H), 3.48 - 3.37 (m, 1H), 3.35 - 3.22 (m, 1H), 2.81 - 2.66 (m, 11H), 2.37(s, 3H), 2.17-2.11(m, 1H). | MS-ESI *m*/*z:670.0* [M+H]+. | **B** |
| **D105** | ¹H NMR (500 MHz, CDCl₃): δ 9.90(s, 1H), 8.71 (s, 1H), 8.63(d, *J* =4.5 Hz, 1H), 8.37(s, 1H), 8.31(d, *J* =8.5 Hz, 1H), 7.70(d, *J* =8.5 Hz, 1H), 7.54 -7.51 (m, 1H), 7.30(s, 1H), 7.08 - 7.06 (m, 1H), 4.95 (dd, *J =* 12.5, 5.5 Hz, 1H), 3.52 - 3.45 (m, 2H), 3.44 (s, 4H), 3.09 - 3.04 (m, 2H), 2.91 - 2.74 (m, 3H), 2.62 (s, 4H), 2.61-2.45(m, 1H), 2.38 (s, 3H), 2.15-2.12(m, 1H), 1.83-1.75(m, 4H), 1.57-1.53(m, 2H), 1.47-1.42(m, 2H) . | MS-ESI *m*/*z:*683.7 [M+H]+. | **C** |
| **D106** | ¹H NMR (500 MHz, CDCl₃): δ 9.57(s, 1H), 8.74 (s, 1H), 8.72(s, 1H), 8.18(d, *J* =7.5 Hz, 1H), 8.02(s, 1H), 7.71(d, *J* =5.0 Hz, 1H), 7.39(d, *J* =4.0 Hz, 1H), 7.31(s, 1H), 7.08(d, *J* =6.5Hz, 1H), 4.93 (dd, *J =* 12.0, 5.0 Hz, 1H), 3.49 (s, 4H), 3.12 - 3.11 (m, 2H), 2.81 - 2.70 (m, 10H), 2.65 (s, 4H), 2.52 (s, 2H), 2.32 (s, 3H), 2.15-2.13(m, 1H), 1.89-1.86(m, 2H) . | MS-ESI *m*/*z:653.9* [M+H]+. | **B** |
| **D107** | ¹H NMR (500 MHz, CDCl₃): δ 9.57 (s, 1H), 8.75 (s, 1H), 8.69(s, 2H), 8.21 (d, *J* =8.0 Hz, 1H), 7.79- 7.76 (m, 1H), 7.72- 7.66 (m, 2H), 7.47- 7.41 (m, 1H), 4.98 (dd, *J =* 12.0, 6.5 Hz, 1H), 3.67 (s, 2H), 2.96 (s, 4H), 2.89 (s,4H), 2.82 - 2.76 (m, 2H),2.57 - 2.55 (m, 2H), 2.45 - 2.34 (m, 3H), 2.32(s, 3H), 2.18-2.17(m, 1H), 1.69-1.58(m, 6H) . | MS-ESI *m*/*z*:664.7 [M+H]+. | **B** |
| **D108** | ¹H NMR (500 MHz, CDCl₃): δ 9.54 (s, 1H), 8.75 (s, 1H), 8.72(s, 1H), 8.20(d, *J* =8.5 Hz, 1H), 8.06 (s, 1H), 7.80(d, *J* =8.0 Hz, 1H), 7.40 (s, 2H), 7.26- 7.24 (m, 1H), 4.95 (dd, *J =* 12.0, 4.0 Hz, 1H), 4.46 (s, 2H), 4.29 (s, 2H), 3.91 (s, 2H), 3.77 (s, 2H), 3.05(s, 4H), 2.91 - 2.62 (m, 9H), 2.31(s, 3H), 2.18-2.13(m, 1H). | MS-ESI *m*/*z:*631.0 [M+H]+. | **B** |
| **D109** | ¹H NMR (500 MHz, CDCl₃): δ 9.97 (s, 1H), 8.91(s, 1H), 8.83(d, *J* =8.5 Hz, 1H), 8.77(d, *J* =4.0 Hz, 1H), 8.65(s, 2H), 7.55- 7.54 (m, 1H), 7.39 (t, *J =* 7.5 Hz, 1H), 7.03(d, *J* =7.0Hz, 1H), 6.76(d, *J* =8.5Hz, 1H), 6.38(d, *J* =5.0Hz, 1H), 6.36 (s, 1H), 4.85 (dd, *J* = 12.0, 6.5 Hz, 1H), 4.46 (s, 2H), 3.89 (t, *J* = 5.0 Hz, 2H), 3.80 (t, *J =* 5.5 Hz, 2H), 3.80 (t, *J* = 5.5 Hz, 2H), 3.19 - 3.15 (m, 2H), 2.89 - 2.87 (m, 1H), 2.77 - 2.70 (m, 2H), 2.36(s, 3H), 2.15-2.13(m, 1H). | MS-ESI *m*/*z*:560.7 [M+H]+. | **B** |
| **D110** | ¹H NMR (500 MHz, CDCl₃): δ 9.99 (s, 1H), 9.20(s, 1H), 8.85(d, *J* =8.0 Hz, 1H), 8.7 9 (s, 1H), 8.10(s, 1H), 7.61- 7.55 (m, 2H), 7.38 (d, *J =* 7.5 Hz, 1H), 7.21(d, *J* =9.0 Hz, 1H), 4.98 (dd, *J* = 12.0, 6.5 Hz, 1H), 4.36 (s, 2H), 3.89-3.60(m, 3H), 3.22 - 3.15 (m, 3H), 2.97 - 2.81 (m, 4H), 2.78 - 2.64 (m, 3H), 2.40(s, 3H), 2.37-2.27(m, 1H) 2.24 - 1.78 (m, 8H), 1.56-1.49(m, 2H). | MS-ESI *m*/*z:* 653.5 [M+H]+. | **B** |
| **D111** | ¹H NMR (500 MHz, CDCl₃): δ 9.91 (s, 1H), 8.88 (s, 1H), 8.70 (s, 1H), 8.67(d, *J* =4.0 Hz, 1H), 8.25(d, *J* =9.0 Hz, 1H), 7.54- 7.50 (m, 2H), 7.12(d, *J* =7.0 Hz, 1H), 6.94(d, *J* =8.5 Hz, 1H), 6.58 (t, *J =* 5.5, 1H), 6.24 (t, *J =* 5.0, 1H), 4.95 (dd, *J =* 12.0, 5.0 Hz, 1H), 3.73-3.63(m, 2H), 3.60-3.50(m, 2H), 3.49-3.45(m, 6H), 3.01-2.95(m, 2H), 2.90-2.75(m, 3H), 2.38(s, 3H), 2.39-2.25(m, 1H), 2.17-2.13(m, 1H), 1.92-1.88(m, 4H). | MS-ESI *m*/*z*:701.7 [M+H]+. | **D** |
| **D112** | ¹H NMR (500 MHz, CDCl₃): δ 9.62 (s, 2H), 8.71 (s, 1H), 8.68 (s, 1H), 8.21 (d, *J* =9.0 Hz, 1H), 7.60- 7.57 (m, 1H), 7.43 - 7.36 (m, 1H), 7.11(d, *J* =7.0 Hz, 1H), 6.93(d, *J* =8.5 Hz, 1H), 6.66 (s, 1H), 6.42 (s, 1H), 4.95 (dd, *J* = 12.0, 5.0 Hz, 1H), 4.45(s,2H), 3.73-3.63(m, 2H), 3.60-3.51(m, 2H), 3.50-3.44(m, 4H), 3.11-3.07(m, 2H), 2.93-2.64(m, 5H), 2.34(s, 3H), 2.32-2.23(m, 1H), 2.14-2.00(m, 1H), 1.96-1.86(m, 2H), 1.68-1.43(m, 4H). | MS-ESI *m*/*z*:671.8 [M+H]+. | **B** |
| **D113** | ¹H NMR (500 MHz, CDCl₃): δ 9.92 (s, 1H), 8.74 (s, 2H), 8.24(d, *J* =7.5 Hz, 1H), 8.23(s, 1H), 7.60- 7.57 (m, 1H),7.51(d, *J* =8.0 Hz, 1H), 7.10(d, *J* =7.0 Hz, 1H), 6.90(d, *J* =8.5 Hz, 1H), 6.26 (s, 1H), 4.92 (dd, *J =* 12.0, 5.0 Hz, 1H), 3.65 (s, 2H), 3.63(s, 2H), 3.51 (s, 2H), 3.34-3.23 (m, 6H), 2.92 - 2.74 (m, 3H), 2.41 - 2.39 (m, 2H), 2.38(s, 3H), 2.15-2.13(m, 1H), 1.75-1.74(m, 4H). | MS-ESI *m*/*z*:685.6 [M+H]+. | **A** |
| **D114** | ¹H NMR (500 MHz, CDCl₃): δ 9.57 (s, 1H), 8.74 (s, 1H), 8.69 (s, 1H), 8.43 (s, 1H), 8.22(d, *J* =8.5 Hz, 1H), 7.43 - 7.36 (m, 2H), 7.09 (d, *J* =7.5 Hz, 1H), 6.91(d, *J* =8.5 Hz, 1H), 6.26 (s, 1H), 4.92 (dd, *J =* 12.0, 5.0 Hz, 1H), 4.46 (s, 2H), 3.63(s, 2H), 3.34- 3.25 (m, 2H), 3.01 (s, 2H), 2.79 (s, 2H), 2.72 - 2.69 (m, 3H), 2.43 - 2.41 (m, 2H), 2.32(s, 3H), 2.14-2.13(m, 1H), 1.76-1.63(m, 6H), 1.54-1.50(m, 2H). | MS-ESI *m*/*z:655.7* [M+H]+. | **B** |
| **D115** | ¹H NMR (500 MHz, CDCl₃): δ 9.90 (s, 1H), 8.73 (s, 2H), 8.23(d, *J* =9.0 Hz, 1H), 7.99 (s, 1H), 7.65(d, *J* =8.5 Hz, 1H), 7.59-7.56(m, 1H), 7.27-7.25(m, 1H), 7.02 (d, *J* =9.0 Hz, 1H), 4.95 (dd, *J =* 11.5, 5.5 Hz, 1H), 3.84-3.81 (m, 4H), 3.69- 3.68 (m, 4H), 3.66- 3.43 (m, 4H), 3.29- 3.25 (m, 4H), 2.92 - 2.74 (m, 3H), 2.73 - 2.67 (m, 8H), 2.38(s, 3H), 2.18-2.13(m, 1H). | MS-ESI *m*/*z*: 756.7 [M+H]+. | **A** |
| **D116** | ¹H NMR (500 MHz, CDCl₃): δ 9.56 (s, 1H), 8.76 (s, 1H), 8.75 (s, 1H), 8.22-8.20(m, 1H), 8.03 (s, 1H), 7.67(d, *J* =8.5 Hz, 1H),7.44-7.41(m, 1H), 7.27-7.25(m, 1H), 7.03 (d, *J* =8.5 Hz, 1H), 4.94 (dd, *J =* 12.0, 5.5 Hz, 1H), 4.45 (s, 2H), 3.85- 3.83 (m, 4H), 3.70- 3.68 (m, 4H), 3.50- 3.43 (m, 4H),3.05- 2.97 (m, 4H), 2.92 - 2.69 (m, 11H), 2.39(s, 3H), 2.16-2.12(m, 1H). | MS-ESI *m*/*z*: 726.7 [M+H]+. | **B** |
| **D117** | ¹H NMR (500 MHz, CDCl₃): δ 9.56 (s, 1H), 8.76 (s, 1H), 8.67(d, *J* =4.0 Hz, 1H), 8.30(d, *J* =7.5 Hz, 1H), 8.00 (s, 1H), 7.67 - 7.64 (m, 1H), 7.59 - 7.53 (m, 1H), 7.48(d, *J* =7.5 Hz, 1H), 7.19(d, *J* =8.0 Hz, 1H), 4.99 (dd, *J =* 17.5, 7.0 Hz, 1H), 3.89 - 3.72 (m, 4H), 3.54 - 3.52 (m, 2H),3.38 - 3.31 (m, 2H), 3.15 (s, 2H), 3.13 - 3.11 (m, 2H), 2.94 - 2.75 (m, 4H), 2.38 (s, 3H), 2.17-2.16(m, 1H) , 2.16-2.15(m, 2H) , 2.14-2.13(m, 2H). | MS-ESI *m*/*z*: 683.7 [M+H]+. | **D** |
| **D118** | ¹H NMR (500 MHz, CDCl₃): δ 9.91 (s, 1H), 8.81 (s, 1H), 8.72(d, *J* =4.0 Hz, 1H), 8.26(d, *J* =8.5 Hz, 1H), 8.04 (s, 1H), 7.66 - 7.63 (m, 1H), 7.59 - 7.56 (m, 1H), 7.46(d, *J* =7.0 Hz, 1H), 7.22(d, *J* =8.5 Hz, 1H), 4.99 (dd, *J =* 17.5, 7.0 Hz, 1H), 3.54 - 3.52 (m, 8H), 2.94 - 2.75 (m, 3H), 2.75 (s, 3H), 2.39-2.14(m, 1H). | MS-ESI *m*/*z*: 572.5 [M+H]+. | **C** |
| **D119** | ¹H NMR (500 MHz, CDCl₃): δ 9.89 (s, 1H), 8.72 (s, 1H), 8.66(d, *J* =4.5 Hz, 1H), 8.33 (s, 1H), 8.29(d, *J* =7.5 Hz, 1H), 7.64 - 7.63 (m, 1H), 7.53(d, *J* =9.0 Hz, 1H), 7.47(d, *J* =6.5 Hz, 1H), 7.18 (d, *J* =8.5 Hz, 1H) , 4.99 (dd, *J =* 17.5, 7.0 Hz, 1H), 3.89 - 3.72 (m, 4H), 3.54 - 3.52 (m, 2H),3.38 - 3.35 (m, 2H), 3.31 - 3.29 (m, 2H), 3.14 - 3.01 (m, 2H), 2.93 - 2.75 (m, 6H), 2.37 (s, 3H), 2.16-2.14(m, 1H), 2.13-2.02(m, 2H) , 1.85-1.76(m, 2H). | MS-ESI *m*/*z*: 653.7 [M+H]+. | **B** |
| **D120** | ¹H NMR (500 MHz, CDCl₃): δ 9.57 (s, 1H), 8.81 (s, 1H), 8.76(d, *J* =3.0 Hz, 1H), 8.23(d, *J* =1.5 Hz, 1H), 8.02 (s, 1H), 7.64(t, *J* =8.5 Hz, 1H), 7.48 - 7.43 (m, 2H), 7.27 - 7.26 (m, 1H), 4.99 (dd, *J =* 12.5, 5.5 Hz, 1H), 4.47(s, 2H), 3.64 (s, 4H), 3.24 (s, 4H), 2.94 - 2.73 (m, 3H), 2.33 (s, 3H), 2.17-2.14(m, 1H). | MS-ESI *m*/*z*: 542.7 [M+H]+. | **B** |
| **D121** | ¹H NMR (500 MHz, CDCl₃): δ 9.90 (s, 1H), 8.89 (s, 1H), 8.70 (s, 1H), 8.65 (d, *J* =3.5 Hz, 1H), 8.23 (d, *J* =8.0 Hz, 1H), 7.54 - 7.50 (m, 2H), 7.12 (d, *J* =7.0 Hz, 1H), 6.98(d, *J* =9.0 Hz, 1H), 6.42(t, *J =* 5.0 Hz, 1H), 6.15 (s, 1H), 4.96 (dd, *J =* 12.5, 5.5 Hz, 1H), 3.59 - 3.49(m,6H), 3.10-2.91(m,2H), 2.88-2.75(m, 3H), 2.38(s, 3H), 2.38 - 2.30(m,1H), 2.18-2.13(m, 1H), 1.94-1.26(m, 4H). | MS-ESI *m*/*z*: 657.8 [M+H]+. | **D** |
| **D122** | ¹H NMR (500 MHz, CDCl₃): δ 9.59 (s, 1H), 8.86 (s, 1H), 8.72 (s, 1H), 8.70 (s, 1H), 8.22 (d, *J* =8.0 Hz, 1H), 7.52 (t, *J* =8.0 Hz, 1H), 7.39 (d, *J* =8.5 Hz, 1H), 7.39 (d, *J* =8.5 Hz, 1H), 7.12 (d, *J* =7.5 Hz, 1H), 6.99 (d, *J* =8.5 Hz, 1H), 6.43 (s, 1H), 6.19 (s, 1H), 4.96 (dd, *J =* 12.5, 5.5 Hz, 1H), 3.49 - 3.42 (m,4H), 3.20-3.10 (m,2H), 2.81-2.71 (m, 4H), 2.33 (s, 3H), 2.28 - 2.14 (m,2H), 2.02-1.92 (m, 5H). | MS-ESI m̅/̅z̅: 627.6 [M+H]+. | **B** |
| **D123** | ¹H NMR (500 MHz, DMSO-d₆): δ 11.11 (s, 1H), 10.51 (s, 1H), 10.43 (s, 1H), 9.09 (s, 1H), 8.75 (s, 1H), 8.31 (d, *J* =8.5 Hz, 1H), 7.89- 7.48 (m, 1H), 7.76- 7.75 (m, 2H), 7.61(d, *J* =9.0 Hz, 1H), 7.42- 7.41 (m, 2H), 7.10- 6.98 (m, 3H), 5.08 (dd, *J =* 9.5, 5.0Hz, 1H), 4.25 (s, 2H), 2.90 - 2.62 (m, 3H), 2.36 (s, 3H), 2.05-2.01(m, 1H). | MS-ESI *m*/*z*: 636.5 [M+H]+. | **E** |
| **D124** | ¹H NMR (500 MHz, DMSO-d₆): δ 11.11 (s, 1H), 10.34 (s, 1H), 9.96 (s, 1H), 8.86 (s, 1H), 8.70 (s, 1H), 8.33 (d, *J* =8.5 Hz, 1H), 7.87 (s, 2H), 7.75-7.74 (m, 1H), 7.62- 7.53 (m, 1H), 7.44- 7.43 (m, 2H), 7.10- 6.98 (m, 3H), 5.11 (dd, *J =* 9.5, 5.0Hz, 1H), 4.25 (s, 2H), 3.16 -3.02 (m, 3H), 2.33 (s, 3H), 2.18-2.05(m, 1H). | MS-ESI *m*/*z:* 606.5 [M+H]+. | **B** |
| **D125** | ¹H NMR (500 MHz, DMSO-d₆): δ 11.15 (s, 1H), 10.52 (s, 1H), 10.16 (s, 1H), 9.09 (d, *J* =4.0 Hz, 1H), 8.74 (s, 1H), 8.30 (d, *J* =9.0 Hz, 1H), 7.89-7.86 (m, 2H), 7.85- 7.84 (m, 2H), 7.79- 7.71 (m, 2H), 7.37- 7.36 (m, 2H), 5.13 (dd, *J =* 12.5, 5.5Hz, 1H), 2.86-2.83 (m, 3H), 2.63-2.57 (m, 2H), 2.35 (s, 3H), 2.05-2.04(m, 1H), 1.68-1.63 (m, 5H) , 1.53-1.52 (m, 3H). | MS-ESI *m*/*z*: 701.7 [M+H]+. | **E** |
| **D126** | ¹H NMR (500 MHz, DMSO-d₆): δ 11.16 (s, 1H), 10.04 (s, 1H), 9.77 (s, 1H), 8.86 (s, 1H), 8.69 (d, *J* =8.5 Hz, 1H), 8.31 (s, 1H), 7.87 (s, 1H), 7.86-7.80 (m, 2H), 7.74- 7.72 (m, 2H), 7.51- 7.53 (m, 1H), 7.39 (d, *J* =8.5 Hz, 1H), 5.14 (dd, *J =* 12.5, 5.5Hz, 1H), 4.15 (s, 2H), 2.72-2.60 (m, 3H), 2.38-2.35 (m, 2H), 2.18-2.06(m, 1H), 1.69-1.63 (m, 5H), 1.54-1.53 (m, 3H). | MS-ESI *m*/*z:* 671.8 [M+H]+. | **B** |
| **D127** | ¹H NMR (500 MHz, CDCl₃): δ 9.91 (s, 1H), 8.75 (s, 2H) , 8.73(s, 1H), 8.21(d, *J* =8.5 Hz, 1H), 7.78- 7.75 (m, 1H), 7.71- 7.63 (m, 2H), 7.59- 7.55 (m, 1H), 4.98 (dd, *J =* 12.0, 6.5 Hz, 1H), 3.81 (s, 2H), 3.73 (s,2H), 3.30 - 3.25 (m, 4H), ,2.88 - 2.70 (m, 5H), 2.52 - 2.40 (m, 4H), 2.38 - 2.35 (m, 2H), 2.34(s, 3H), 2.15-2.13(m, 1H), 1.66-1.60(m, 2H) . | MS-ESI *m*/*z*: 694.7 [M+H]+. | **A** |
| **D128** | ¹H NMR (500 MHz, CDCl₃): δ 9.91 (s, 1H), 8.71 (s, 2H) , 8.51(s, 1H), 8.23 (d, *J* =9.0 Hz, 1H), 7.58-7.55 (m, 1H), 7.04 (d, *J* =7.0 Hz, 1H), 6.90 (d, *J* =8.5 Hz, 1H), 6.50 (s, 1H), 4.91 (dd, *J =* 12.5, 5.0 Hz, 1H), 3.83 - 3.78 (m, 6H), 3.75- 3.65 (m, 6H), 3.48 - 3.47 (m, 2H), 3.26 - 3.22 (m, 2H), 2.91 - 2.74 (m, 3H), 2.66 - 2.63 (m, 2H), 2.38 (s, 3H), 2.18-2.14(m, 1H). | MS-ESI *m*/*z:* 731.7 [M+H]+. | **A** |
| **D129** | ¹H NMR (500 MHz, CDCl₃): δ 9.56 (s, 1H), 8.78 (s, H), 8.74 (s, H), 8.66 (s, 1H), 8.21 (d, *J* =8.0 Hz, 1H), 7.44- 7.40 (m, 2H), 7.03 (d, *J* =7.0 Hz, 1H), 6.88 (d, *J* =8.5 Hz, 1H), 6.51 (t, *J* = 6.0 Hz, 1H), 4.91 (dd, *J =* 12.5, 5.0 Hz, 1H), 4.25 (s, 2H), 3.83 - 3.78 (m, 2H), 3.74 - 3.69 (m, 8H), 3.49- 3.45 (m, 2H), 3.00 - 2.69 (m, 11H), 2.32 (s, 3H), 2.14-2.11(m, 1H). | MS-ESI *m*/*z*: 701.7 [M+H]+. | **B** |
| **D130** | ¹H NMR (500 MHz, CDCl₃): δ 9.84 (s, 1H), 8.75 (s, 2H), 8.58 (d, *J* =12.5 Hz, 1H), 8.12 (s, 1H), 7.46- 7.45 (m, 1H), 7.44- 7.43 (m, 1H), 7.06 (d, *J* =6.5 Hz, 1H), 6.91 (d, *J* =8.5 Hz, 1H), 6.51 (s, 1H), 4.95 (dd, *J =* 12.0, 5.5 Hz, 1H), 4.65 - 4.62 (m, 2H), 4.43 - 4.40 (m, 1H), 3.84 - 3.81 (m, 1H), 3.75 - 3.60 (m, 12H), 3.50 - 3.47 (m, 2H), 3.28 - 3.23 (m, 2H), 3.00 - 2.64 (m, 7H), 3.36 (s, 3H), 2.18-2.14(m, 1H). | MS-ESI *m*/*z*: 771.5 [M+H]+. | **A** |
| **D131** | ¹H NMR (500 MHz, CDCl₃): δ 11.56 (s, 1H), 9.80 (s, 1H), 8.72 (s, 2H), 8.15 (s, 1H), 7.66- 7.45 (m, 2H), 7.26- 7.23 (m, 1H), 7.06 (d, *J* =6.5 Hz, 1H), 4.95 (dd, *J =* 12.0, 5.5 Hz, 1H), 4.65 - 4.62 (m, 2H), 4.43 - 4.40 (m, 1H), 3.85 - 3.82 (m, 1H), 3.80 - 3.60 (m, 10H), 3.40 - 3.27 (m, 6H), 2.99 - 2.94 (m, 3H), 2.76 - 2.63 (m, 8H),3.35 (s, 2H), 2.18-2.14(m, 1H). | MS-ESI *m*/*z*: 796.8 [M+H]+. | **A** |
| **D132** | ¹H NMR (500 MHz CDCl₃): δ 2.04(m, 1H), 2.18(s, 3H), 2.28(s, 3H), 2.60(m, 2H), 2.87(m, 1H), 3.19(s, 4H), 3.49(s, 4H), 5.14(dd, J = 12.9, 5.4 Hz, 1H), 7.40(d, J = 8.0 Hz, 1H) , 7.44(d, J = 8 Hz, 1H), 7.57(d, J = 7 Hz, 1H), 7.74(m, 1H), 8.08(d, J = 8 Hz, 1H), 8.64(s, 1H), 8.79(d, J = 1.5, Hz, 1H), 9.48(s, 1H), 10.15(s, 1H), 11.09(s, 1H). | MS-ESI *m*/*z*: 587.4 [M+H]+. | **C** |
| **D133** | ¹H NMR (500 MHz, CDCl₃): δ 9.92 (s, 1H), 8.76 (s, 1H), 8.65 (s, 1H), 8.35 (d, *J* =9.0 Hz, 1H), 7.98 (s, 1H), 7.60 - 7.40 (m, 2H), 7.39 (s, 1H), 7.21(d, *J* =7.5 Hz, 1H), 4.98 (dd, *J =* 12.0, 5.5 Hz, 1H), 3.33 (s, 8H), 2.93 - 2.74 (m, 3H), 2.39 (s, 3H), 2.14-2.12(m, 1H), 1.83(s, 4H), 1.75(s, 4H). | MS-ESI *m*/*z*: 640.7 [M+H]+. | **C** |
| **D134** | ¹H NMR (500 MHz, DMSO-d₆): δ 11.11 (s, 1H), 10.43 (s, 1H), 8.85(d, *J* =4.0 Hz, 1H), 8.69 (s, 1H), 8.33 (d, *J* =9.0 Hz, 1H), 7.79- 7.73 (m, 2H), 7.46- 7.40 (m, 2H), 5.13 (dd, *J =* 13.0, 5.5Hz, 1H), 3.64 (s, 4H), 3.50 (s, 4H), 2.90 - 2.84 (m, 1H), 2.63 - 2.58 (m, 2H), 2.33 (s, 3H), 2.08-2.03(m, 1H). | MS-ESI *m*/*z*: 552.6 [M+H]+. | **F** |
| **D135** | ¹H NMR (500 MHz, DMSO-d₆): δ 11.10 (s, 1H), 10.03 (s, 1H), 8.83(d, *J* =4.0 Hz, 1H), 8.66 (s, 1H), 8.58 (d, *J* =7.0 Hz, 1H), 7.77- 7.74 (m, 1H), 7.61- 7.59 (m, 1H), 7.46(d, *J* =8.0 Hz, 1H), 7.41(d, *J* =7.0 Hz, 1H), 5.13 (dd, *J* = 12.5, 5.5Hz, 1H), 3.54 (s, 4H), 3.42 (s, 4H), 3.29 (s, 3H), 3.11(s, 3H), 2.87 - 2.84 (m, 1H), 2.63 - 2.59 (m, 2H), 2.25 (s, 3H), 2.05-1.99(m, 1H). | MS-ESI *m*/*z*: 570.3 [M+H]+. | **F** |
| **D136** | ¹H NMR (500 MHz, CDCl₃): δ 9.95 (s, 1H), 8.80 (s, 1H), 8.75(d, *J* =3.0 Hz, 1H), 8.30 - 8.28 (m, 1H), 7.96 (s, 1H), 7.75 (d, *J* =9.0 Hz, 1H), 7.61-7.59 (m, 1H), 7.32(d, *J* =2.5 Hz, 1H), 7.11 - 7.09 (m, 1H), 4.97 (dd, *J =* 12.0, 5.0 Hz, 1H), 3.64 - 3.62 (m, 4H), 3.50-3.48 (m, 4H), 2.94 - 2.72 (m, 3H), 2.40 (s, 3H), 2.18-2.15(m, 1H). | MS-ESI *m*/*z*: 572.6 [M+H]+. | **F** |
| **D137** | ¹H NMR (500 MHz, CDCl₃): δ 9.75 (s, 1H), 8.77 (s, 1H), 8.60(d, *J* =4.5 Hz, 1H), 7.99 (s, 2H), 7.68 - 7.65 (m, 1H), 7.48-7.46 (m, 1H), 7.39 (s, 1H), 7.25 (s, 1H), 6.81 (s, 1H), 5.00 (dd, *J =* 12.5, 5.5 Hz, 1H), 3.58 - 3.52 (m, 8H), 2.94 - 2.74 (m, 3H), 2.37 (s, 3H), 2.18-2.14(m, 1H). | MS-ESI *m*/*z*: 527.3 [M+H]+. | **F** |
| **D138** | ¹H NMR (500 MHz, CDCl₃): δ 9.93 (s, 1H), 8.79-8.77 (m, 2H), 8.25 - 8.23 (m, 1H), 7.95 (t, *J* =8.5 Hz, 1H), 7.89 (s, 1H), 7.62 - 7.60 (m, 1H), 7.48-7.39 (m, 1H), 6.51-6.49 (m, 1H), 6.31-6.28 (m, 1H), 5.57 (s, 1H), 4.79 (dd, *J* = 13.0, 6.0 Hz, 1H), 3.97 (s, 2H), 3.89 - 3.87 (m, 2H), 3.66 - 3.64 (m, 2H), 3.34 - 3.28 (m, 4H), 2.84 - 2.71 (m, 3H), 2.40 (s, 3H), 1.98-1.94(m, 1H). | MS-ESI *m*/*z*: 621.1 [M+H]+. | **A** |
| **D139** | ¹H NMR (500 MHz, CDCl₃): δ 9.89 (s, 1H), 8.71-8.69 (m, 3H), 8.21 (d, *J* =9.0 Hz, 1H), 7.82 (t, *J* =9.0Hz, 1H), 7.57 - 7.54 (m, 1H), 7.39-7.35 (m, 1H), 6.45 (d, *J* =9.0 Hz, 1H), 6.27-6.25 (m, 1H), 4.75 (dd, *J =* 11.0, 5.0 Hz, 1H), 3.84 - 3.82 (m, 4H), 3.73 - 3.72 (m, 2H), 3.66 - 3.64 (m, 2H), 3.47 (s, 2H), 3.30 - 3.22 (m, 6H), 2.78 - 2.75 (m, 2H), 2.66 - 2.63 (m, 3H), 2.37 (s, 3H), 1.93-1.90(m, 1H). | MS-ESI *m*/*z*: 679.2 [M+H]+. | **A** |
| **D140** | ¹ H NMR (500 MHz, DMSO-d₆): δ 11.10 (s, 1H), 10.40 (s, 1H), 9.57 (s, 1H), 9.12(d, *J* =8.5 Hz, 1H), 8.67 (d, *J* =4.5 Hz, 1H), 8.53 (s, 1H), 7.72- 7.70 (m, 1H), 7.57- 7.54 (m, 1H), 7.30(d, *J* =9.0 Hz, 1H), 7.00 (d, *J* =7.5 Hz, 1H), 6.82 (s, 1H), 5.00 (dd, *J* = 13.5, 5.5 Hz, 1H), 3.72 (m, 4H),2.42 - 2.37 (m, 3H), 2.35 (s, 3H), 2.11-2.01(m, 1H). | MS-ESI *m*/*z:* 546.0 [M+H]+. | **C** |
| **D141** | ¹H NMR (500 MHz, CDCl₃): δ 9.85 (s, 1H), 8.86 (d, *J* =3.0 Hz, 1H), 8.75 (d, *J* =4.0 Hz, 1H), 8.15- 8.13 (m, 1H), 7.99 (s, 1H), 7.65 (t, *J* =7.5 Hz, 1H), 7.54- 7.52 (m, 1H), 7.47- 7.45 (m, 1H), 7.24- 7.23 (m, 1H), 4.99 (dd, *J =* 12.0, 5.5 Hz, 1H), 4.72 - 4.67 (m, 2H), 4.50 - 4.49 (m, 1H), 3.74 - 3.73 (m, 1H), 3.64 - 3.56 (m, 4H), 3.52 - 3.48 (m, 2H), 3.26 - 3.22 (m, 2H), 2.94 - 2.63 (m,3H), 3.38 (s, 3H), 2.18-2.14(m, 1H). | MS-ESI *m*/*z:* 612.3 [M+H]+. | **F** |
| **D142** | ¹H NMR (500 MHz CDCl₃): δ 9.92(s, 1H), 8.70(s, 1H), 8.69(s, 1H), 8.21(d, J = 8.5 Hz, 1H), 7.5 2 - 7.5 1 (m, 3H), 7.13(d, *J =* 7.5 Hz,1H), 6.90(d, *J* = 8.5 Hz, 1H), 4.96(dd, *J*= 12.5, 5.0 Hz, 1H), 4.13-4.12 (m, 1H), 4.43 (bs, 2H), 3.99(d, J = 5.5 Hz, 2H), 3.58-3.57(m, 2H), 3.36-3.32(m, 6H), 2.88-2.67(m, 9H), 2.40(s, 3H), 2.15-2.11(m, 1H). | MS-ESI *m*/*z*: 615.3 [M+H]+. | **C** |
| **D143** | ¹H NMR (500 MHz CDCl₃): δ 9.91(s, 1H), 8.76(s, 1H), 8.7(d, *J =* 1.5, Hz, 1H), 8.27(dd, *J =* 8.5, 1.5 Hz, 1H), 8.05(s, 1H), 7.57(d, *J =* 4 Hz, 1H), 7.52(d, *J =* 7 Hz, 1H), 7.14(d, *J =* 7 Hz, 1H), 6.95(d, *J =* 8.5 Hz, 1H), 6.30(d, *J* = 7.5Hz, 1H), 4.94(dd, H = 14.5, 5 Hz, 1H), 3.69-3.67(m, 1H), 3.55-3.53(m, 2H), 3.19-3.17(m, 2H), 2.93-2.74 (m, 3H), 2.14-2.11(m, 3H), 1.79-1.73(m, 2H). | MS-ESI *m*/*z:* 586.3 [M+H]+. | **C** |
| **D144** | ¹H NMR (500 MHz CDCl₃): δ 9.90(s, 1H), 8.73(s, 1H), 8.63(s, 1H), 8.30(d, *J =* 8.5 Hz, 1H), 8.0(s, 1H) , 7.8(d, *J =* 8 Hz, 1H), , 7.47(d, *J =* 8 Hz, 1H), 7.38(s, 1H), 7.21(d, *J =* 2 Hz, 1H), 4.98(d, *J =* 7.5, 1H), 4.08-4.05(m, 2H), 3.70-3.69(m, 2H), 3.07-3.05(m, 2H), 2.74-2.93(m, 3H), 2.37(s, 3H), 2.17-2.13(m, 1H), 1.87-1.86(m, 5H), 1.45-1.44(m, 2H). | MS-ESI *m*/*z*: 615.5 [M+H]+. | **C** |
| **D145** | ¹H NMR (500 MHz CDCl₃): δ 9.56(s, 1H), 8.75(s, 1H), 8.73(d, *J =* 4.5 Hz, 1H), 8.20(d, *J =* 8.5 Hz, 1H), 8.00(s, 1H), 7.80(d, *J =* 8 Hz, 1H), 7.24(dd, J = 8, 2 Hz, 1H), 4.98(dd, *J =* 12.5, 5.0, Hz), 7.41(d, *J =* 4 Hz, 1H), 7.39(s, 1H), 4.22(s, 1H), 4.13(m, 2H), 3.70-3.68(m, 2H), 3.13-3.00(m, 1H), 2.93-2.74(m, 3H), 2.32(s, 3H),2.16-2.14(m, 1H), 1.90(m, 4H), 1.77-1.70(m, 1H), 1.45-1.43(m, 2H). | MS-ESI *m*/*z:* 585.6 [M+H]+. | **B** |
| **D146** | ¹H NMR (500 MHz CDCl₃): δ 9.91(s, 1H), 8.75(s, 1H), 8.67(s, 1H), 8.28(d, *J =* 8.5 Hz, 1H), 8.00(b, 1H), 7.81(d, *J =* 8.5 Hz, 1H), 7.55(d, *J =* 4.0 Hz, 1H), 7.36(s, 1H), 7.23 (d, *J=* 8.5 Hz, 1H), 4.98(d, *J =* 7.5, Hz, 1H), 4.43(bs, 2H), 4.13-4.10(m, 1H), 3.99(d, *J =* 5.5 Hz, 2H), 3.58-3.54(m, 2H), 3.13-3.12(m, 2H), 2.75-2.92(m, 3H), 2.38(s, 3H), 2.27-2.15(m, 2H), 1.97-1.80(m, 2H). | MS-ESI *m*/*z*: 601.6 [M+H]+. | **C** |
| **D147** | ¹H NMR (500 MHz CDC1₃): δ 9.57(s, 1H), 8.76(s, 1H), 8.72(s, 1H), 8.22(d, *J =* 8.5 Hz, 1H), 8.18(bs, 1H), 7.81(d, *J =* 8.5 Hz, 1H), 7.41(d, *J =* 4 Hz, 1H), 7.39(s, 1H), 7.24(d, *J =* 8.5 Hz, 1H), 4.98(dd, *J =* 12.5, 5.0, Hz, 1H), 4.13-4.10(m, 1H), 4.43(bs, 2H), 4.03(d, *J =* 6 Hz, 2H), 3.18-3.10(m, 2H), 2.72-2.93(m, 5H), 2.32(s, 3H), 2.16-2.14(m, 1H), 2.01-2.07(m, 4H). | MS-ESI *m*/*z*: 571.6 [M+H]+. | **B** |
| **D148** | ¹H NMR (500 MHz CDCl₃): δ 9.91 (s, 1H), 8.70 - 8.69 (m, 1H), 8.60 (s, 1H), 8.53 (s, 1H), 8.38- 8.36 (m, 1H), 7.59- 7.56 (m, 1H), 7.45 (t, *J =* 8.0 Hz, 1H), 7.05 (d, *J* =6.5 Hz, 1H), 6.90 (d, *J* =8.5 Hz, 1H), 6.52 (t, *J* =5.5 Hz, 1H), 4.92 (dd, *J =* 12.0, 5.5 Hz, 1H), 3.88 - 3.82 (m, 4H), 3.75 - 3.74 (m, 4H), 3.73 - 3.72 (m, 4H), 3.54 - 3.46 (m, 6H), 2.91 - 2.66 (m, 5H), 3.39 (s, 3H), 2.16-2.13(m, 1H). | MS-ESI *m*/*z*: 712.4 [M+H]+. | **A** |
| **D149** | ¹H NMR (500 MHz, CDCl₃): δ 9.71 (s, 1H), 8.71 (s, 1H), 8.66 (s, 1H), 8.60 (d, *J* =8.0 Hz, 1H), 8.48 (s, 1H),7.49- 7.44 (m, 2H), 7.09 (d, *J* =7.5 Hz, 1H), 6.92 (d, *J* =8.0 Hz, 1H), 6.53 (t, *J* =5.5 Hz, 1H), 4.92 (dd, *J =* 12.0, 5.0 Hz, 1H), 3.87 - 3.68 (m, 13H), 3.50 - 3.47 (m, 2H), 3.03 - 2.85 (m, 9H), 2.82 - 2.64 (m, 5H), 3.34 (s, 3H), 2.15-2.11(m, 1H). | MS-ESI *m*/*z*: 730.7 [M+H]+. | **A** |
| **D150** | ¹H NMR (500 MHz CDCl₃): δ 9.01(s, 1H), 8.77(s, 1H), 8.21(d, J = 8.5 Hz, 1H),8.18(s, 1H), 7.77-7.75(m, 1H), 7.72-7.70(m, 2H), 7.62(t, *J =* 4.5 Hz, 1H), 7.44(t, *J =* 8.5Hz, 1H), 5.0(dd, H = 11.2, 5.0 Hz, 1H), 3.73(d, *J =* 5 Hz, 2H), 3.57(d, *J =* 5 Hz, 2H), 3.11-3.25(m, 5H), , 2.73-2.95(m, 4H), 2.18-2.14(m, 1H) 1.89(m, 2H), 1.02(t, *J =* 7.5 Hz, 3H). | MS-ESI *m*/*z*: 636.7 [M+H]+. | **C** |
| **D151** | ¹H NMR (500 MHz, CDCl₃): δ 9.93 (s, 1H), 8.77 (s, 1H), 8.75 (s, 1H), 8.52 (s, 1H), 8.25 (d, *J* =7.5 Hz, 1H), 7.59 (d, *J* =12.5 Hz, 1H), 7.48- 7.44 (m, 1H), 7.37- 7.31 (m, 1H), 6.87 (s, 1H), 5.28 (dd, *J =* 13.5, 5.0 Hz, 1H), 4.47 - 4.44 (m, 1H), 4.29 (s, 1H), 3.83 - 3.75 (m, 2H), 3.63 - 3.62 (m, 2H), 3.32 - 3.26 (m, 6H), 2.94 - 2.82 (m, 2H), 2.43 - 2.36 (m, 4H), 2.24-2.20(m, 1H), 1.76 - 1.74 (m, 4H), 1.73 - 1.71 (m, 4H). | MS-ESI *m*/*z*: 671.6 [M+H]+. | **A** |

### A-3. Preparation of compounds of class II

**Compounds of class II have a structure in which an E3 ubiquitin ligase binding domains is linked at position 5 of the quinoline skeleton via a linker.**

### (1) Method G

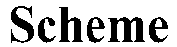

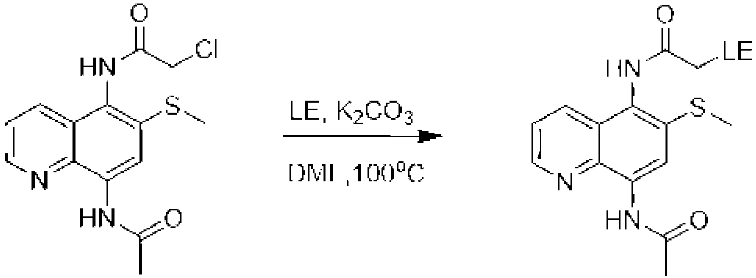

LE:

| | |
|---|---|
| LE-005 | 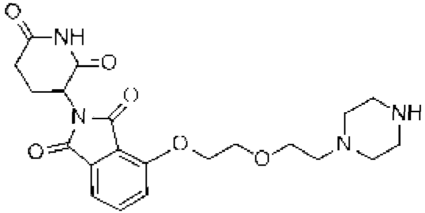 |
| LE-013 | 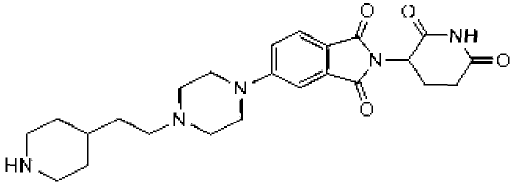 |
| LE-023 | 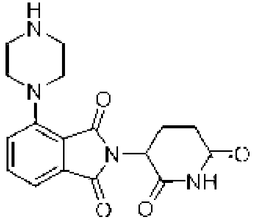 |
| LE-045 | 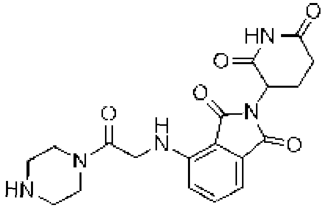 |

### Preparation example

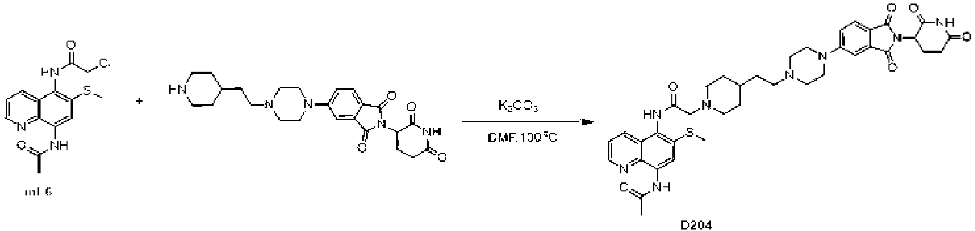

### Preparation of Compound D204

32 mg of **int-6,** i.e., *N*-(8-acetamido-6-(methylthio)quinolin-5-yl)-2-chloroacetamide, was dissolved in 2 mL of DMF. Next, 1.5 eq of LE-023 2-(2,6-dioxopiperidin-3-yl)-5-(4-(2-(piperidin-4-yl)ethyl)piperazin-1-yl)isoindoline-1,3-dione, and 4.5 eq of K₂CO₃ were added thereto and stirred at 100°C for 16 hours to carry out the reaction. After the reaction was completed, the reacted mixture was added to water, stirred and filtered. After filtration, the solid was taken and extracted with DCM and H₂O. After that, the DCM layer was taken out, dried and concentrated, and then it was purified by a column to afford 36 mg of Compound **D204** (48.6% yield).

In this example, **Compounds D201 to D204** were prepared. The structures of **Compounds D201 to D204** are shown in Table 10 below, and the methods respectively used to prepare **Compounds D201 to D204,** as well as the respective proton nuclear magnetic resonance analysis results and liquid chromatography-mass spectrometry analysis results for **Compounds D201 to D204,** are shown in Table 11.

**Table 11**

| **Compound number** | **Structure** |
|---|---|
| **D201** | 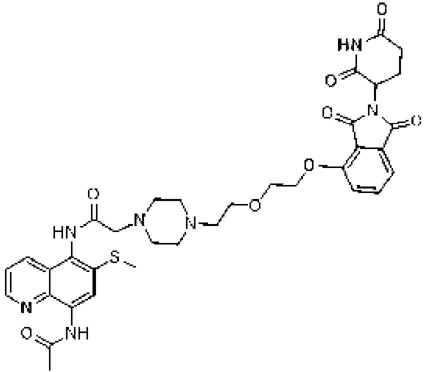 |
| **D202** | 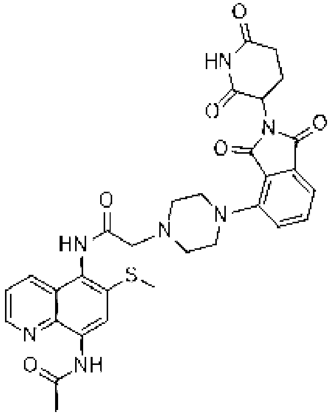 |
| **D203** | 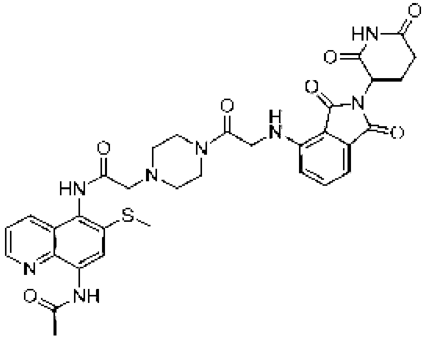 |
| **D204** | 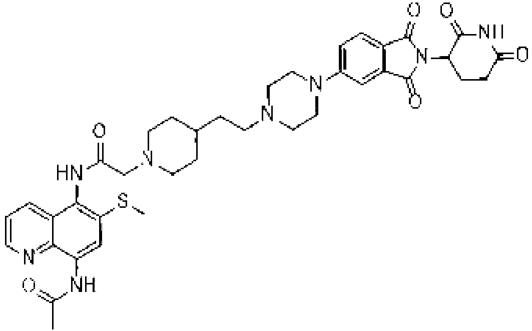 |

**Table 11**

| **Compound number** | **HNMR** | **LCMS** | **Method** |
|---|---|---|---|
| **D201** | ¹H NMR (500 MHz, DMSO-d₆): δ 11.11 (s, 1H), 10.19 (s, 1H), 9.45(s, 1H), 8.84(s, 1H), 8.71(s, 1H), 8.08(d, *J* =8.5 Hz, 1H), 7.80 (t, *J* = 7.5 Hz, 1H), 7.61(d, *J* =4.0 Hz, 1H), 7.60- 7.52 (m, 1H), 7.46- 7.44 (m, 1H), 5.08 (dd, *J =* 9.5, 5.0Hz, 1H), 4.35 (s, 2H), 3.78 (s, 2H), 3.64 (s, 2H), 3.29 - 3.28(m, 1H), 3.21 (s, 2H), 2.88 - 2.83(m, 1H), 2.60 - 2.40(m, 14H), 2.33(s, 3H), 2.01-2.00(m, 1H). | MS-ESI *m*/*z:* 718.7 [M+H]+. | **G** |
| **D202** | ¹H NMR (500 MHz, CDCl₃): δ 9.83 (s, 1H), 9.03 (s, 1H), 8.88(s, 1H), 8.74(d, *J* =3.0 Hz, 1H), 8.14 - 8.03 (m, 2H), 7.65 - 7.62 (m, 1H), 7.49 - 7.47 (m, 1H), 7.46- 7.45 (m, 1H), 7.23(d, *J* =8.0 Hz, 1H), 4.99 (dd, *J =* 12.0, 5.5 Hz, 1H), 3.63-3.52 (m, 4H), 3.50 - 3.43 (m, 2H), 3.29- 3.06 (m, 4H), 2.93 - 2.70 (m, 3H), 2.31(s, 3H), 2.37 (s, 3H), 2.16-2.14(m, 1H). | MS-ESI *m*/*z:* 630.6 [M+H]+. | **G** |
| **D203** | ¹H NMR (500 MHz, CDCl₃): δ 9.83 (s, 1H), 8.91 (s, 1H), 8.87 (s, 1H), 8.73 (d, *J* =3.0 Hz, 1H), 8.33(s, 1H), 8.00(d, *J* =8.0 Hz, 1H), 7.52- 7.46 (m, 2H), 7.17- 7.13 (m, 2H), 7.79(d, *J* =8.0 Hz, 1H), 4.94 (dd, *J =* 12.0, 5.5 Hz, 1H), 4.07 (s, 2H), 3.84 (s, 2H), 3.63 (s, 2H), 3.37 (s, 2H), 2.90 - 2.71(m, 7H), 2.62 (s, 3H), 2.38(s, 3H), 2.18-2.15(m, 1H). | MS-ESI *m*/*z:* 687.7 [M+H]+. | **G** |
| **D204** | ¹H NMR (500 MHz, CDCl₃): δ 9.82(s, 1H), 9.12(s, 1H), 8.84(s, 1H), 8.71-8.70 (m, 1H), 8.65 (s, 1H), 8.00(d, *J* =8.5 Hz, 1H), 7.68(d, *J* =8.5 Hz, 1H), 7.46(d, *J* =3.5 Hz, 1H), 7.44(d, *J* =4.5 Hz, 1H), 7.28 (s, 1H), 7.05(d, *J* =8.5 Hz, 1H), 4.94 (dd, *J =* 12.0, 5.5Hz, 1H), 3.43 (s, 4H), 3.28 (s, 2H), 3.18-3.16 (m, 2H), 2.89 - 2.73(m, 3H), 2.60 - 2.56(m, 2H), 2.48 - 2.33(m, 5H), 2.17 - 2.13(m, 1H), 1.81-1.80 (m, 2H), 1.53-1.44 (m, 2H), 1.43-1.42(m, 2H). | MS-ESI *m*/*z:* 741.7 [M+H]+. | **G** |

### A-4. Preparation of compounds of class III

**Compounds of class III have a structure in which an E3 ubiquitin ligase binding domains is linked at position 8 of the quinoline skeleton via a linker.**

### (1) Method H

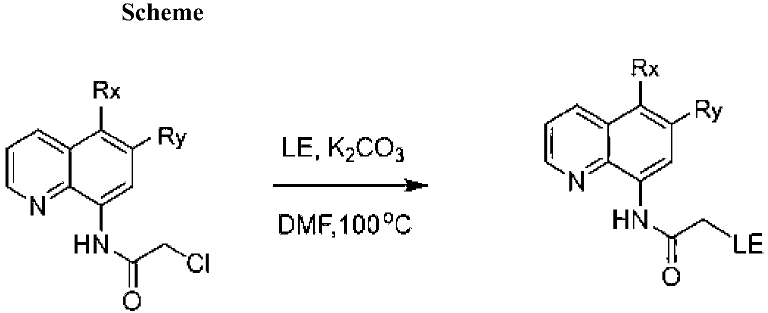

Rₓ is NO₂, CN or 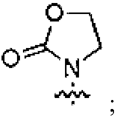 R_{y} is OCH₃, SCH₃, morpholine, n-methylpiperazine, pyrrolidin-3-amine, 4-aminopiperidine or 4-(N-Boc-amino)piperidine;
LE:

| | |
|---|---|
| LE-013 | 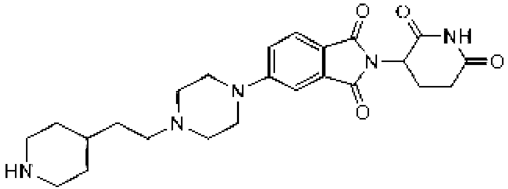 |
| LE-023 | 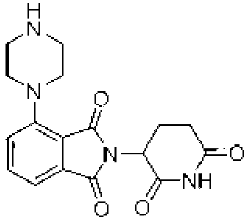 |
| LE-030 | 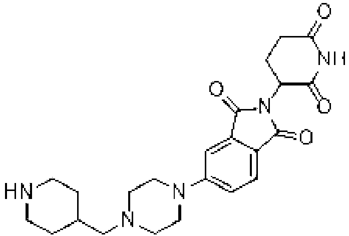 |

### Preparation example

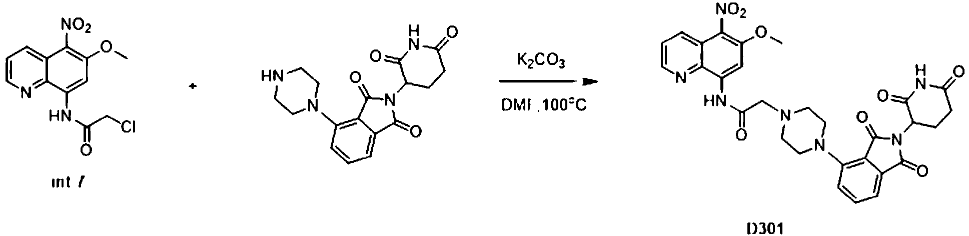

### Preparation of Compound D301

32 mg of **int-7,** i.e., 2-chloro-*N*-(6-methoxy-5-nitroquinolin-8-yl)acetamide, was dissolved in 2 mL of DMF. Next, 1.5 eq of 2-(2,6-dioxopiperidin-3-yl)-4-(piperidin-4-yl)isoindole-1,3-dione, and 4.5 eq of K₂CO₃ were added thereto and stirred at 100°C for 16 hours to carry out the reaction. After the reaction was completed, the reacted mixture was added to water, stirred and filtered. After filtration, the solid was taken and extracted with DCM/H₂O. After that, the DCM layer was taken out, dried and concentrated, and then it was purified by a column to afford 36 mg of Compound **D301** (48.5% yield).

In this example, **Compounds D301 to D316** were prepared. The structures of **Compounds D301 to D316** are shown in Table 12 below, and the methods respectively used to prepare **Compounds D301 to D316,** as well as the respective proton nuclear magnetic resonance analysis results and liquid chromatography-mass spectrometry analysis results for **Compounds D301 to D316,** are shown in Table 13.

**Table 12**

| **Compound number** | **Structure** |
|---|---|
| **D301** | 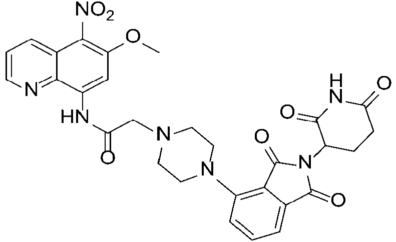 |
| **D302** | 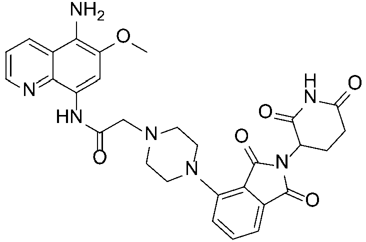 |
| **D303** | 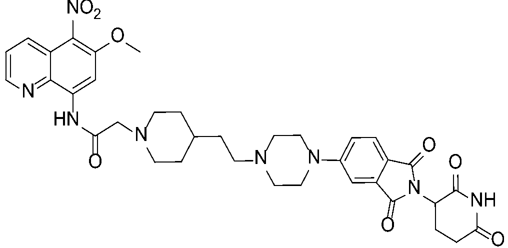 |
| **D304** | 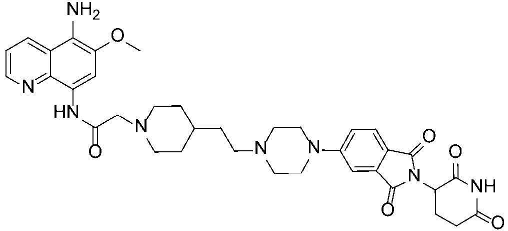 |
| **D305** | 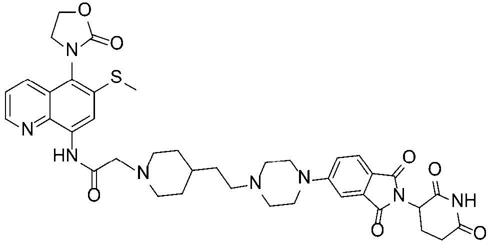 |
| **D306** | 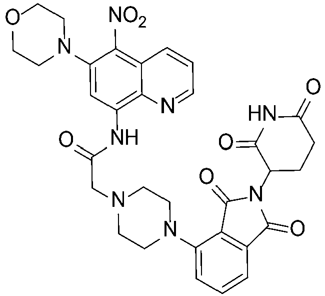 |
| **D307** | 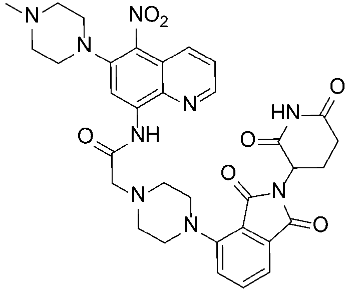 |
| **D308** | 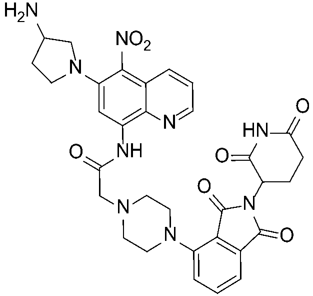 |
| **D309** | 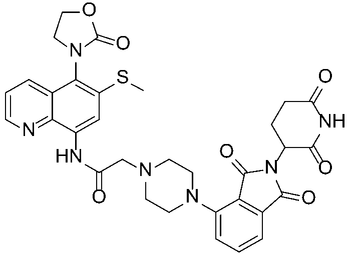 |
| **D310** | 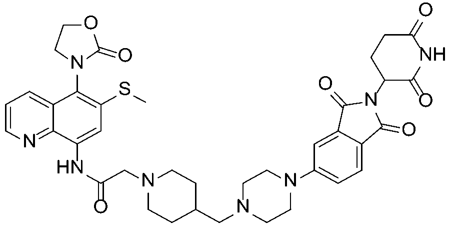 |
| **D311** | 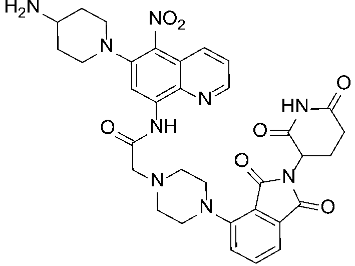 |
| **D312** | 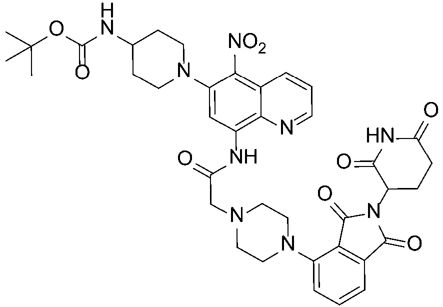 |
| **D313** | 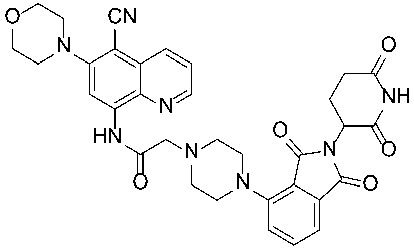 |
| **D314** | 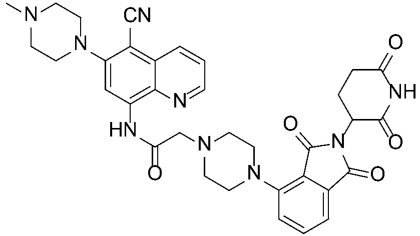 |
| **D315** | 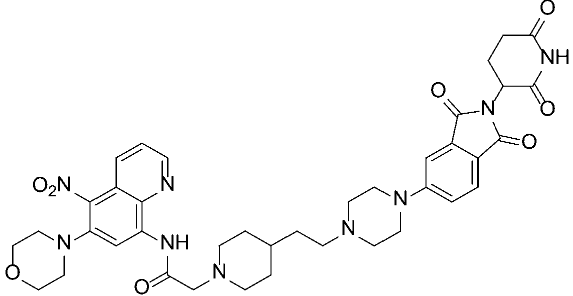 |
| **D316** | 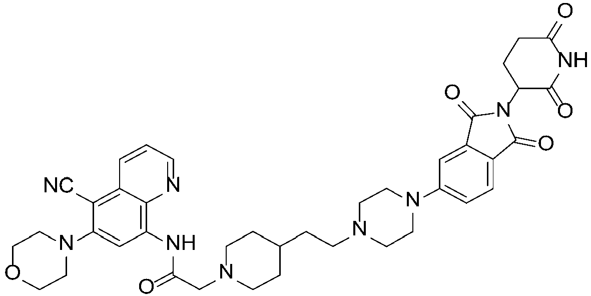 |

**Table 13**

| **Compound Number** | **HNMR** | **LCMS** | **Method** |
|---|---|---|---|
| **D301** | ¹H NMR (500 MHz, DMSO-d₆): δ 11.67 (s, 1H), 11.12 (s, 1H), 8.92 (d, *J* =3.5 Hz, 1H), 8.78 (s, 1H), 8.23 (d, *J* =8.0 Hz, 1H), 7.79- 7.73 (m, 2H), 7.44- 7.39 (m, 2H), 5.10 (dd, *J =* 12.5, 5.5Hz, 1H), 4.05 (s, 3H), 3.47 (s, 4H), 3.44 (s, 2H), 2.83 (s, 4H), 2.60-2.56(m, 3H), 2.03-2.02(m, 1H). | MS-ESI *m*/*z:* 602.7 [M+H]+. | **H** |
| **D302** | ¹H NMR (500 MHz, CDCl₃): δ 11.28 (s, 1H), 8.75 (s, 1H), 8.69 (d, *J* =4.5 Hz, 1H), 8.17 (d, *J* =8.5 Hz, 1H), 8.03 (s, 2H), 7.66- 7.63 (m, 2H), 7.45- 7.44 (m, 1H), 4.97 (dd, *J* = 12.0, 6.0Hz, 1H), 4.04 (s, 3H), 3.55 (s, 4H), 3.37 (s, 2H), 2.96 (s, 4H), 2.89-2.73(m, 3H), 2.13-2.10(m, 1H). | MS-ESI *m*/*z:* 572.8 [M+H]+. | **B** |
| **D303** | ¹H NMR (500 MHz, CDCl₃): δ 11.79 (s, 1H), 8.85 (s, 1H), 8.78 (d, *J* =4.5 Hz, 1H), 8.25 (d, *J* =9.0 Hz, 1H), 7.99 (s, 1H), 7.72- 7.60 (m, 1H), 7.59- 7.58 (m, 1H), 7.31 (s, 1H), 7.09- 7.08 (m, 1H), 4.96 (dd, *J* = 12.0, 5.5Hz, 1H), 4.11 (s, 3H), 3.64 (s, 4H), 3.29 (s, 2H), 2.99-2.93(m, 2H), 2.90-2.75(m, 3H), 2.64 (s, 4H), 2.50-2.47(m, 2H), 2.36-2.32(m, 2H), 2.18 - 2.16 (m, 1H), 1.82-1.79(m, 2H), 1.59-1.54(m, 4H), 1.43-1.26(m, 1H). | MS-ESI *m*/*z:* 713.7 [M+H]+. | **H** |
| **D304** | ¹H NMR (500 MHz, CDCl₃): δ 11.32 (s, 1H), 8.75 (s, 1H), 8.71 (d, *J* =4.0 Hz, 1H), 8.17 (d, *J =* 8.0 Hz, 1H), 8.08 (d, *J* =7.5 Hz, 1H), 7.71(d, *J* =8.5 Hz, 1H), 7.40- 7.38 (m, 1H), 7.31 (s, 1H), 7.09- 7.08 (m, 1H), 4.96 (dd, *J =* 13.0, 5.5Hz, 1H), 4.86(s, 2H), 4.03 (s, 3H), 3.64 (s, 4H), 3.24 (s, 2H), 3.08-2.93(m, 2H), 2.89-2.77(m, 3H), 2.64 (s, 4H), 2.50-2.47(m, 2H), 2.32-2.28(m, 2H), 2.18-2.14(m, 1H), 1.79-1.77(m, 2H), 1.63-1.56(m, 4H), 1.43-1.26(m, 1H). | MS-ESI *m*/*z:* 683.7 [M+H]+. | **B** |
| **D305** | ¹H NMR (500 MHz, CDCl₃): δ 11.56 (s, 1H), 8.90 (s, 1H), 8.79 (d, *J* =3.5 Hz, 1H), 8.07- 8.02 (m, 2H), 7.71 (d, *J* =8.0 Hz, 1H), 7.54- 7.51 (m, 1H), 7.30 (d, *J* =7.0 Hz, 1H), 7.08 (d, *J* =8.0 Hz, 1H), 4.95 (dd, *J* = 13.0, 5.5 Hz, 1H), 4.70 - 4.67 (m, 2H), 4.23 - 4.21 (m, 1H), 3.85 - 3.84 (m, 1H), 3.50 - 3.46 (m, 8H), 3.26 (s, 2H), 2.98 - 2.77 (m, 6H), 2.64 - 2.60 (m, 8H), 2.49 - 2.46 (m, 2H), 2.31 - 2.22 (m, 2H), 2.18-2.14(m, 1H), 2.09-2.02 (m, 1H). | MS-ESI m/z: 769.7 [M+H]+. | **H** |
| **D306** | ¹H NMR (500 MHz, DMSO-d₆): δ 8.89 (d, *J =* 3 Hz, 1H), 8.70 (s, 1H), 8.22 (d, *J =* 9 Hz, 1H), 7.76-7.73 (m, *2H),* 7.43 (d, *J =* 8.5 Hz, 1H), 7.39 (d, *J =* 7.5 Hz, 1H), 5.14 (dd, *J =* 12.5, 5.0 Hz, 1H), 3.79 (s, 2H), 3.73 (m, 4H), 3.41 (m, 8H), 3.15 (m, 4H), 2.60-2.49 (m, 3H), 2.05-1.97 (m, 1H). | MS-ESI *m*/*z:* 657.7 [M+H]+. | **H** |
| **D307** | ¹H NMR(500 MHz, CD₃OD) :δ 8.84 (d, *J =* 2.5 Hz, 1H), 8.76 (s, 1H), 7.69 (d, *J =* 8.5 Hz, 1H), 7.71-7.70 (m, 1H), 7.67-7.65 (m, 1H), 7.42-7.40 (m, 2H), 5.13 (dd, *J* = 12.5, 5.0 Hz, 1H), 3.58 (m, 4H), 3.42 (s, 2H), 2.92 (m, 6H), 2.73-2.72 (m, 6H), 2.44 (s, 3H), 2.02- 1.96(d, *J*=7.5 Hz, 4H). | MS-ESI *m*/*z:* 670.8 [M+H]+. | **H** |
| **D308** | ¹H NMR (500 MHz, DMSO-d₆): δ 11.61 (s, 1H), 8.64 (s, 1H), 8.59-8.58 (m, 1H), 8.40 (d, *J =* 8.5 Hz, 1H), 8.03 (s, 1H), 7.67-7.64 (m, 1H), 7.50(dd, *J =* 8.5, 4.0 Hz, 1H), 7.42 (d, *J =* 8.5 Hz, 1H), 7.27 (d, *J =* 8.0 Hz, 1H), 4.96 (dd, *J =* 12.5, 5.0 Hz, 1H), 3.76-3.74 (m, 1H), 3.66-3.73 (m, 1H), 3.57-3.47 (m, 2H), 3.54 (m, 4H), 3.36 (s, 2H), 3.06-3.03 (m, 1H), 2.90 (m, 4H), 2.84-2.72 (m, 2H), 2.21-2.12 (m, 3H), 1.86-1.83 (m, 1H) | MS-ESI *m*/*z*: 656.7 [M+H]+. | **H** |
| **D309** | ¹H NMR (500 MHz, CDCl₃): δ 11.49 (s, 1H), 8.92 (s, 1H), 8.78 (s, 1H), 8.24- 8.19 (m, 1H), 8.07 (s, 1H), 7.69- 7.65 (m, 1H), 7.53- 7.47 (m, 2H), 7.22- 7.19 (m, 1H), 4.97 (dd, *J =* 12.0, 6.0Hz, 1H), 4.65 - 4.62 (m, 2H), 4.43 - 4.40 (m, 1H), 3.84 - 3.81 (m, 1H), 3.63 - 3.57 (m, 2H), 3.49 (s, 2H), 3.44 - 3.42 (m, 2H), 3.02 - 2.64 (m, 10H), 2.25-2.22(m, 1H). | MS-ESI *m*/*z:* 658.7 [M+H]+. | **H** |
| **D310** | ¹H NMR (500 MHz, CDCl₃): δ 11.52 (s, 1H), 8.91 (s, 1H), 8.81 (s, 1H), 8.08- 8.06 (m, 2H), 7.71 (d, *J* =9.0 Hz, 1H), 7.54- 7.51 (m, 1H), 7.30- 7.29 (m, 1H), 7.08- 7.06 (m, 1H), 4.97 (dd, *J* = 12.0, 5.5Hz, 1H), 4.70 - 4.67 (m, 2H), 4.25 - 4.20 (m, 1H), 3.87 - 3.84 (m, 1H), 3.46 (s, 2H), 3.29 (s, 2H), 3.00 (s, 1H), 2.90 - 2.73 (m, 3H), 2.65 (s, 3H), 2.62 - 2.61 (m, 2H), 2.35 - 2.33 (m, 2H), 2.15-2.14(m, 1H, 1.98 - 1.67 (m, 4H). | MS-ESI *m*/*z*: 755.7 [M+H]+. | **H** |
| **D311** | ¹ H NMR (500 MHz, DMSO-d₆): δ 8.87 (d, *J =* 1.5 Hz, 1H), 8.7 0(s, 1H), 8.23 (d, *J* = 9.0 Hz, 1H), 7.74-7.73 (m, 2H), 7.43 (d, *J =* 9.0 Hz, 1H), 7.39 (d, *J =* 7.0 Hz, 1H), 5.10 (dd, *J =* 12.5, 5.0 Hz, 1H), 3.56-3.52 (m, 3H), 3.40-3.43 (m, 2H), 3.47 (m, 4H), 2.91 (m, 4H), 3.08-3.03 (m, 2H), 2.91-2.87 (m, 2H), 1.95-1.97 (m, 3/2H), 1.58-1.62 (m, 3/2H). | MS-ESI *m*/*z:* 670.7 [M+H]+. | **H** |
| **D312** | ¹H NMR (500 MHz, CDCl₃): δ 8.70-8.67 (m, 2H), 8.24 (d, *J =* 8.5 Hz, 1H), 7.65-7.64 (m, 1H), 7.55-7.54 (m, 1H), 7.49-7.47 (m, 1H), 7.21 (d, *J =* 7.5 Hz, 1H), 4.99-4.96 (m, 1H), 4.56 (s, 2H), 3.69 (m, 8H), 3.46-3.44 (m, 3H), 3.14-3.10 (m, 2H), 2.92-2.80 (m, 2H), 2.92-2.81(m, 2H), 2.80-2.72 (m, 2H), 1.58-1.63 (m, 2H), 1.46 (s, 9H). | MS-ESI *m*/*z:* 770.8 [M+H]+. | **H** |
| **D313** | ¹H NMR (500 MHz, DMSO-d₆): δ 9.56 (d, *J* = 9.0 Hz, 1H), 9.32 (d, *J =* 5.0 Hz, 1H), 8.57-8.54 (m, 1H), 8.49 (s, 1H), 8.18-8.17 (m, 1H), 8.06 (d, *J* = 7.0 Hz, 1H), 7.82 (d, *J =* 8.5 Hz, 1H), 5.69 (dd, *J =* 12.5, 5.0 Hz, 1H), 5.09 (s, 2H), 4.54 (m, 4H), 4.46-4.08 (m, 12H), 3.45-3.34 (m, 2H), 3.26-3.17 (m, 1H), 2.72-2.63 (m, 1H). | MS-ESI *m*/*z:* 637.3 [M+H]+. | **H** |
| **D314** | ¹H NMR (500 MHz, DMSO-d₆): δ 9.29-9.27 (m, 1H), 9.15-9.14 (m, 1H), 8.30-8.28 (m, 2H), 7.87-7.84 (m, 1H), 7.30-7.20 (m, 1H), 7.49-7.48 (m, 1H), 5.37-5.35 (m, 1H), 4.74 (s, 2H), 4.31-4.34 (m, 4H), 4.04-3.88 (m, 8H), 3.62-3.57 (m, 4H), 3.21 (s, 3H), 3.06-3.10 (m, 2H), 2.90-2.82 (m, 1H), 2.35-2.34 (m, 1H). | MS-ESI *m*/*z:* 650.6 [M+H]+. | **H** |
| **D315** | ¹H NMR (500 MHz, DMSO-d₆): δ 9.08 (d, *J =* 6.0 Hz, 1H), 9.07 (d, *J =* 5.5 Hz, 1H), 8.21 (d, *J =* 5.0 Hz, 1H), 8.16 (s, 1H), 7.95-7.92 (m, 1H), 7.70 (s, 1H), 7.39 (s, 1H), 5.38- 5.35 (m, 1H), 4.56 (s, 2H), 4.24-4.22 (m, 2H), 4.18-4.07 (m, 4H), 3.97-3.91 (m, 4H), 3.67-3.58 (m, 6H), δ 3.46-3.35 (m, 4H), 3.29-3.22 (m, 2H), 3.10-3.06 (m, 2H), 2.94-2.91 (m, 2H), 2.36-2.35 (m, 1H), 2.21-2.15(m, 2H), 2.00-1.92 (m, 4H). | MS-ESI *m*/*z*: 768.7 [M+H]+. | **H** |
| **D316** | ¹H NMR (500 MHz, DMSO-d₆): δ 9.23 (d, *J =* 8.5 Hz, 1H), 8.96 (d, *J =* 4.0 Hz, 1H), 8.21-8.23 (m, 1H), 8.15 (s, 1H), 7.94 (d, *J =* 7.5 Hz, 1H), 7.71 (s, 1H), 7.34 (d, *J =* 8.5 Hz, 1H), 5.37 (dd, *J =* 12.5, 4.0 Hz, 1H), 4.56 (s, 2H), 4.25-4.22 (m, 4H), 3.98-3.92 (m, 10H), 3.67 (m, 2H), 3.63-3.60 (m, 2H), 3.47-3.42 (m, 4H), 3.37-3.35 (m, 2H), 3.18-3.06 (m, 2H), 2.97-2.85 (m, 1H), 2.37-2.35 (m, 1H), 2.17-2.14 (m, 2H), 2.05-1.81 (m, 3H). | MS-ESI *m*/*z:* 748.7 [M+H]+. | **H** |

### B. Analysis of the degradation activity of the compounds on transactive response DNA-binding protein-43 (TDP-43)

In this experiment, the activities of the compounds in degrading TDP-43 were analyzed by the NanoLuc-TDP-43 fusion protein assay system.

This experiment used 293-H cells (cat. no. 11631017) purchased from Thermo Fisher Scientific as the target cells for transfection. The cells were cultured in DMEM medium (Corning Incorporated; cat. no. 10-013-CM) containing 10% fetal bovine serum (FBS) (Thermo Fisher Scientific; cat. no. 10437028). After that, the cells were seeded in a 24-well culture plate at a density of 100,000 cells/well, and transfection was performed after 2 days of culture.

Transfection was performed using a transfection reagent (ViaFect^{™} Transfection Reagent, Promega; cat. no. E4982) according to the manufacturer's instructions. 500 ng of expression vector and 1.5 µL of transfection reagent were added to each well of the aforementioned 24-well plate to perform transfection on the cells. The expression vectors employed were pcDNA3.1-Myc-NanoLuc-LinkerTDP43 (abbreviated as NanoLuc-TDP-43(WT), whose map is shown in FIG. 1) and pcDNA3.1-Myc-NanoLuc-LinkerTDP43CTD (abbreviated as NanoLuc-TDP-43(CTD), whose map is shown in FIG. 2).

At 3 hours post-transfection, the compounds to be tested were added to respective wells, and NanoLuc luminescence activity in each well was analyzed after overnight incubation. Total protein was extracted from cells in each well using 100 µL of CelLytic^{™} M cell lysis buffer (Merck KGaA; cat. no. C2978-250ML), and coelenterazine-H (Regis Technologies; cat. no. 50909-86-9) was used as the substrate for NanoLuc. Luminescence analysis was performed using a multifunctional microplate reader (Thermo Varioskan LUX) to determine the luminescence intensity of each well, thereby confirming the NanoLuc activity in each well.

For the activity of NanoLuc, the relative inhibition rate of each group treated with different compounds was calculated by setting the luminescence intensity of the untreated group as 100%, and the degradation ability of each compound on transactive response DNA-binding protein-43 (TDP-43) was thereby determined.

The results are shown in Table 14 to Table 16.

**Table 14: Inhibitory activities of compounds of class I against NanoLuc-TDP43(WT) fusion protein and NanoLuc-TDP43(CTD) fusion protein**

| | **Analysis Item** | |
|---|---|---|
| **Compound name** | **Inhibitory activity against NanoLuc-TDP43(WT) fusion protein** | **Inhibitory activity against NanoLuc-TDP43(CTD) fusion protein** |
| **D101** | + | + |
| **D102** | + | + |
| **D103** | ++ | +++ |
| **D104** | +++ | + |
| **D105** | + | + |
| **D106** | + | + |
| **D107** | + | + |
| **D108** | + | ++ |
| **D109** | + | + |
| **D110** | + | + |
| **D111** | ++ | ++ |
| **D112** | + | + |
| **D113** | +++ | ++ |
| **D114** | +++ | + |
| **D115** | +++ | ++ |
| **D116** | ++ | +++ |
| **D117** | + | + |
| **D118** | ++++ | ++++ |
| **D119** | ++ | + |
| **D120** | + | ++ |
| **D121** | ++ | + |
| **D122** | + | + |
| **D123** | ++ | + |
| **D124** | ++ | ++ |
| **D125** | + | + |
| **D126** | +++ | + |
| **D127** | ++ | ++ |
| **D128** | ++++ | ++++ |
| **D129** | + | + |
| **D130** | + | ++ |
| **D131** | + | ++ |
| **D132** | +++ | ++ |
| **D133** | ++ | + |
| **D134** | +++ | ++ |
| **D135** | +++ | + |
| **D136** | ++ | ++ |
| **D137** | + | ++ |
| **D138** | + | + |
| **D139** | + | ++ |
| **D140** | + | + |
| **D141** | +++ | +++ |
| **D142** | ++ | ++ |
| **D143** | + | + |
| **D144** | + | + |
| **D145** | + | + |
| **D146** | + | + |
| **D147** | + | + |
| **D148** | +++ | ++ |
| **D149** | + | + |
| **D150** | +++ | ++ |
| **D151** | ++ | ++ |
| ++++: Inhibition activity >75%; +++: Inhibition activity 75%~50%; ++: Inhibition activity 49%~25%; +: Inhibition activity <25%. | | |

**Table 15: Inhibitory activities of compounds of class II against NanoLuc-TDP43(WT) fusion protein and NanoLuc-TDP43(CTD) fusion protein**

| | **Analysis Item** | |
|---|---|---|
| **Compound name** | **Inhibitory activity against NanoLuc-TDP43(WT) fusion protein** | **Inhibitory activity against NanoLuc-TDP43(CTD) fusion protein** |
| **D201** | ++ | +++ |
| **D202** | ++ | ++ |
| **D203** | + | + |
| **D204** | + | + |
| +++: Inhibition activity 75%~50%; ++: Inhibition activity 49%~25%; +: Inhibition activity <25%. | | |

**Table 16: Inhibitory activities of compounds of class III against NanoLuc-TDP43(WT) fusion protein and NanoLuc-TDP43(CTD) fusion protein**

| | **Analysis Item** | |
|---|---|---|
| **Compound name** | **Inhibitory activity against NanoLuc-TDP43(WT) fusion protein** | **Inhibitory activity against NanoLuc-TDP43(CTD) fusion protein** |
| **D301** | + | + |
| **D302** | + | + |
| **D303** | ++ | + |
| **D304** | ++ | ++ |
| **D305** | + | + |
| **D306** | + | +++ |
| **D307** | + | + |
| **D308** | + | + |
| **D309** | + | ++ |
| **D310** | ++ | ++ |
| **D311** | + | + |
| **D312** | + | ++ |
| **D313** | ++ | + |
| **D314** | + | + |
| **D315** | ++ | + |
| **D316** | + | + |
| +++: Inhibition activity 75%~50%; ++: Inhibition activity 49%~25%; +: Inhibition activity <25%. | | |

Table 14 to Table 16 above demonstrate that all compounds prepared in the examples exhibit the inhibitory activities against both the NanoLuc-TDP43(WT) fusion protein and the NanoLuc-TDP43(CTD) fusion protein.

In other words, all compounds prepared in the examples exhibit degradation activity against transactive response DNA-binding protein-43. That is, all proteolysis-targeting chimeras (PROTACs) prepared in the examples against transactive response DNA-binding protein-43 can effectively degrade transactive response DNA-binding protein-43, and can therefore be applied to the treatment and/or prevention of diseases associated with transactive response DNA-binding protein-43 accumulation.

While the invention has been described by way of example and in terms of the preferred embodiments, it should be understood that the invention is not limited to the disclosed embodiments. On the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. A compound or a salt thereof, wherein the compound has a structure represented by Formula (I), Formula (II) or Formula (III): 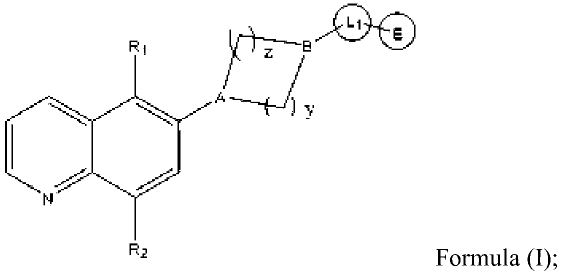 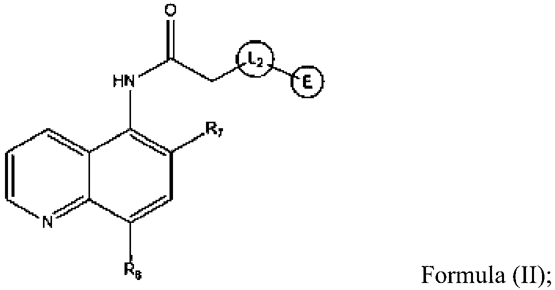 and 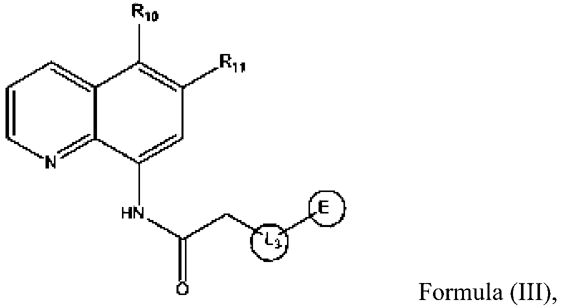 wherein
R₁ is H, cyano, NH₂, NO₂, NHR₃, NHR₃R₄, a heterocycle, NHCOR₅ or 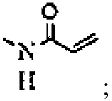
R₂ is NHCOR₆ or NH₂;
A and B are independently N, C, or absent;
y and z are independently an integer from 1 to 3;
R₃, R₄, R₅ and R₆ are independently C₁-C₆ alkyl, and the the alkyl is a linear or branched alkyl, and is optionally replaced by one or more halogen atoms, oxygen atoms, sulfur atoms or amines;
R₇ is SCH₃ or OCH₃;
R₈ is NHCOR₉ or NH₂;
R₉ is C₁-C₆ alkyl;
R₁₀ is H, cyano, NH₂, NO₂, NHR₃, NHR₃R₄, a heterocycle or NHCOR₅;
R₁₁ is SCH₃, OCH₃ or a heterocycle;
L₁ is a linker having a structure selected from a group consisiting of the structures shown in the following:
| Structure of L₁ |
|---|
| 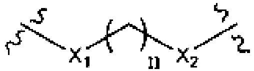 |
| 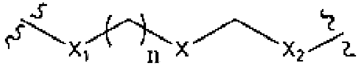 |
| 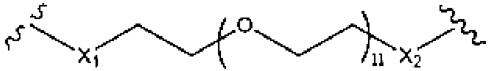 |
| 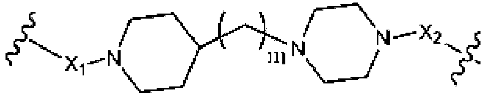 |
| 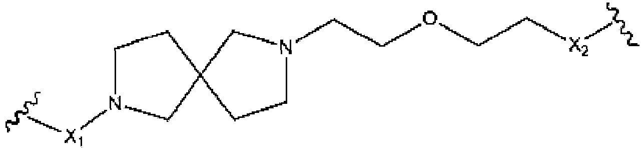 |
| 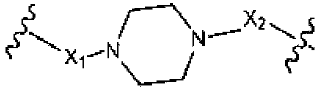 |
| 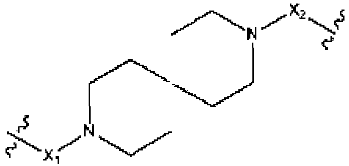 |
| 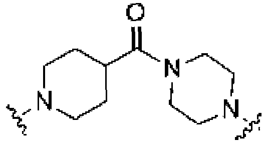 |
| 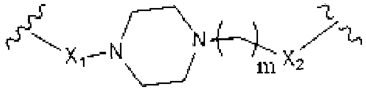 |
| 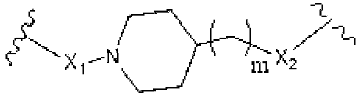 |
| 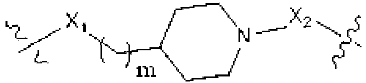 |
| 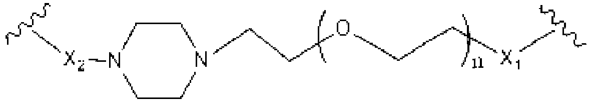 |
| 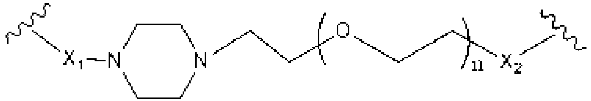 |
| 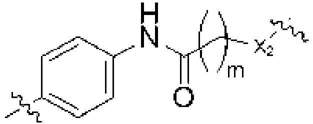 |
;
L₂ and L₃ are independently a linker having a structure selected from a group consisiting of the structures shown in the following:
| Structures of L₂ and L₃ |
|---|
| 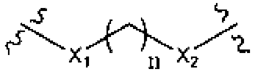 |
| 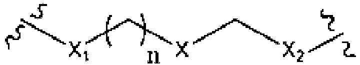 |
| 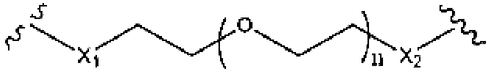 |
| 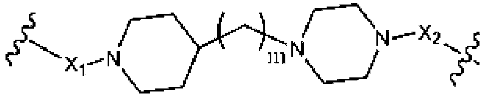 |
| 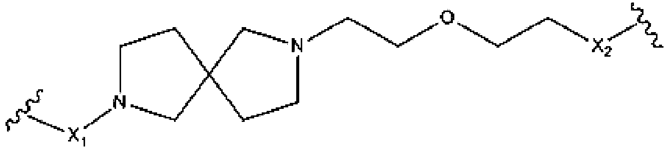 |
| 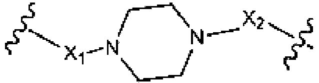 |
| 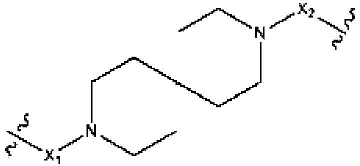 |
| 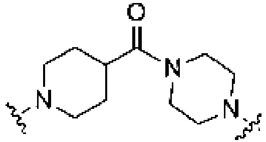 |
, wherein
X₁ is a bond, -NH-, -O, -CO-, CONH or -PhNHCO-;
X₂ is a bond, -NH-, -O-, -NHCOCH₂NH-, -NHCOCH₂O- or - alkyne-;
X is a heterocycle;
n is an integer from 1 to 6;
m is an integer from 0 to 8, and
wherein E is an E3 ubiquitin ligase binding domain, which comprises one of the structures shown in the following:
| Structure of E3 | | |
|---|---|---|
| 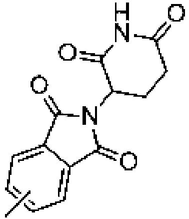 | 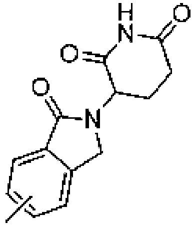 | 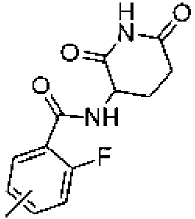 |

2. The compound or a salt thereof as claimed in claim 1, wherein the compound has a structure represented by Formula (I).

3. The compound or a salt thereof as claimed in claim 2, wherein R₁ is H, cyano, NO₂, N(CH₃)₂, NHAc or 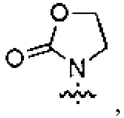 and R₂ is NHAc.

4. The compound or a salt thereof as claimed in claim 2, wherein the compound comprises one of the compounds shown in the following:
| **Compound number** | **Structure** |
|---|---|
| **D101** | 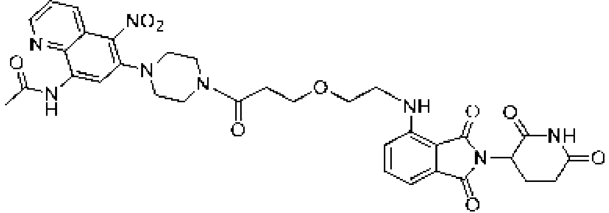 |
| **D102** | 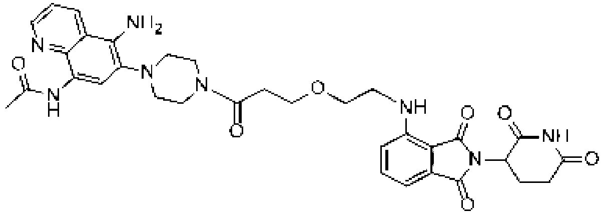 |
| **D103** | 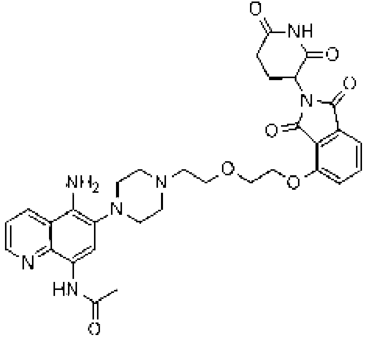 |
| **D104** | 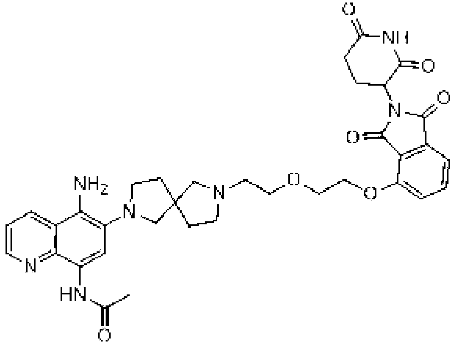 |
| **D105** | 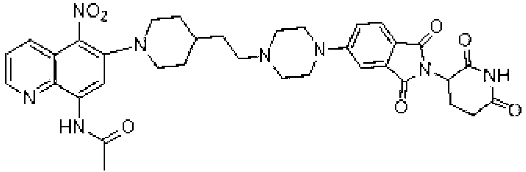 |
| **D106** | 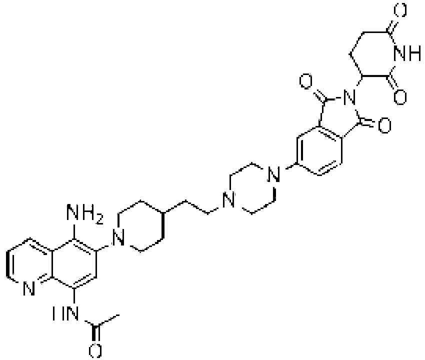 |
| **D107** | 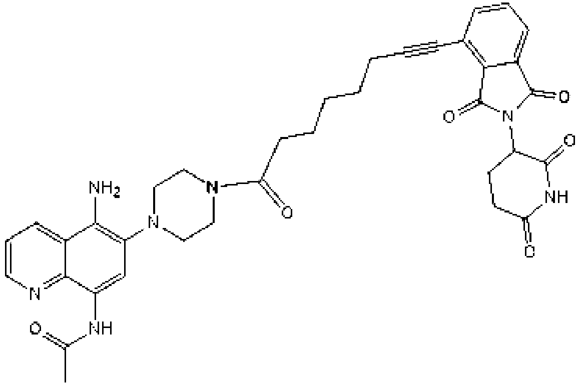 |
| **D108** | 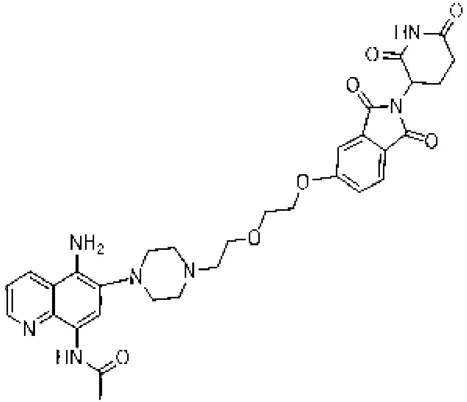 |
| **D109** | 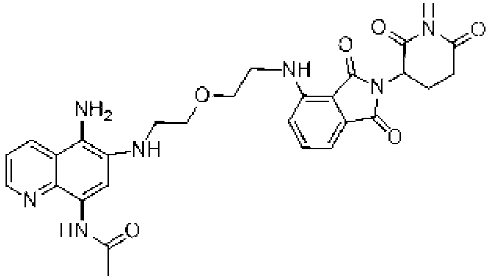 |
| **D110** | 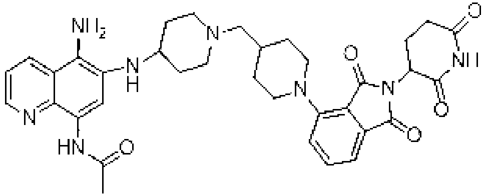 |
| **D111** | 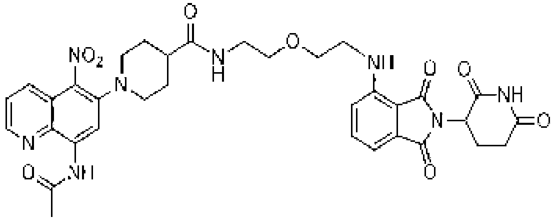 |
| **D112** | 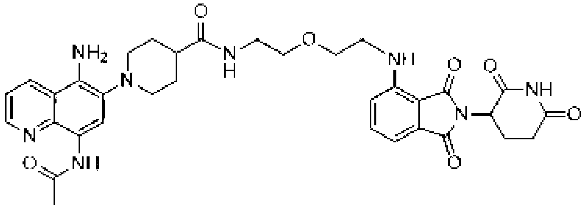 |
| **D113** | 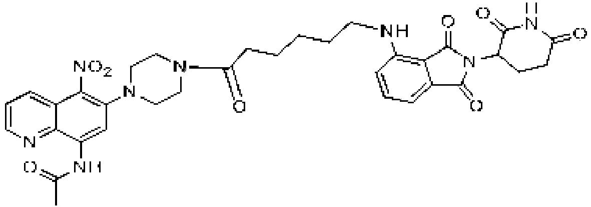 |
| **D114** | 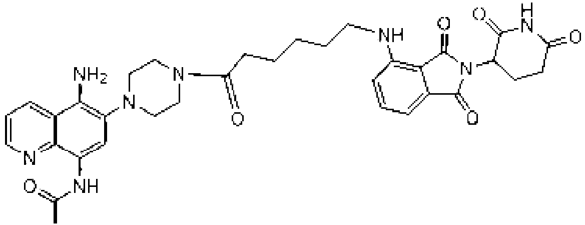 |
| **D115** | 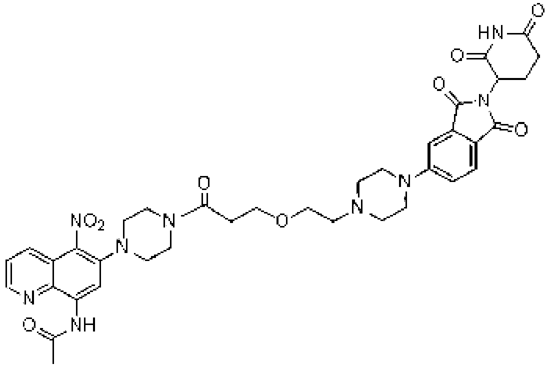 |
| **D116** | 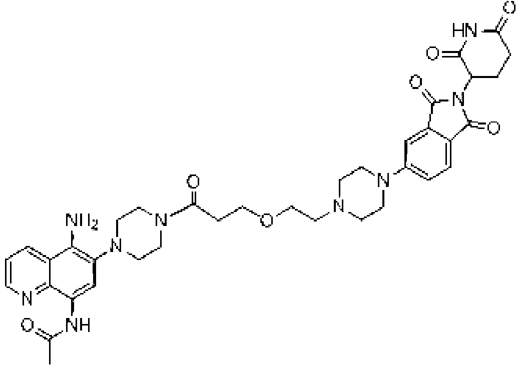 |
| **D117** | 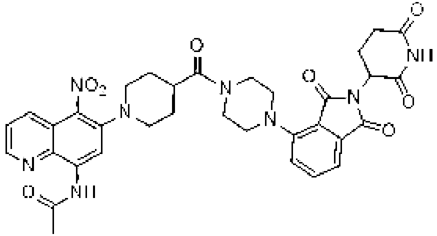 |
| **D118** | 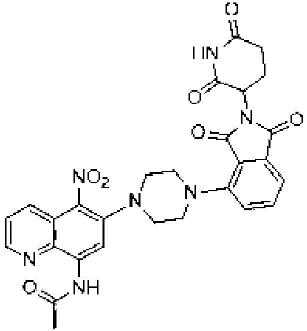 |
| **D119** | 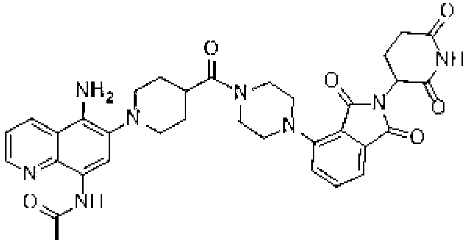 |
| **D120** | 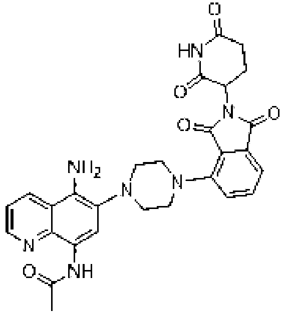 |
| **D121** | 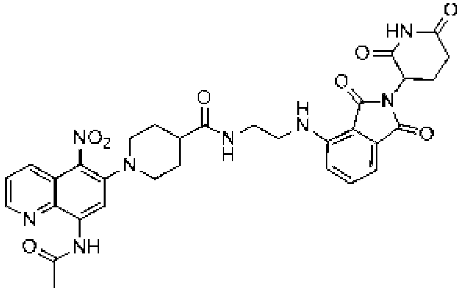 |
| **D122** | 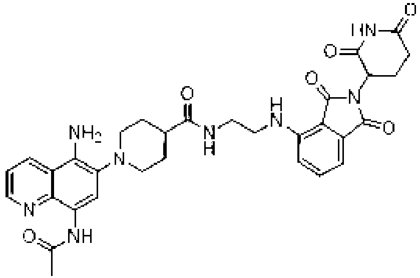 |
| **D123** | 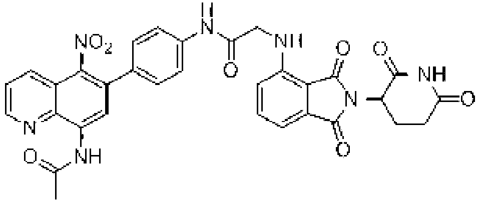 |
| **D124** | 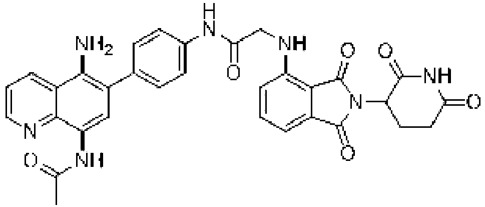 |
| **D125** | 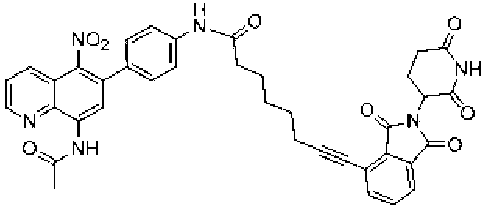 |
| **D126** | 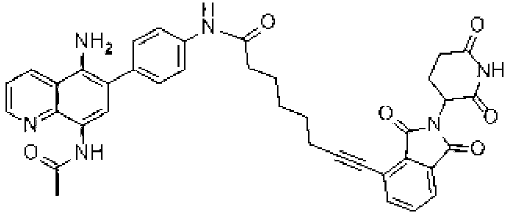 |
| **D127** | 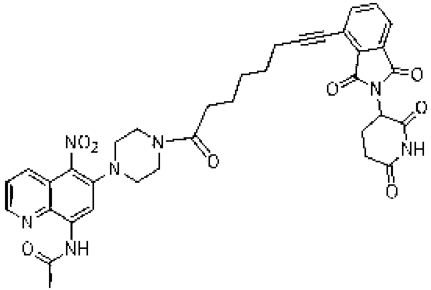 |
| **D128** | 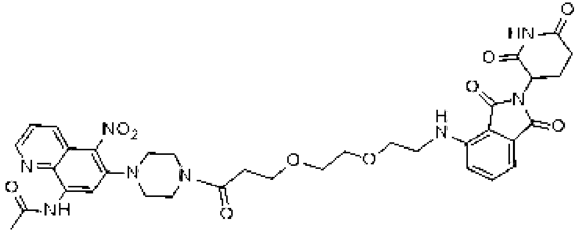 |
| **D129** | 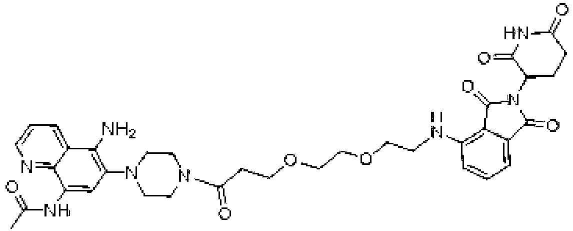 |
| **D130** | 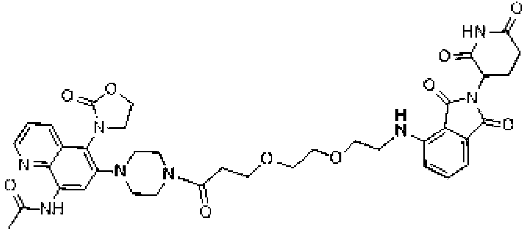 |
| **D131** | 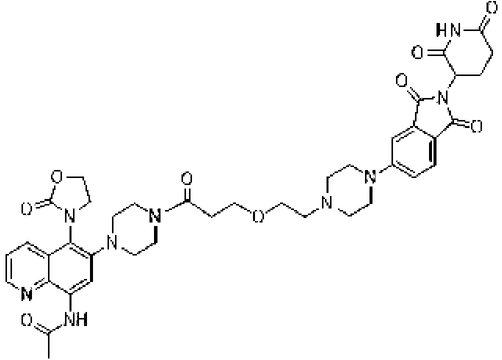 |
| **D132** | 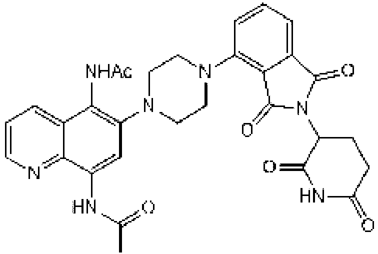 |
| **D133** | 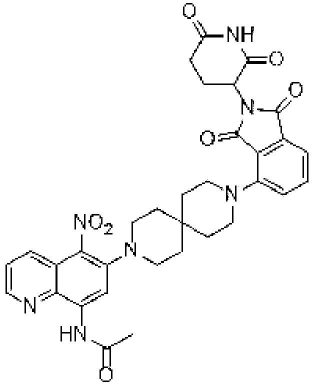 |
| **D134** | 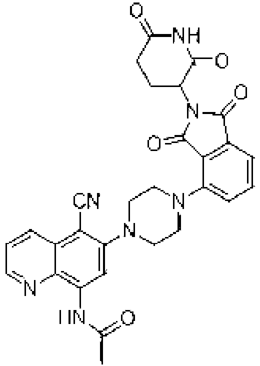 |
| **D135** | 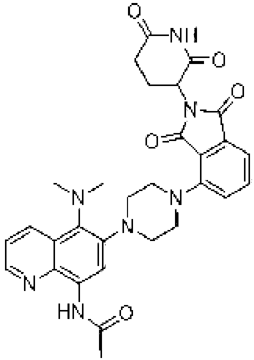 |
| **D136** | 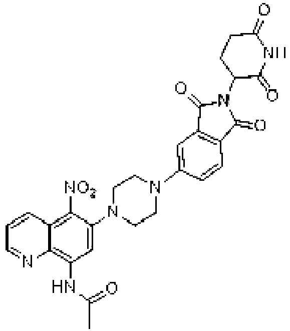 |
| **D137** | 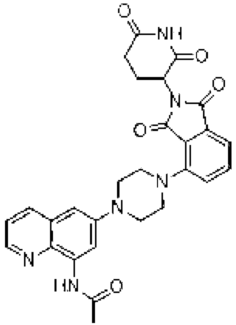 |
| **D138** | 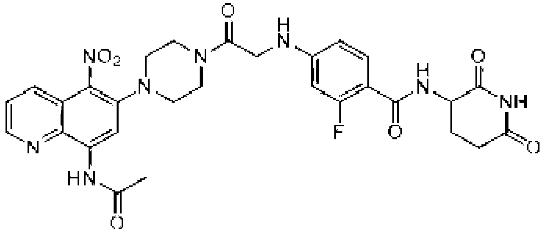 |
| **D139** | 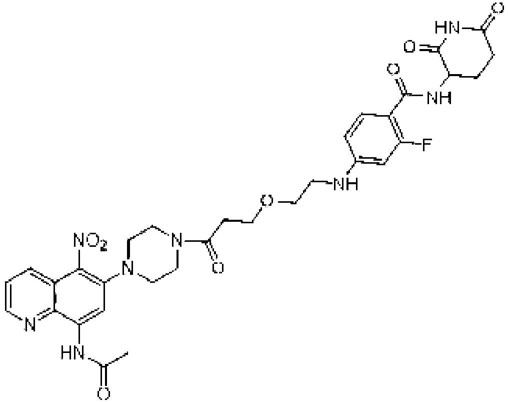 |
| **D140** | 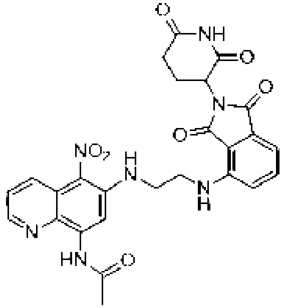 |
| **D141** | 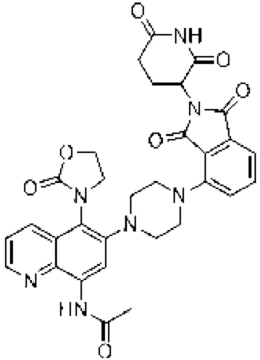 |
| **D142** | 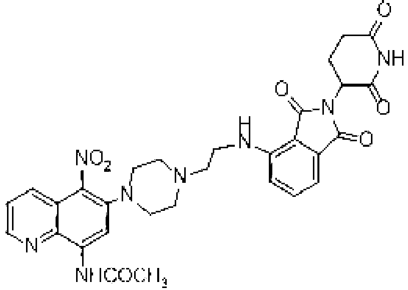 |
| **D143** | 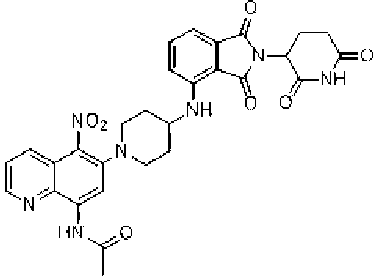 |
| **D144** | 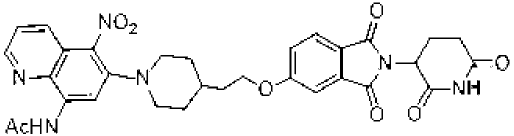 |
| **D145** | 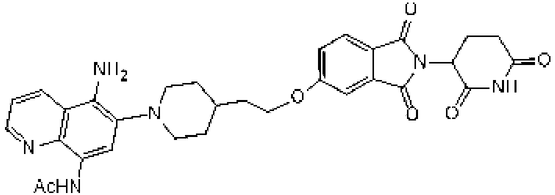 |
| **D146** | 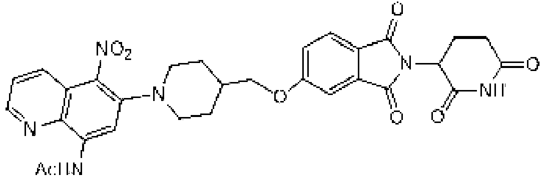 |
| **D147** | 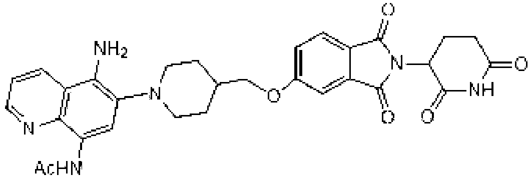 |
| **D148** | 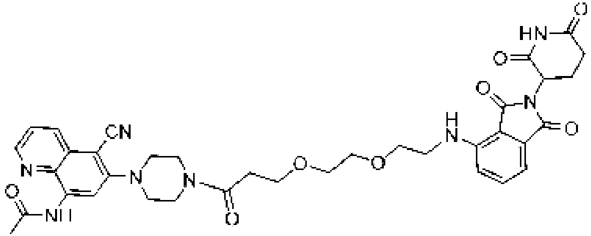 |
| **D149** | 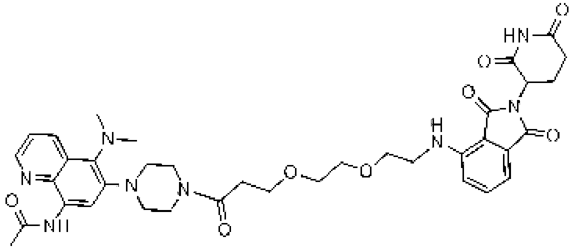 |
| **D150** | 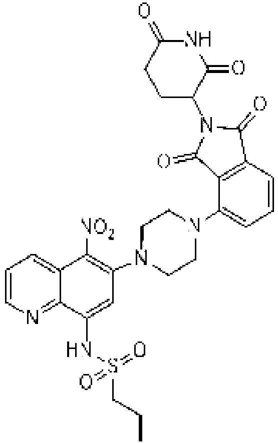 |
| **D151** | 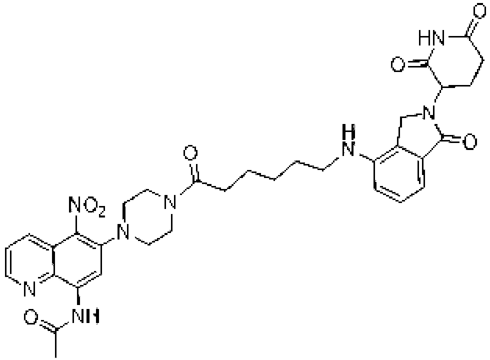 |

5. The compound or a salt thereof as claimed in claim 1, wherein the compound has a structure represented by Formula (II).

6. The compound or a salt thereof as claimed in claim 5, wherein R₇ is SCH₃, and R₈ is NHAc.

7. The compound or a salt thereof as claimed in claim 5, wherein the compound comprises one of the compounds shown in the following:
| **Compound number** | **Structure** |
|---|---|
| **D201** | 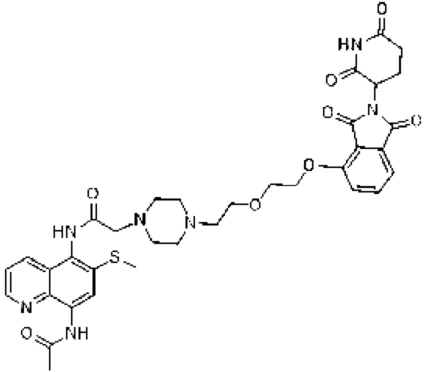 |
| **D202** | 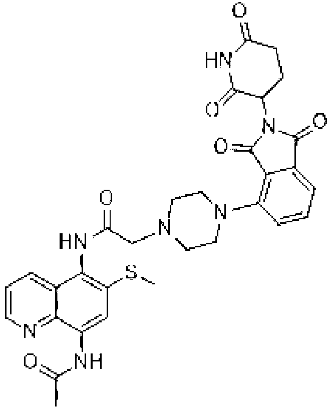 |
| **D203** | 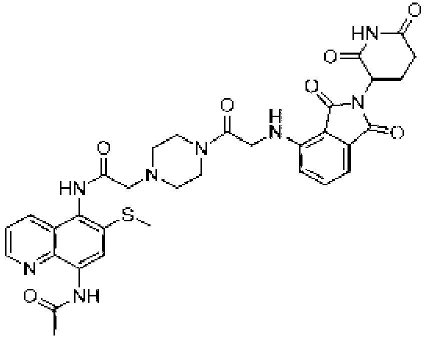 |
| **D204** | 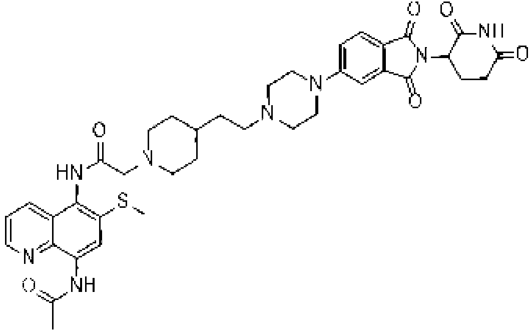 |
.

8. The compound or a salt thereof as claimed in claim 1, wherein the compound has a structure represented by Formula (III).

9. The compound or a salt thereof as claimed in claim 8, wherein R₁₀ is NO₂, CN or 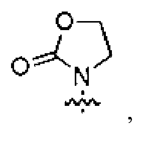 , and R₁₁ is OCH₃, SCH₃, morpholine, n-methylpiperazine, pyrrolidin-3-amine, 4-aminopiperidine or 4-(N-Boc-amino)piperidine.

10. The compound or a salt thereof as claimed in claim 8, wherein the compound comprises one of the compounds shown in the following:
| **Compound number** | **Structure** |
|---|---|
| **D301** | 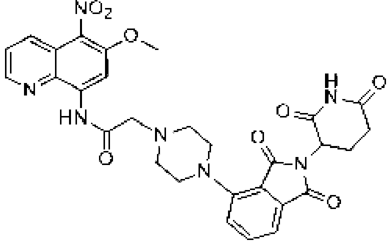 |
| **D302** | 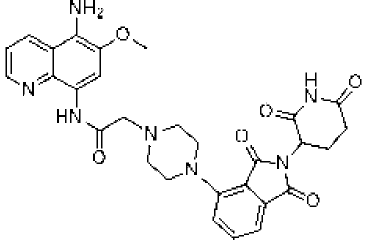 |
| **D303** | 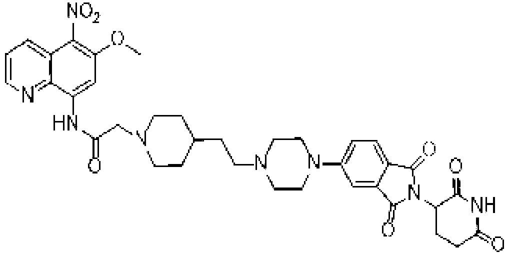 |
| **D304** | 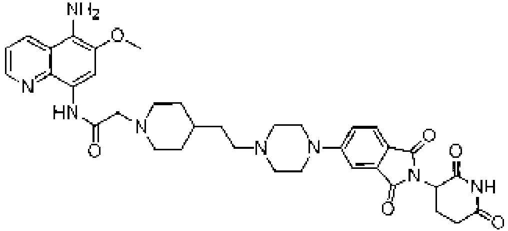 |
| **D305** | 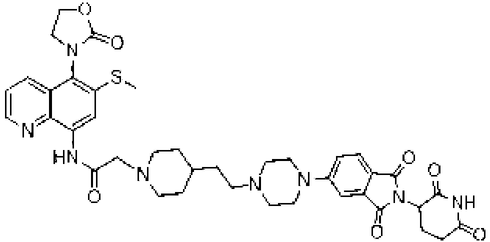 |
| **D306** | 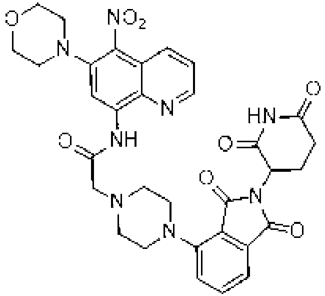 |
| **D307** | 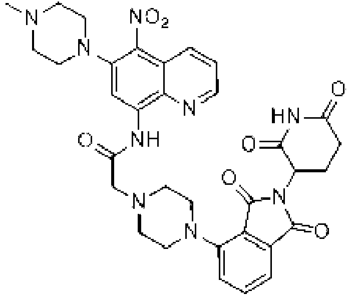 |
| **D308** | 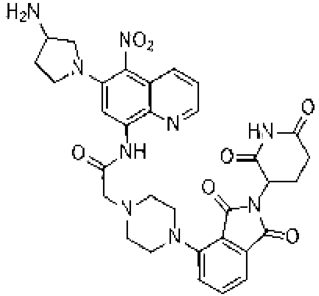 |
| **D309** | 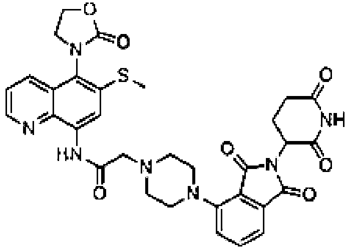 |
| **D310** | 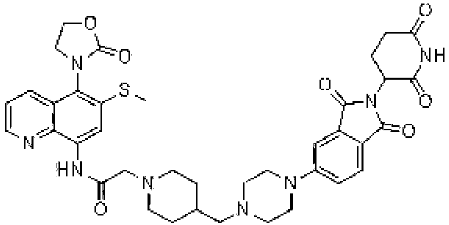 |
| **D311** | 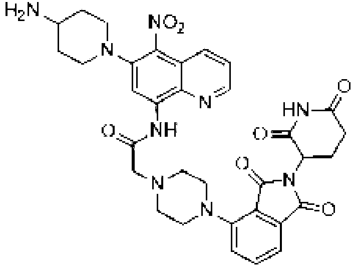 |
| **D312** | 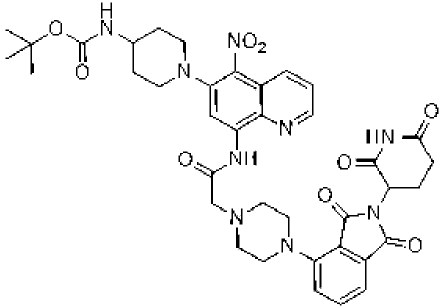 |
| **D313** | 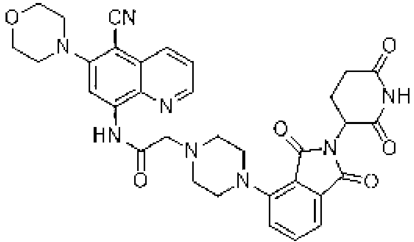 |
| **D314** | 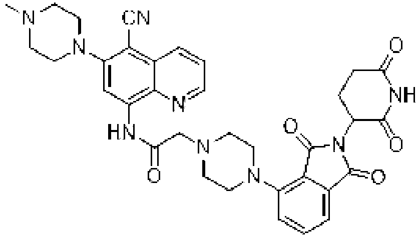 |
| **D315** | 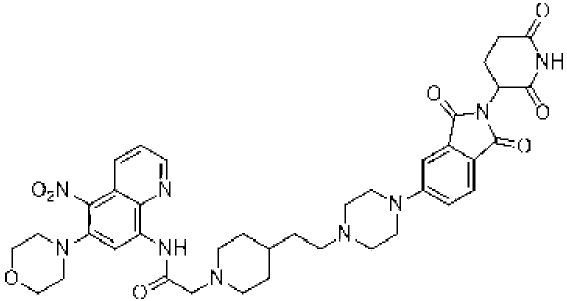 |
| **D316** | 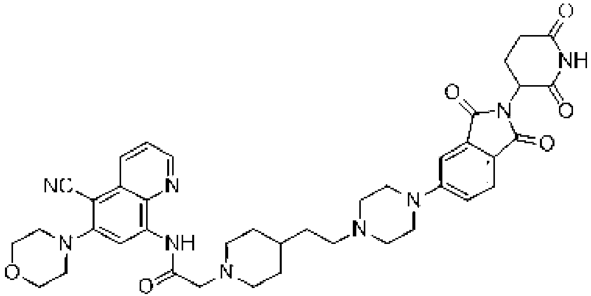 |

11. A pharmaceutical composition, comprising:
the compound or a salt thereof as claimed in any one of claim 1-10; and
a pharmaceutically acceptable carrier or salt.

12. An agent for degrading transactive response DNA-binding protein-43 and/or inhibiting the activity of transactive response DNA-binding protein-43, comprising:
the compound or a salt thereof as claimed in any one of claim 1-10.

13. The agent for degrading transactive response DNA-binding protein-43 and/or inhibiting the activity of transactive response DNA-binding protein-43 as claimed in claim 12, wherein the transactive response DNA-binding protein-43 is a full-length transactive response DNA-binding protein-43 or a C-terminal domain of transactive response DNA-binding protein-43 alone.

14. A pharmaceutical composition for treating and/or preventing a disease associated with transactive response DNA-binding protein-43 accumulation, comprising:
the compound or a salt thereof as claimed in any one of claims 1-10.

15. The pharmaceutical composition for treating and/or preventing a disease associated with transactive response DNA-binding protein-43 accumulation as claimed in claim 14, wherein the disease associated with transactive response DNA-binding protein-43 accumulation comprises a neurodegenerative disease.
